(19) Europäisches Patentamt
European Patent Office
Office européen des brevets

(11) EP 3 996 692 B1

(12) # EUROPEAN PATENT SPECIFICATION

(45) Date of publication and mention of the grant of the patent:
**11.06.2025 Bulletin 2025/24**

(21) Application number: **20735641.1**

(22) Date of filing: **08.07.2020**

(51) International Patent Classification (IPC):
***A61K 9/70*** *(2006.01)*

(52) Cooperative Patent Classification (CPC):
**A61K 9/7069; A61K 9/703; A61K 9/7084**

(86) International application number:
**PCT/EP2020/069270**

(87) International publication number:
**WO 2021/005117 (14.01.2021 Gazette 2021/02)**

(54) **TRANSDERMAL THERAPEUTIC SYSTEM COMPRISING AN ACTIVE AGENT-CONTAINING LAYER COMPRISING A SILICONE-CONTAINING POLYMER AND A SKIN CONTACT LAYER COMPRISING A SILICONE GEL ADHESIVE**

TRANSDERMALES THERAPEUTISCHES SYSTEM MIT EINER WIRKSTOFFHALTIGEN SCHICHT MIT EINEM SILIKONHALTIGEN POLYMER UND EINER HAUTKONTAKTSCHICHT MIT EINEM SILIKONGELKLEBSTOFF

SYSTÈME THÉRAPEUTIQUE TRANSDERMIQUE COMPRENANT UNE COUCHE CONTENANT UN AGENT ACTIF COMPORTANT UN POLYMÈRE CONTENANT DU SILICIUM ET UNE COUCHE DE CONTACT AVEC LA PEAU COMPORTANT UN ADHÉSIF DE GEL DE SILICONE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.07.2019 EP 19185343**

(43) Date of publication of application:
**18.05.2022 Bulletin 2022/20**

(73) Proprietor: **LTS Lohmann Therapie-Systeme AG**
**56626 Andernach (DE)**

(72) Inventors:
- **EMGENBROICH, Marco**
  **53359 Rheinbach (DE)**
- **KLAFFENBACH, Peter**
  **41564 Kaarst (DE)**
- **REUM, Nico**
  **56743 Mendig (DE)**
- **WAUER, Gabriel**
  **83727 Schliersee (DE)**
- **MOHR, Patrick**
  **53498 Bad Breisig (DE)**
- **SCHLÜTER, Anna**
  **53639 Königswinter (DE)**
- **WOLF, Hans-Werner**
  **56566 Neuwied (DE)**

(74) Representative: **Maiwald GmbH**
**Elisenhof**
**Elisenstraße 3**
**80335 München (DE)**

(56) References cited:
**EP-A1- 2 599 847**
**WO-A1-2014/028049**
**WO-A1-2016/130408**
**WO-A2-2010/124187**
**WO-A2-2011/022199**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 996 692 B1

## Description

### TECHNICAL FIELD OF THE INVENTION

The present invention relates to a transdermal therapeutic system (TTS) for the transdermal administration of an active agent to the systemic circulation, wherein the TTS comprises an active agent-containing layer comprising a silicone-containing polymer and a skin contact layer comprising a silicone gel adhesive, and processes of manufacture, method of treatments and uses thereof.

### BACKGROUND OF THE INVENTION

It is known in the art that transdermal therapeutic systems (TTS) may cause skin irritation depending on the adhesive layer.

Silicone gel adhesives (SGA) have been described in WO 2011/022199 A2 to address this problem by using an active-free skin contact layer based on silicone gel adhesives on top of an active agent-containing layer, so as to obtain a system with two different adhesive layers. Furthermore, WO 2014/028049 A1 describes a system comprising a backing substrate, an active-containing layer, a support substrate, and an active-free skin contact layer based on a silicone gel adhesive. WO2016/130408 A1 discloses a preparation of a multi-phase silicone acrylic hybrid visco-elastic composition which involves providing (a) a silicon-containing pressure sensitive adhesive composition comprising acrylate or methacrylate functionality and a condensation reaction product, pressure sensitive adhesive, formed by reacting a silicone resin, a silicone polymer and a silicon-containing capping agent. EP-2599847 discloses a TTS with layered structure of a backing layer, an active agent containing silicone acrylic hybrid polymer layer and a silicone-based release liner (like Dow Corning Syl-off 7680, a silicone gel used for liners and/or adhesive tapes). Possible active agent is buprenorphine (in a list).

However, it has been found that the systems described in the art have disadvantages either in view of an instable connection between the different adhesive layers or between the adhesive layers and the support substrate.

For example, it has been found that if the commercially available rivastigmine-containing TTS Exelon®, which comprises a rivastigmine-containing layer based on acrylate polymers, and a skin contact layer based on silicone polymers, is modified by using a skin contact layer based on silicone gel adhesives, problems arise from the instable connection between the rivastigmine-containing layer and the skin contact layer. In particular, it has been found that the TTS can be damaged upon removal of the release liner.

It is therefore desirable to provide a TTS for the transdermal delivery of an active agent, which overcomes the problem of skin irritation, but nevertheless has advantageous properties regarding the stability as well as the release of the active agent and the adhesiveness.

### OBJECTS AND SUMMARY OF THE INVENTION

The scope of the invention is defined by the claims. It is an object of the present invention to provide a TTS for the transdermal administration of an active agent, which is improved in comparison to the systems described in the prior art.

It is a further object of the present invention to provide a TTS for the transdermal administration of an active agent, which reduces the problem of skin irritation.

It is a further object of the present invention to provide a TTS for the transdermal administration of an active agent, which has a high mechanical stability. In particular, it is an object of the present invention that the layers of the TTS stick together also upon removal of the release liner, so that a damage of the TTS can be avoided.

It is a further object of the present invention to provide a TTS for the transdermal administration of an active agent, which avoids a complex structure.

It is a further object of the present invention to provide a TTS for the transdermal administration of an active agent, which provides suitable drug delivery properties and plasma concentrations of the active agent to be therapeutically effective.

It is a further object of the present invention to provide a TTS for the transdermal administration of an active agent, which has the required adhesive properties for administration for at least 24 hours or at least 72 hours.

It is a further object of the present invention to provide a TTS for the transdermal administration of an active agent, which can be used in a method of treatment.

It has surprisingly been found that at least one of these objects and others are accomplished by the present invention, which according to one aspect relates to a TTS for the transdermal administration of an active agent comprising an active agent-containing layer structure, said active agent-containing layer structure comprising:

A) a backing layer;

B) an active agent-containing layer comprising at least one silicone-containing polymer; and
C) a skin contact layer;

wherein the skin contact layer is an adhesive layer comprising a silicone gel adhesive, which is directly attached to the active agent-containing layer, wherein the active agent is rivastigmine or buprenorphine.

It has been found that the TTS according to the present invention, which comprises a silicone gel adhesive based skin contact layer, which is directly attached to an active agent-containing layer comprising at least one silicone-containing polymer has advantageous properties regarding reduced skin irritation, but at the same time has a high stability regarding a potential damage upon removal of a release liner from the system. In particular, it has been found that the content of the silicone-containing polymer is decisive for the stability of the TTS.

In a preferred embodiment, the at least one silicone-containing polymer is a silicone acrylic hybrid polymer, a silicone-based polymer, or a combination thereof. Further details regarding silicone acrylic hybrid polymers and silicone-based polymers will be provided below. Particularly preferred in the context of the present invention are silicone acrylic hybrid polymers.

In another preferred embodiment, the at least one silicone-containing polymer is present in the active agent-containing layer in an amount of from 20 to 90 % by weight, preferably 60 to 90 % by weight, based on the total weight of the active agent-containing layer.

In another preferred embodiment, the at least one active agent is present in the active agent-containing layer in an amount of from 5 to 25 % by weight, preferably 8 to 22 % by weight, based on the total weight of the active agent-containing layer.

In another preferred embodiment, the active agent-containing layer is an active agent-containing matrix layer.

According to one specific aspect, the present invention therefore relates to a transdermal therapeutic system for the transdermal administration of an active agent comprising an active agent-containing layer structure, said active agent-containing layer structure comprising:

A) a backing layer;
B) an active agent-containing matrix layer comprising an active agent in an amount of from 5 to 25 % by weight, and at least one silicone-containing polymer, which is a silicone acrylic hybrid polymer, a silicone-based polymer, or a combination thereof, in an amount of from 20 to 90 % by weight, in each case based on the total weight of the active agent-containing matrix layer; and
C) a skin contact layer;

wherein the skin contact layer is an adhesive layer comprising a silicone gel adhesive, which is directly attached to the active agent-containing layer,
and wherein the silicone gel adhesive is obtainable by reacting a gel producing composition comprising (i) a copolymer of vinylmethylsiloxane and dimethylsiloxane with (ii) methylhydrogen polysiloxane with trimethylsilyl endgroups in the presence of (iii) a platinum catalyst.

According to another specific aspect, the present invention relates to a transdermal therapeutic system for the transdermal administration of an active agent comprising an active agent-containing layer structure, said active agent-containing layer structure comprising:

A) a backing layer;
B) an active agent-containing matrix layer comprising an active agent in an amount of from 8 to 22 % by weight, and at least one silicone-containing polymer, which is a silicone acrylic hybrid polymer, a silicone-based polymer, or a combination thereof, in an amount of from 60 to 90 % by weight, in each case based on the total weight of the active agent-containing matrix layer; and
C) a skin contact layer;

wherein the skin contact layer is an adhesive layer comprising a silicone gel adhesive, which is directly attached to the active agent-containing layer,
and wherein the silicone gel adhesive is obtainable by reacting a gel producing composition comprising (i) a copolymer of vinylmethylsiloxane and dimethylsiloxane with (ii) methylhydrogen polysiloxane with trimethylsilyl endgroups in the presence of (iii) a platinum catalyst.

Preferred active agents in the context of the present invention are buprenorphine and rivastigmine. The at least one silicone-containing polymer is preferably a silicone acrylic hybrid polymer.

In a further aspect, the present invention relates to a TTS as described herein for use in a method of treatment,

wherein the transdermal therapeutic system is preferably applied to the skin of the patient for at least 24 hours or at least 72 hours.

In yet a further aspect, the present invention relates to a process for manufacturing an active agent-containing containing layer structure for use in a transdermal therapeutic system according to the present invention comprising the steps of:

1.1) coating a coating composition comprising

- an active agent; and
- at least one silicone-containing polymer on a first foil;

1.2) drying the coated coating composition to form the active agent-containing layer;
1.3) laminating the active agent-containing layer with a backing layer;
2.1) coating a gel producing composition comprising

(i) at least one alkenyl-substituted polydiorganosiloxane,
(ii) at least one organosiloxane, which contains silicone-bonded hydrogen atoms, and
(iii) at least one catalyst for the reaction of the SiH groups with the Si-alkenyl groups, on a second foil;

2.2) crosslinking the gel producing composition at a temperature of from 50 °C to 150 °C or by applying UV light to form the skin contact layer;
2.3) laminating the skin contact layer with a release liner;
3.1) removing the foils from the active agent-containing layer and the skin contact layer; and
3.2) laminating the open side of the active agent-containing layer onto the open side of the skin contact layer to obtain an active agent-containing layer structure.

It is to be understood that steps 1.1) to 1.3) are for the preparation of the active agent-containing layer, while steps 2.1) to 2.3) are for the preparation of the skin contact layer, and while steps 3.1) to 3.2) are for the preparation of the active agent-containing layer structure comprising the two layers prepared before. It is to be understood that the preparation of the active agent-containing layer and the preparation of the skin contact layer may be performed in any order, i.e. the skin contact layer may be prepared before the active agent-containing layer is prepared or vice versa. Furthermore, the preparation of the active agent-containing layer and the preparation of the skin contact layer may also be performed simultaneously. Once the two layers have been prepared, steps 3.1) and 3.2) will be performed.

## DEFINITIONS

Within the meaning of this invention, the term "transdermal therapeutic system" (TTS) refers to a system by which the active agent (e.g. rivastigmine) is administered to the systemic circulation *via* transdermal delivery and refers to the entire individual dosing unit that is applied, after removing an optionally present release liner, to the skin of a patient, and which comprises a therapeutically effective amount of active agent in an active agent-containing layer structure and optionally an additional adhesive overlay on top of the active agent-containing layer structure. The active agent-containing layer structure may be located on a release liner (a detachable protective layer), thus, the TTS may further comprise a release liner. Within the meaning of this invention, the term "TTS" in particular refers to systems providing transdermal delivery, excluding active delivery for example *via* iontophoresis or microporation. Transdermal therapeutic systems may also be referred to as transdermal drug delivery systems (TDDS) or transdermal delivery systems (TDS).

Within the meaning of this invention, the term "active agent-containing layer structure" refers to the layer structure comprising a backing layer, an active agent-containing layer, and a skin contact layer as described herein. The active agent-containing layer structure comprises a therapeutically effective amount of an active agent. Preferably, the active agent-containing layer structure is an active agent-containing self-adhesive layer structure.

Within the meaning of this invention, the term "therapeutically effective amount" refers to a quantity of active agent in the TTS sufficient to provide, if administered by the TTS to a patient, the desired pharmacological effect. A TTS usually contains more active in the system than is in fact provided to the skin and the systemic circulation. This excess amount of active agent is usually necessary to provide enough driving force for the delivery from the TTS to the systemic circulation.

Within the meaning of this invention, the terms "active", "active agent", and the like, as well as the term "rivastigmine" or "buprenorphine" refer to the respective active agent in any pharmaceutically acceptable chemical and morphological form and physical state. These forms include without limitation the active agent in its free base / free acid form, protonated or partially protonated form, deprotonated or partially deprotonated form, salts, cocrystals and in

particular acid / base addition salts formed by addition of an inorganic or organic acid / base such as hydrochloride or tartrate salts, solvates, hydrates, clathrates, complexes and so on, as well as the active agent in the form of particles, which may be micronized, crystalline and/or amorphous, and any mixtures of the aforementioned forms. The active agent, where contained in a medium such as a solvent, may be dissolved or dispersed or in part dissolved and in part dispersed.

When the active agent is mentioned to be used in a particular form in the manufacture of the TTS, this does not exclude interactions between this form of the active agent and other ingredients of the active agent-containing layer structure, e.g. salt formation or complexation, in the final TTS. This means that, even if the active agent is included in its free base / acid form, it may be present in the final TTS in protonated or partially protonated / or deprotonated or partially deprotonated form or in the form of an acid addition salt, or, if it is included in the form of a salt, parts of it may be present as free base in the final TTS. Unless otherwise indicated, in particular the amount of the active agent in the layer structure relates to the amount of the active agent included in the TTS during manufacture of the TTS and is calculated based on the active agent itself, but not on other forms thereof. E.g., when a) 0.1 mmol (equal to 25.03 mg) rivastigmine base or b) 0.1 mmol (equal to 40.04 mg) rivastigmine tartrate is included in the TTS during manufacture, the amount of rivastigmine in the layer structure is, within the meaning of the invention, in both cases 0.1 mmol or 25.03 mg.

The active agent starting material included in the TTS during manufacture of the TTS may be in the form of particles. The active agent may e.g. be present in the active agent-containing layer structure in the form of particles and/or dissolved.

Within the meaning of this invention, the term "particles" refers to a solid, particulate material comprising individual particles, the dimensions of which are negligible compared to the material. In particular, the particles are solid, including plastic/deformable solids, including amorphous and crystalline materials.

Within the meaning of this invention, the term "dispersing" refers to a step or a combination of steps wherein a starting material (e.g. rivastigmine) is not totally dissolved. Dispersing in the sense of the invention comprises the dissolution of a part of the starting material (e.g. rivastigmine particles), depending on the solubility of the starting material (e.g. the solubility of rivastigmine in the coating composition).

There are two main types of TTS for active agent delivery, i.e. matrix-type TTS and reservoir-type TTS. The release of the active agent in a matrix-type TTS is mainly controlled by the matrix including the active agent itself. In contrast thereto, a reservoir-type TTS typically needs a rate-controlling membrane controlling the release of the active agent. In principle, also a matrix-type TTS may contain a rate-controlling membrane. However, matrix-type TTS are advantageous in that, compared to reservoir-type TTS, usually no rate determining membranes are necessary and no dose dumping can occur due to membrane rupture. In summary, matrix-type transdermal therapeutic systems (TTS) are less complex in manufacture and easy and convenient to use by patients.

Within the meaning of this invention, "matrix-type TTS" refers to a system or structure wherein the active is homogeneously dissolved and/or dispersed within a polymeric carrier, i.e. the matrix, which forms with the active agent and optionally remaining ingredients a matrix layer. In such a system, the matrix layer controls the release of the active agent from the TTS. Preferably, the matrix layer has sufficient cohesion to be self-supporting so that no sealing between other layers is required. Accordingly, the active agent-containing layer may in one embodiment of the invention be an active agent-containing matrix layer, wherein the active agent is homogeneously distributed within a polymer matrix. In certain embodiments, the active agent-containing matrix layer may comprise two active agent-containing matrix layers, which may be laminated together. Matrix-type TTS may in particular be in the form of a "drug-in-adhesive"-type TTS referring to a system wherein the active is homogeneously dissolved and/or dispersed within a pressure-sensitive adhesive matrix. In this connection, the active agent-containing matrix layer may also be referred to as active agent-containing pressure sensitive adhesive layer or active agent-containing pressure sensitive adhesive matrix layer. A TTS comprising the active agent dissolved and/or dispersed within a polymeric gel, e.g. a hydrogel, is also considered to be of matrix-type in accordance with present invention. It is to be understood that a TTS comprising an active agent-containing matrix layer may additionally also comprise a skin contact layer.

TTS with a liquid active agent-containing reservoir are referred to by the term "reservoir-type TTS". In such a system, the release of the active agent is preferably controlled by a rate-controlling membrane. In particular, the reservoir is sealed between the backing layer and the rate-controlling membrane. Accordingly, the active agent-containing layer may in one embodiment be an active agent-containing reservoir layer, which preferably comprises a liquid reservoir comprising the active agent. Furthermore, the reservoir-type TTS typically additionally comprises a skin contact layer, wherein the reservoir layer and the skin contact layer may be separated by the rate-controlling membrane. In the reservoir layer, the active agent is preferably dissolved in a solvent such as ethanol or water or in silicone oil. The skin contact layer typically has adhesive properties.

Reservoir-type TTS are not to be understood as being of matrix-type within the meaning of the invention. However, microreservoir TTS (biphasic systems having deposits (e.g. spheres, droplets) of an inner active-containing phase dispersed in an outer polymer phase), considered in the art to be a mixed form of a matrix-type TTS and a reservoir-type TTS that differ from a homogeneous single phase matrix-type TTS and a reservoir-type TTS in the concept of drug transport and drug delivery, are considered to be of matrix-type within the meaning of the invention. The sizes of

microreservoir droplets can be determined by an optical microscopic measurement (for example by Leica MZ16 including a camera, for example Leica DSC320) by taking pictures of the microreservoirs at different positions at an enhancement factor between 10 and 400 times, depending on the required limit of detection. By using imaging analysis software, the sizes of the microreservoirs can be determined.

Within the meaning of this invention, the term "active agent-containing layer" refers to a layer containing the active agent and providing the area of release. The term covers active agent-containing matrix layers and active agent-containing reservoir layers. If the active agent-containing layer is an active agent-containing matrix layer, said layer is present in a matrix-type TTS. According to the present invention, an additional skin contact layer is present as adhesive layer. Furthermore, an adhesive overlay may be present. The skin contact layer is typically manufactured such that it is active agent-free. However, due to the concentration gradient, the active agent will migrate from the matrix layer to the skin contact layer over time, until an equilibrium is reached. If the active agent-containing layer is an active agent-containing reservoir layer, said layer is present in a reservoir-type TTS, and the layer comprises the active agent in a liquid reservoir. In addition, a skin contact layer is present, in order to provide adhesive properties. The skin contact layer is typically manufactured such that it is active agent-free. If the skin contact layer is free of active agent the active agent will migrate, due to the concentration gradient, from the reservoir layer to the skin contact layer over time, until an equilibrium is reached. Additionally, an adhesive overlay may be provided.

As used herein, the active agent-containing layer is preferably an active agent-containing matrix layer, and it is referred to the final solidified layer. Preferably, an active agent-containing matrix layer is obtained after coating and drying the solvent-containing coating composition as described herein. Alternatively, an active agent-containing matrix layer is obtained after melt-coating and cooling. The active agent-containing matrix layer may also be manufactured by laminating two or more such solidified layers (e.g. dried or cooled layers) of the same composition to provide the desired area weight. Preferably, the matrix layer is a pressure sensitive adhesive matrix layer. Optionally, an adhesive overlay may be present.

Within the meaning of this invention, the term "pressure-sensitive adhesive" (also abbreviated as "PSA") refers to a material that in particular adheres with finger pressure, is permanently tacky, exerts a strong holding force and should be removable from smooth surfaces without leaving a residue. A pressure sensitive adhesive layer, when in contact with the skin, is "self-adhesive", i.e. provides adhesion to the skin so that typically no further aid for fixation on the skin is needed. A "self-adhesive" layer structure according to the present invention includes a pressure sensitive adhesive layer for skin contact which is provided in the form of an additional layer, i.e. a pressure sensitive adhesive skin contact layer. The pressure-sensitive adhesive properties of a pressure-sensitive adhesive depend on the polymer or polymer composition used.

Within the meaning of this invention, the term "silicone acrylic hybrid polymer" refers to a polymerization product including repeating units of a silicone sub-species and an acrylate-sub species. The silicone acrylic hybrid polymer thus comprises a silicone phase and an acrylic phase. The term "silicone acrylic hybrid" is intended to denote more than a simple blend of a silicone-based sub-species and an acrylate-based sub-species. Instead, the term denotes a polymerized hybrid species that includes silicone-based sub-species and acrylate-based sub-species that have been polymerized together. The silicone acrylic hybrid polymer may also be referred to as a "silicone acrylate hybrid polymer" as the terms acrylate and acrylic are generally used interchangeably in the context of the hybrid polymers used in the present invention.

Within the meaning of this invention, the term "silicone acrylic hybrid pressure-sensitive adhesive" refers to a silicone acrylic hybrid polymer in the form of a pressure-sensitive adhesive. Silicone acrylic hybrid pressure-sensitive adhesives are described, for example, in EP 2 599 847 and WO 2016/130408. Examples of silicone acrylic hybrid pressure-sensitive adhesives include the PSA series 7-6100 and 7-6300 manufactured and supplied in n-heptane or ethyl acetate by Dow Corning (7-610X and 7-630X; X=1 n-heptane-based / X=2 ethyl acetate-based). It was found that, depending on the solvent in which the silicone acrylic hybrid PSA is supplied, the arrangement of the silicone phase and the acrylic phase providing a silicone or acrylic continuous external phase and a corresponding discontinuous internal phase is different. If the silicone acrylic hybrid PSA is supplied in n-heptane, the composition contains a continuous, silicone external phase and a discontinuous, acrylic internal phase. If the silicone acrylic hybrid PSA composition is supplied in ethyl acetate, the composition contains a continuous, acrylic external phase and a discontinuous, silicone internal phase.

Within the meaning of this invention, the term "silicon-containing pressure-sensitive adhesive composition comprising acrylate or methacrylate functionality" comprises the condensation reaction product of a silicone resin, a silicone polymer, and a silicon-containing capping agent which provides said acrylate or methacrylate functionality. It is to be understood that the silicon-containing pressure-sensitive adhesive composition comprising acrylate or methacrylate functionality can include only acrylate functionality, only methacrylate functionality, or both acrylate functionality and methacrylate functionality.

Within the meaning of this invention, the term "non-hybrid polymer" is used synonymously for a polymer which does not include a hybrid species. Preferably, the non-hybrid polymer is a pressure-sensitive adhesive (e.g. a silicone- or acrylate-based pressure-sensitive adhesives). A preferred non-hybrid polymer according to the present invention is a "silicone-based polymer", which, as used herein, is a polymer obtainable by polycondensation of silanol endblocked polydimethylsiloxane with a silicate resin.

Within the meaning of this invention, the term "silicone-containing polymer" refers to a polymer containing a polysiloxane. The term covers silicone acrylate hybrid polymers as well as silicone-based non-hybrid polymers as defined above, as both polymers contain polysiloxanes. Preferably, the silicone-containing polymer according to the present invention is a non-curing polymer as explained in further detail below. Accordingly, the silicone-containing polymer is typically applied by a hot-melt or a solvent based process and preferably does not undergo further curing to solidify.

Within the meaning of this invention, the term "silicone gel adhesive" refers to an elastic, jelly-like material formed by lightly crosslinking silicone polymers. It may be prepared from a gel producing composition as described further below upon curing. In particular, the silicone gel adhesive preferably forms upon curing of polysiloxanes comprising reactive groups such as Si-H reactive groups and aliphatic unsaturated groups, which react with each other in the presence of a hydrosilylation catalyst. Thus, the silicone gel adhesive is obtained from a curable gel producing composition, while the silicone-containing polymers according to the present invention are preferably non-curing polymers. Accordingly, the silicone gel adhesive is typically applied by using a curable gel producing composition, which solidifies upon curing.

As used herein, an active agent-containing matrix layer is a layer containing the active agent dissolved or dispersed in at least one polymer, or containing the active agent dissolved in a solvent to form an active agent-solvent mixture that is dispersed in the form of deposits (in particular droplets) in at least one polymer. Preferably, the at least one polymer is a polymer-based pressure-sensitive adhesive (e.g. a silicone acrylic hybrid pressure-sensitive adhesive). Within the meaning of this invention, the term "pressure-sensitive adhesive layer" refers to a pressure-sensitive adhesive layer obtained from a solvent-containing adhesive coating composition after coating on a film and evaporating the solvents.

Within the meaning of this invention, the term "skin contact layer" refers to the layer included in the active agent-containing layer structure to be in direct contact with the skin of the patient during administration. When the TTS comprises a skin contact layer, the other layers of the active agent-containing layer structure do not contact the skin and do not necessarily have self-adhesive properties. As outlined above, an additional skin contact layer attached to the active agent-containing layer may over time absorb parts of the active agent. The sizes of the skin contact layer and the active agent-containing layer are usually coextensive and correspond to the area of release. However, the area of the skin contact layer may also be greater than the area of the active agent-containing layer. In such a case, the area of release still refers to the area of the active agent-containing layer.

Within the meaning of this invention, the term "area weight" refers to the dry weight of a specific layer, e.g. of the matrix layer, provided in $g/m^2$. The area weight values are subject to a tolerance of $\pm$ 10 %, preferably $\pm$ 7.5 %, due to manufacturing variability.

If not indicated otherwise "%" refers to weight-% (% by weight).

Within the meaning of this invention, the term "polymer" refers to any substance consisting of so-called repeating units obtained by polymerizing one or more monomers, and includes homopolymers which consist of one type of monomer and copolymers which consist of two or more types of monomers. Polymers may be of any architecture such as linear polymers, star polymer, comb polymers, brush polymers, of any monomer arrangements in case of copolymers, e.g. alternating, statistical, block copolymers, or graft polymers. The minimum molecular weight varies depending on the polymer type and is known to the skilled person. Polymers may e.g. have a molecular weight above 2000, preferably above 5000 and more preferably above 10,000 Dalton. Correspondingly, compounds with a molecular weight below 2000, preferably below 5000 or more preferably below 10,000 Dalton are usually referred to as oligomers.

Within the meaning of this invention, the term "cross-linking agent" refers to a substance which is able to cross-link functional groups contained within the polymer.

Within the meaning of this invention, the term "adhesive overlay" refers to a self-adhesive layer structure that is free of active agent and larger in area than the active agent-containing structure and provides additional area adhering to the skin, but no area of release of the active agent. It enhances thereby the overall adhesive properties of the TTS. The adhesive overlay comprises a backing layer that may provide occlusive or non-occlusive properties and an adhesive layer. Preferably, the backing layer of the adhesive overlay provides non-occlusive properties.

Within the meaning of this invention, the term "backing layer" refers to a layer which supports the active agent-containing layer or forms the backing of the adhesive overlay. At least one backing layer in the TTS and usually the backing layer of the active agent-containing layer is substantially impermeable to the active agent contained in the layer during the period of storage and administration and thus prevents active loss or cross-contamination in accordance with regulatory requirements. Preferably, the backing layer is also occlusive, meaning substantially impermeable to water and water-vapor. Suitable materials for a backing layer include polyethylene terephthalate (PET), polyethylene (PE), ethylene vinyl acetate-copolymer (EVA), polyurethanes, and mixtures thereof. Suitable backing layers are thus for example PET laminates, EVA-PET laminates and PE-PET laminates. Also suitable are woven or non-woven backing materials.

The TTS according to the present invention can be characterized by certain parameters as measured in an *in vitro* skin permeation test.

In general, the *in vitro* permeation test is performed in a Franz diffusion cell, with EVA membrane (e.g. 9 % vinyl acetate and 50 $\mu$m thickness, preferably provided by 3M), and with phosphate buffer pH 5.5 or 7.4 as receptor medium (32

°C with 0.1 % saline azide).

Further, *in vitro* permeation test may be performed in a Franz diffusion cell, with human or animal skin and preferably with dermatomed split-thickness human skin with a thickness of 800 μm and an intact epidermis, and with phosphate buffer pH 5.5 or 7.4 as receptor medium (32 °C with 0.1 % saline azide) with or without addition of a maximum of 40 vol-% organic solvent e.g. ethanol, acetonitrile, isopropanol, dipropylenglycol, PEG 400 so that a receptor medium may e.g. contain 60 vol-% phosphate buffer pH 5.5, 30 vol-% dipropylenglycol and 10 vol-% acetonitrile.

Where not otherwise indicated, the *in vitro* permeation test is performed with EVA membrane (9 % vinyl acetate, 50 μm), and with phosphate buffer pH 5.5 as receptor medium (32 °C with 0.1 % saline azide). The amount of active permeated into the receptor medium is determined in regular intervals using a validated HPLC method (column: stainless steel column 150 mm x 4.6 mm internal diameter with C18 base and acid deactivated stationary phase, 3.5 μm particle size, e.g. Zorbax SB C18 (Agilent); column temperature: 25 °C; mobile phase: acetonitrile / water / TEA = 20:80:0.35 (v/v/v) pH 3.5; flow rate: 1.0 ml/min; pressure: 135 bar; injection volume: 50 μL; stop time: 8 min) with a UV photometric detector by taking a sample volume. The receptor medium is completely or in part replaced by fresh medium when taking the sample volume, and the measured amount of active permeated relates to the amount permeated between the two last sampling points and not the total amount permeated so far.

Thus, within the meaning of this invention, the parameter "permeated amount" is provided in μg/cm$^2$ and relates to the amount of active permeated in a sample interval at certain elapsed time. E.g., in an *in vitro* permeation test as described above, wherein the amount of active permeated into the receptor medium has been e.g. measured at hours 0, 2, 4, 8, 12 and 24, the "permeated amount" of active can be given e.g. for the sample interval from hour 8 to hour 12 and corresponds to the measurement at hour 12, wherein the receptor medium has been exchanged completely at hour 8.

The permeated amount can also be given as a "cumulative permeated amount", corresponding to the cumulated amount of active permeated at a certain point in time. E.g., in an *in vitro* permeation test as described above, wherein the amount of active permeated into the receptor medium has been e.g. measured at hours 0, 2, 4, 8, 12 and 24, the "cumulative permeated amount" of active at hour 12 corresponds to the sum of the permeated amounts from hour 0 to hour 2, hour 2 to hour 4, hour 4 to hour 8 and hour 8 to hour 12.

Within the meaning of this invention, the parameter "skin permeation rate" for a certain sample interval at certain elapsed time is provided in μg/(cm$^2$*h) and is calculated from the permeated amount in said sample interval as measured by *in vitro* permeation test as described above in μg/cm$^2$, divided by the hours of said sample interval. E.g. the skin permeation rate in an *in vitro* permeation test as described above, wherein the amount of active permeated into the receptor medium has been e.g. measured at hours 0, 2, 4, 8, 12 and 24, the "skin permeation rate" at hour 12 is calculated as the permeated amount in the sample interval from hour 8 to hour 12 divided by 4 hours.

A "cumulative skin permeation rate" can be calculated from the respective cumulative permeated amount by dividing the cumulative permeated amount by the elapsed time. E.g. in an *in vitro* permeation test as described above, wherein the amount of active permeated into the receptor medium has been e.g. measured at hours 0, 2, 4, 8, 12 and 24, the "cumulative skin permeation rate" at hour 12 is calculated as the cumulative permeated amount for hour 12 (see above) divided by 12 hours.

Within the meaning of this invention, the above parameters "permeated amount" and "skin permeation rate" (as well as "cumulative permeated amount" and "cumulative skin permeation rate") refer to mean values calculated from at least 3 *in vitro* permeation test experiments. Where not otherwise indicated, the standard deviation (SD) of these mean values refer to a corrected sample standard deviation, calculated using the formula:

$$SD = \sqrt{\frac{1}{n-1}\sum_{i=1}^{n}(x_i - \overline{x})^2}$$

wherein n is the sample size, $\{x_1, x_2, \ldots x_n\}$ are the observed values and $\overline{x}$ is the mean value of the observed values.

The TTS according to the present invention can also be characterized by certain parameters as measured in an *in vivo* clinical study.

Within the meaning of this invention, the parameter "mean release rate" refers to the mean release rate in μg/h (μg/hour) or in mg/day over the period of administration (e.g., 1 to 7 days) by which the active agent is released through the human skin into the systemic circulation and is based on the AUC obtained over said period of administration in a clinical study.

Within the meaning of this invention, the term "extended period of time" relates to a period of at least or about 24 hours, at least or about 48 hours, at least or about 84 hours, at least or about 168 hours, at least or about 1 day, at least or about 3.5 days, or at least or about 7 days, or to a period of about 24 hours to about 168 hours or 1 to 7 day(s), or about 24 hours to about 84 hours or 1 to 3.5 day(s).

For a continuous drug treatment, the frequency of drug administration is preferably kept sufficiently high so as to maintain therapeutically effective blood plasma concentration. In other words, the interval between two dosage form administrations, also called dosing interval, needs to be adapted accordingly. Within the meaning of the present invention, the term "dosing interval" refers to the period of time between two consecutive TTS administrations, i.e. the interval between two consecutive points in time a TTS is applied to the skin of the patient. Once applied, the TTS is usually maintained on the skin of the patient for the entire dosing interval and only removed at the end of the dosing interval, at which time a new TTS is applied to the skin. E.g., if the dosing interval is 24 hours or 1 day, the TTS is applied to and maintained on the skin of the patient for 24 hours or 1 day. After 24 hours or 1 day, the TTS is removed from the skin and a new TTS is applied. Thus, a dosing interval of 24 hours or 1 day allows a daily TTS exchange mode in an around-the-clock treatment.

Within the meaning of this invention, the term "room temperature" refers to the unmodified temperature found indoors in the laboratory where the experiments are conducted and usually lies within 15 to 35 °C, preferably about 18 to 25 °C.

Within the meaning of this invention, the term "patient" refers to a subject who has presented a clinical manifestation of a particular symptom or symptoms suggesting the need for treatment, who is treated preventatively or prophylactically for a condition, or who has been diagnosed with a condition to be treated.

Within the meaning of this invention the term "pharmacokinetic parameters" refers to parameters describing the blood plasma curve, e.g. $C_{max}$, $C_t$ and $AUC_{t1-t2}$ obtained in a clinical study, e.g. by single-dose, multi-dose or steady state administration of the active agent-containing TTS, e.g. the rivastigmine-containing TTS to healthy human subjects. The pharmacokinetic parameters of the individual subjects are summarized using arithmetic and geometric means, e.g. a mean $C_{max}$, a mean AUCt and a mean AUCINF, and additional statistics such as the respective standard deviations and standard errors, the minimum value, the maximum value, and the middle value when the list of values is ranked (Median). In the context of the present invention, pharmacokinetic parameters, e.g. the $C_{max}$, Ct and $AUC_{t1-t2}$ refer to geometric mean values if not indicated otherwise. It cannot be precluded that the absolute mean values obtained for a certain TTS in a clinical study vary to a certain extent from study to study. To allow a comparison of absolute mean values between studies, a reference formulation, e.g. in the future any product based on the invention, may be used as internal standard. A comparison of the AUC per area of release of the respective reference product in the earlier and later study can be used to obtain a correction factor to take into account differences from study to study.

Clinical studies according to the present invention refer to studies performed in full compliance with the International Conference for Harmonization of Clinical Trials (ICH) and all applicable local Good Clinical Practices (GCP) and regulations.

Within the meaning of this invention, the term "healthy human subject" refers to a male or female subject with a body weight ranging from 55 kg to 100 kg and a body mass index (BMI) ranging from 18 to 29.4 and normal physiological parameters, such as blood pressure, etc. Healthy human subjects for the purposes of the present invention are selected according to inclusion and exclusion criteria which are based on and in accordance with recommendations of the ICH.

Within the meaning of this invention, the term "subject population" refers to at least five, preferably at least ten individual healthy human subjects.

Within the meaning of this invention, the term "geometric mean" refers to the mean of the log transformed data back-transformed to the original scale.

Within the meaning of this invention, the term "arithmetic mean" refers to the sum of all values of observation divided by the total number of observations.

Within the meaning of this invention, the parameter "AUC" corresponds to the area under the plasma concentration-time curve. The AUC value is proportional to the amount of active agent absorbed into the blood circulation in total and is hence a measure for the bioavailability.

Within the meaning of this invention, the parameter "$AUC_{t1-t2}$" is provided in (ng / ml) h and relates to the area under the plasma concentration-time curve from hour t1 to t2 and is calculated by the linear trapezoidal method, unless otherwise indicated. Other calculation methods are e.g. the logarithmic and linear log trapezoidal method.

Within the meaning of this invention, the parameter "$C_{max}$" is provided in (ng / ml) and relates to the maximum observed blood plasma concentration of the active agent.

Within the meaning of this invention, the parameter "Ct" is provided in (ng / ml) and relates to the blood plasma concentration of the active agent observed at hour t.

Within the meaning of this invention, the parameter "$t_{max}$" is provided in hours and relates to the time point at which the $C_{max}$ value is reached. In other words, $t_{max}$ is the time point of the maximum observed plasma concentration.

Within the meaning of this invention, the term "mean plasma concentration" is provided in (ng / ml) and is a mean of the individual plasma concentrations of active agent, e.g. rivastigmine, at each point in time.

Within the meaning of this invention, the term "coating composition" refers to a composition comprising all components of the matrix layer in a solvent, which may be coated onto the backing layer or release liner to form the matrix layer upon drying.

Within the meaning of this invention, the term "pressure sensitive adhesive composition" refers to a pressure sensitive adhesive at least in mixture with a solvent (e.g. n-heptane or ethyl acetate).

Within the meaning of this invention, the term "dissolve" refers to the process of obtaining a solution, which is clear and does not contain any particles, as visible to the naked eye.

Within the meaning of this invention, the term "solvent" refers to any liquid substance, which preferably is a volatile organic liquid such as methanol, ethanol, isopropanol, acetone, ethyl acetate, methylene chloride, hexane, n-heptane, toluene and mixtures thereof.

## BRIEF DESCRIPTION OF THE DRAWINGS

Fig. 1 depicts the rivastigmine cumulative permeated amount of TTS prepared according to Examples 1a, 1b, 2a and 2b as well as Comparative Example 1 (Exelon®).

Fig. 2 depicts the rivastigmine cumulative permeated amount of TTS prepared according to Examples 3a, 3b, 4a and 4b as well as Comparative Example 1 (Exelon®).

Fig. 3 depicts the buprenorphine cumulative permeated amount of TTS prepared according to Example 5 and Comparative Example 2.

Fig. 4 depicts the buprenorphine cumulative permeated amount of TTS prepared according to Examples 6a and 6b and Comparative Examples 3a and 3b.

## DETAILED DESCRIPTION

### TTS STRUCTURE

The present invention relates to a transdermal therapeutic system for the transdermal administration of an active agent comprising an active agent-containing layer structure, said active agent-containing layer structure comprising:

A) a backing layer;
B) an active agent-containing layer comprising at least one silicone-containing polymer; and
C) a skin contact layer;

wherein the skin contact layer is an adhesive layer comprising a silicone gel adhesive, which is directly attached to the active agent-containing layer.

The TTS according to the present invention may be a matrix-type TTS or a reservoir-type TTS. Thus, the active agent-containing layer may preferably be a matrix layer or a reservoir layer. Preferably, the TTS according to the present invention is a matrix-type TTS, wherein the active agent is homogeneously dissolved and/or dispersed within a polymeric carrier, i.e. the matrix, which forms the together with the active agent and optionally further ingredients a matrix layer. Accordingly, it is preferred that the active agent-containing layer is an active agent-containing matrix layer. Thus, in a preferred embodiment of the TTS according to the present invention, the active agent-containing layer is an active agent-containing matrix layer comprising

- the active agent; and
- the at least one silicone-containing polymer.

Thus, in a preferred embodiment, the present invention relates to a transdermal therapeutic system for the transdermal administration of an active agent comprising an active agent-containing layer structure, said active agent-containing layer structure comprising

A) a backing layer;
B) an active agent-containing matrix layer comprising

- an active agent; and
- at least one silicone-containing polymer; and

C) a skin contact layer;

wherein the skin contact layer is an adhesive layer comprising a silicone gel adhesive, which is directly attached to the

active agent-containing layer.

The at least one silicone-containing polymer is a hybrid or non-hybrid polymer comprising polysiloxanes. It is preferably a thermoplastic polymer, which is applied by hot-melt or a solvent based process and typically does not undergo further curing to solidify. In a preferred embodiment, the at least one silicone-containing polymer is a silicone acrylic hybrid polymer, silicone-based polymer, or a combination thereof. Further details regarding these polymers are provided below. In a particularly preferred embodiment of the invention, the at least one silicone-containing polymer in the TTS is a silicone acrylic hybrid polymer. Optionally, further polymers, in particular non-hybrid polymers, such as acrylic polymers or silicone-based polymers may additionally be present.

Thus, in a preferred embodiment, the present invention relates to a transdermal therapeutic system for the transdermal administration of an active agent comprising an active agent-containing layer structure, said active agent-containing layer structure comprising

A) a backing layer;
B) an active agent-containing matrix layer comprising

- an active agent; and
- at least one non-curing silicone-containing polymer; and

C) a skin contact layer;

wherein the skin contact layer is an adhesive layer comprising a silicone gel adhesive, which is directly attached to the active agent-containing layer.

In another preferred embodiment, the present invention relates to a transdermal therapeutic system for the transdermal administration of an active agent comprising an active agent-containing layer structure, said active agent-containing layer structure comprising

A) a backing layer;
B) an active agent-containing matrix layer comprising

- an active agent; and
- at least one silicon acrylic hybrid polymer; and

C) a skin contact layer;

wherein the skin contact layer is an adhesive layer comprising a silicone gel adhesive, which is directly attached to the active agent-containing layer.

The TTS according to the present invention comprises a skin contact layer. The skin contact layer is an adhesive layer comprising a silicone gel adhesive, which is directly attached to the active agent-containing layer. Thus, the TTS according to the present invention comprises at least two layers. The silicone gel adhesive in the skin contact layer provides for the adhesive properties, while at the same time reducing the problem of skin irritation. Typically, the skin contact layer is manufactured active-free. However, due to the concentration gradient, the active agent may migrate from the active agent-containing layer to the skin contact layer over time, until an equilibrium is reached.

The silicone gel adhesive is an elastic, jelly-like material formed by lightly crosslinking silicone polymers. It may be prepared from a gel producing composition as described further below upon curing. Thus, while the silicone-containing polymer as used in the active agent-containing layer of the TTS of the present invention is preferably a non-curing polymer, i.e. a polymer, which does not undergo a curing reaction to solidify, the silicone gel adhesive forms upon curing of silicones comprising reactive groups such as Si-H reactive groups and aliphatic unsaturated groups, which react with each other in the presence of a hydrosilylation catalyst. Further details in this regard are provided below.

Thus, in a preferred embodiment, the present invention relates to a transdermal therapeutic system for the transdermal administration of an active agent comprising an active agent-containing layer structure, said active agent-containing layer structure comprising

A) a backing layer;
B) an active agent-containing matrix layer comprising

- an active agent; and
- at least one non-curing silicone-containing polymer; and

C) a skin contact layer;

wherein the skin contact layer is an adhesive layer comprising a silicone gel adhesive, which is directly attached to the active agent-containing layer,
and wherein the silicone gel adhesive is obtained from curable silicones comprising reactive groups, in particular Si-H groups and aliphatic unsaturated groups.

In another preferred embodiment, the present invention relates to a transdermal therapeutic system for the transdermal administration of an active agent comprising an active agent-containing layer structure, said active agent-containing layer structure comprising

A) a backing layer;
B) an active agent-containing matrix layer comprising

- an active agent; and
- at least one silicone acrylic hybrid polymer; and

C) a skin contact layer;

wherein the skin contact layer is an adhesive layer comprising a silicone gel adhesive, which is directly attached to the active agent-containing layer,
and wherein the silicone gel adhesive is obtained from curable silicones comprising reactive groups, in particular Si-H groups and aliphatic unsaturated groups.

It has surprisingly been found that good adhesion between the active agent-containing layer and the skin contact layer can be achieved according to the present invention. This is due to the content of the (non-curing) silicone-containing polymer in the active agent-containing layer. It has been found that it is advantageous if the (non-curing) silicone-containing polymer is present in the active agent-containing layer in an amount of at least 20 % by weight, preferably at least 60 % by weight. In a preferred embodiment, the at least one (non-curing) silicone-containing polymer is present in the active agent-containing layer in an amount of from 20 to 90 % by weight, preferably 60 to 90 % by weight, based on the total weight of the active agent-containing layer.

If the silicone-containing polymer is a silicone acrylic hybrid polymer, it is preferred that the silicone acrylic hybrid polymer is present in the active agent-containing layer in an amount of at least 20 % by weight, preferably at least 60 % by weight. In a preferred embodiment, the silicone acrylic hybrid polymer is present in the active agent-containing layer in an amount of from 20 to 90 % by weight, preferably 60 to 90 % by weight, based on the total weight of the active agent-containing layer.

It is to be understood that the TTS according to the invention contains a therapeutically effective amount of the active agent. Preferred active agents include rivastigmine and buprenorphine. In a preferred embodiment, the active agent is present in the active agent-containing layer in an amount of from 5 to 25 % by weight, preferably 8 to 22 % by weight, based on the total weight of the active agent-containing layer.

The area of release of the TTS according to the invention may be from 1 to 30 $cm^2$, preferably from 2 to 22 $cm^2$.

The backing layer is preferably substantially impermeable for the active agent. In a preferred embodiment, the backing layer is occlusive as outlined above.

According to certain embodiments of the invention, the TTS may further comprise an adhesive overlay. This adhesive overlay is in particular larger in area than the active agent-containing structure and is attached thereto for enhancing the adhesive properties of the overall transdermal therapeutic system. Said adhesive overlay comprises a backing layer and an adhesive layer. The adhesive overlay provides additional area adhering to the skin but does not add to the area of release of the active agent. The adhesive overlay comprises a self-adhesive polymer or a self-adhesive polymer mixture selected from the group consisting of silicone acrylic hybrid polymers, acrylic polymers, silicone-based polymers, polyisobutylenes, styrene-isoprene-styrene copolymers, and mixtures thereof, which may be identical to or different from any polymer or polymer mixture included in the rivastigmine-containing layer structure.

The active agent-containing layer structure according to the invention is normally located on a detachable protective layer (release liner), from which it is removed immediately before application to the surface of the patient's skin. Thus, the TTS may further comprise a release liner. A TTS protected this way is usually stored in a blister pack or a seam-sealed pouch. The packaging may be child resistant and/or senior friendly.

**ACTIVE AGENT-CONTAINING LAYER**

As outlined in more detail above, the TTS according to the present invention comprises an active agent-containing layer structure comprising inter alia an active agent-containing layer, which comprises at least one silicone-containing polymer.

In a preferred embodiment, the active agent-containing layer comprises a therapeutically affective amount of the active agent. In a particular preferred embodiment, the active agent is present in the active agent-containing layer in an amount of from 5 to 25 % by weight, preferably 8 to 22 % by weight, based on the total weight of the active agent-containing layer. Preferred active agents include rivastigmine and buprenorphine.

The active agent is preferably homogeneously distributed within the active agent-containing layer. In a preferred embodiment, the active agent-containing layer is therefore an active agent-containing matrix layer.

The silicone-containing polymer is preferably a non-curing hybrid or non-hybrid polymer comprising at least one polysiloxane moiety. In a preferred embodiment, the at least one silicone-containing polymer is a silicone acrylic hybrid polymer, silicone-based polymer, or a combination thereof. Further details regarding these polymers are provided below. In a preferred embodiment, the at least one silicone-containing polymer is present in the active agent-containing layer in an amount of from 20 to 90 % by weight, preferably 60 to 90 % by weight, based on the total weight of the active agent-containing layer. It is to be understood that the aforementioned weight percent amounts refer to the overall amount of the at least one silicone-containing polymer. For example, if two silicone-containing polymers are present, the overall amount in the active agent-containing layer is from 20 to 90 % by weight, preferably 60 to 90 % by weight, based on the total weight of the active agent-containing layer.

In one preferred embodiment, the at least one silicone-containing polymer is a silicone-based polymer, which is preferably present in an amount of from 20 to 90 % by weight, especially in an amount of from 60 to 90 % by weight, based on the total weight of the active agent-containing layer. In another preferred embodiment, the at least one silicone-containing polymer is a silicone acrylic hybrid polymer. Preferably, the silicone acrylic hybrid polymer is present in an amount of from 20 to 90 % by weight, especially in an amount of from 60 to 90 % by weight, based on the total weight of the active agent-containing layer. In a particularly preferred embodiment of the invention, the at least one silicone-containing polymer in the TTS is a silicone acrylic hybrid polymer, which preferably comprises a silicone phase and an acrylate phase in a weight ratio of from 20:80 to 90:10.

In addition to the silicone-containing polymer, the active agent-containing layer may further comprise a non-silicone-containing polymer such as an acrylic polymer. In a preferred embodiment, the active agent-containing layer optionally further comprises at least one acrylic polymer in an amount of from 5 to 80 % by weight, preferably 5 to 20 % by weight, based on the total weight of the active agent-containing layer. Preferably, the acrylic polymer is obtainable from one or more monomers selected from acrylic acid, butylacrylate, 2-ethylhexylacrylate, glycidylmethacrylate, 2-hydroxyethylacrylate, methylacrylate, methylmethacrylate, butylmethacrylate, t-octylacrylamide, and vinylacetate, preferably from one or more monomers selected from ethylhexylacrylate, glycidylmethacrylate, 2-hydroxyethylacrylate, and vinylacetate.

In view of the above, it can be preferred that the active agent-containing layer comprises either a silicone-containing polymer alone, a combination of silicone-containing polymers or a combination of at least one silicone-containing polymer and at least one non-silicone-containing polymer such as an acrylic polymer. In a preferred embodiment, the active agent-containing layer comprises

- a silicone acrylic hybrid polymer, or
- a polymer blend comprising a silicone-based polymer and an acrylic polymer, or
- a polymer blend comprising a silicone-based polymer and a silicone acrylic hybrid polymer, or
- a polymer blend comprising a silicone acrylic hybrid polymer and an acrylic polymer.

In connection with the silicone acrylic hybrid polymer, it is preferred that the silicone acrylic hybrid polymer comprises a silicone phase and an acrylate phase in a weight ratio of from 20:80 to 90:10, more preferably from 40:60 to 60:40. In connection with the polymer blend comprising a silicone-based polymer and an acrylic polymer, it is preferred that the silicone-based polymer is present in an amount of at least 20 % by weight based on the total weight of the polymer blend, more preferably in an amount of from 50 to 90 % by weight. In connection with the polymer blend comprising a silicone-based polymer and a silicone acrylic hybrid polymer, the weight ratios may vary over a broad range. However, it is preferred that the silicone acrylic hybrid polymer is present in an amount of at least 20 % by weight based on the total weight of the polymer blend, more preferably in an amount of from 50 to 90 % by weight. In connection with the polymer blend comprising a silicone acrylic hybrid polymer and an acrylic polymer, the weight ratios may vary over a broad range. However, it is preferred that that the silicone acrylic hybrid polymer is present in an amount of at least 20 % by weight based on the total weight of the polymer blend, more preferably in an amount of from 50 to 90 % by weight.

In a more preferred embodiment, the active agent-containing layer comprises

- a silicone acrylic hybrid polymer, wherein preferably the silicone acrylic hybrid polymer comprises a silicone phase and an acrylate phase in a weight ratio of from 20:80 to 90:10, or
- a polymer blend comprising a silicone-based polymer and an acrylic polymer, wherein the silicone-based polymer is present in an amount of at least 20 % by weight based on the total weight of the polymer blend, or
- a polymer blend comprising a silicone-based polymer and a silicone acrylic hybrid polymer, or
- a polymer blend comprising a silicone acrylic hybrid polymer and an acrylic polymer, wherein the silicone acrylic hybrid polymer is present in an amount of at least 20 % by weight based on the total weight of the polymer blend.

In an even more preferred embodiment, the active agent-containing layer comprises

- a silicone acrylic hybrid polymer, wherein preferably the silicone acrylic hybrid polymer comprises a silicone phase and an acrylate phase in a weight ratio of from 20:80 to 90:10, or
- a polymer blend comprising a silicone-based polymer and an acrylic polymer, wherein the silicone-based polymer is present in an amount of from 50 to 90 % by weight based on the total weight of the polymer blend, or
- a polymer blend comprising a silicone-based polymer and a silicone acrylic hybrid polymer, wherein the silicone acrylic hybrid polymer is present in an amount of from 50 to 90 % by weight based on the total weight of the polymer blend, and wherein preferably the silicone acrylic hybrid polymer comprises a silicone phase and an acrylate phase in a weight ratio of from 20:80 to 90:10, or
- a polymer blend comprising a silicone acrylic hybrid polymer and an acrylic polymer, wherein the silicone acrylic hybrid polymer is present in an amount of from 50 to 90 % by weight based on the total weight of the polymer blend, and wherein preferably the silicone acrylic hybrid polymer comprises a silicone phase and an acrylate phase in a weight ratio of from 20:80 to 90:10.

In an even more preferred embodiment, the active agent-containing layer comprises

- a silicone acrylic hybrid polymer, wherein the silicone acrylic hybrid polymer comprises a silicone phase and an acrylate phase in a weight ratio of from 60:40 to 40:60, or
- a polymer blend comprising a silicone-based polymer and an acrylic polymer, wherein the silicone-based polymer is present in an amount of from 50 to 90 % by weight based on the total weight of the polymer blend, or
- a polymer blend comprising a silicone-based polymer and a silicone acrylic hybrid polymer, wherein the silicone acrylic hybrid polymer is present in an amount of from 50 to 90 % by weight based on the total weight of the polymer blend, and wherein the silicone acrylic hybrid polymer comprises a silicone phase and an acrylate phase in a weight ratio of from 60:40 to 40:60, or
- a polymer blend comprising a silicone acrylic hybrid polymer and an acrylic polymer, wherein the silicone acrylic hybrid polymer is present in an amount of from 50 to 90 % by weight based on the total weight of the polymer blend, and wherein the silicone acrylic hybrid polymer comprises a silicone phase and an acrylate phase in a weight ratio of from 60:40 to 40:60.

In one embodiment of the invention, the area weight of the active agent-containing layer ranges from 40 to 250 $g/m^2$, preferably from 50 to 200 $g/m^2$. In another embodiment of the invention, the active agent-containing layer has a thickness of from 50 to 150 $\mu$m.

**SKIN CONTACT LAYER**

As outlined in more detail above, the TTS according to the present invention comprises an active agent-containing layer structure comprising inter alia a skin contact layer, wherein the skin contact layer is an adhesive layer comprising a silicone gel adhesive. The skin contact layer is directly attached to the active agent-containing layer.

The skin contact layer is typically provided free of active agent. However, due to the concentration gradient, the active agent may migrate from the active agent-containing layer to the skin contact layer over time, until an equilibrium is reached.

The silicone gel adhesive in the skin contact layer is decisive for the adhesive properties as well as the reduction of skin irritation inter alia due to its resiliency. Furthermore, the silicone gel adhesive does not negatively affect the transdermal delivery of the active agent. In a preferred embodiment the skin contact layer comprises the silicone gel adhesive in an amount of at least 95 % by weight, preferably at least 99 % by weight. Particularly preferably, the skin contact layer essentially consists of the silicone gel adhesive.

The silicone gel adhesive is an elastic, jelly-like material formed by lightly crosslinking silicone polymers provided as a so-called gel producing composition. The silicone gel adhesives are generally formed from linear or branched silicones having reactive groups thereon. Such reactive groups undergo a crosslinking reaction during curing. Examples of

crosslinking reactions include the hydrosilylation reaction in which a silicone having an Si-H reactive group reacts with a silicone having an aliphatic unsaturated reactive group in the presence of a hydrosilylation catalyst. Typically, the silicone gel adhesive is obtainable by reacting a gel producing composition comprising (i) at least one alkenyl-substituted polydiorganosiloxane, (ii) at least one organosiloxane, which contains silicone-bonded hydrogen atoms, and (iii) at least one catalyst for the reaction of the Si-H groups with the Si-alkenyl groups. Preferably, the silicone gel adhesive is obtainable by reacting a gel producing composition comprising (i) a copolymer of vinylmethylsiloxane and dimethylsiloxane with (ii) methylhydrogen polysiloxane with trimethylsilyl endgroups in the presence of (iii) a platinum catalyst. Further details in this regard are provided below.

In a preferred embodiment, the skin contact layer has a thickness of from 30 to 220 μm, preferably from 40 to 160 μm. In another preferred embodiment, the skin contact layer has an area weight of from 20 to 120 g/$m^2$, preferably from 30 to 90 g/$m^2$.

## SILICONE ACRYLIC HYBRID POLYMER

As indicated above, the TTS according to the present invention comprises at least one silicone-containing polymer in the active agent-containing layer. In a preferred embodiment, the at least one silicone-containing polymer is a silicone acrylic hybrid polymer, a silicone-based polymer, or a combination thereof. The silicone acrylic hybrid polymer is described in further detail hereinafter.

The silicone acrylic hybrid polymer comprises a polymerized hybrid species that includes silicone-based sub-species and acrylate-based sub-species that have been polymerized together. The silicone acrylic hybrid polymer thus comprises a silicone phase and an acrylic phase. In view of the polysiloxane content in the silicone phase, it is considered to be a silicone-containing polymer in the context of the present invention. Preferably, the silicone acrylic hybrid polymer is a silicone acrylic hybrid pressure-sensitive adhesive.

The silicone acrylic hybrid pressure-sensitive adhesives are usually supplied and used in solvents like n-heptane and ethyl acetate. The solids content of the pressure-sensitive adhesives is usually between 30 % and 80 %. The skilled person is aware that the solids content may be modified by adding a suitable amount of solvent.

Preferably, the weight ratio of silicone to acrylate in the silicone acrylic hybrid pressure-sensitive adhesive is from 5:95 to 95:5, or from 20:80 to 80:20, more preferably from 40:60 to 60:40, and most preferably the ratio of silicone to acrylate is about 50:50. Suitable silicone acrylic hybrid pressure-sensitive adhesives having a weight ratio of silicone to acrylate of 50:50 are, for example, the commercially available silicone acrylic hybrid pressure-sensitive adhesives 7-6102, Silicone/Acrylate Ratio 50/50, and 7-6302, Silicone/Acrylate Ratio 50/50, supplied in ethyl acetate by Dow Corning.

The preferred silicone acrylic hybrid pressure-sensitive adhesives in accordance with the invention are characterized by a solution viscosity at 25 °C and about 50 % solids content in ethyl acetate of more than about 400 cP, or from about 500 cP to about 3,500 cP, in particular from about 1,000 cP to about 3,000 cP, more preferred from about 1,200 cP to about 1,800, or most preferred of about 1,500 cP or alternatively more preferred from about 2,200 cP to about 2,800 cP, or most preferred of about 2,500 cP, preferably as measured using a Brookfield RVT viscometer equipped with a spindle number 5 at 50 RPM.

These silicone acrylic hybrid pressure-sensitive adhesives may also be characterized by a complex viscosity at 0.1 rad/s at 30 °C of less than about 1.0e9 Poise, or from about 1.0e5 Poise to about 9.0e8 Poise, or more preferred from about 9.0e5 Poise to about 1.0e7 Poise, or most preferred about 4.0e6 Poise, or alternatively more preferred from about 2.0e6 Poise to about 9.0e7 Poise, or most preferred about 1.0e7 Poise, preferably as measured using a Rheometrics ARES rheometer, wherein the rheometer is equipped with 8 mm plates and the gap zeroed.

To prepare samples for measuring the rheological behavior using a Rheometrics ARES rheometer, between 2 and 3 grams of adhesive solution can be poured onto a SCOTCH-PAK 1022 fluoropolymer release liner and allow to sit for 60 minutes under ambient conditions. To achieve essentially solvent-free films of the adhesive, they can be placed in an oven at 110 °C +/-10 °C for 60 minutes. After removing from the oven and letting equilibrate to room temperature. The films can be removed from the release liner and folded over to form a square. To eliminate air bubbles the films can be compressed using a Carver press. The samples can then be loaded between the plates and are compressed to 1.5 +/-0.1 mm at 30 °C. The excess adhesive is trimmed and the final gap recorded. A frequency sweep between 0.01 to 100 rad/s can be performed with the following settings: Temperature = 30 °C; strain = 0.5-1 % and data collected at 3 points/decade.

Suitable silicone acrylic hybrid pressure-sensitive adhesives which are commercially available include the PSA series 7-6100 and 7-6300 manufactured and supplied in n-heptane or ethyl acetate by Dow Corning (7-610X and 7-630X; X=1 n-heptane-based / X=2 ethyl acetate-based). For example, the 7-6102 silicone acrylic hybrid PSA having a silicone/acrylate ratio of 50/50 is characterized by a solution viscosity at 25 °C and about 50 % solids content in ethyl acetate of 2,500 mPa.s and a complex viscosity at 0.1 rad/s at 30 °C of 1.0e6 Pa.s. The 7-6302 silicone acrylic hybrid PSA having a silicone/acrylate ratio of 50/50 has a solution viscosity at 25 °C and about 50 % solids content in ethyl acetate of 1,500 mPa.s and a complex viscosity at 0.1 rad/s at 30 °C of 4.0e5 Pa.s.

Depending on the solvent in which the silicone acrylic hybrid pressure-sensitive adhesive is supplied, the

arrangement of the silicone phase and the acrylic phase providing a silicone or acrylic continuous external phase and a corresponding discontinuous internal phase is different. If the silicone acrylic hybrid pressure-sensitive adhesive is provided in n-heptane, the composition contains a continuous, silicone external phase and a discontinuous, acrylic internal phase. If the silicone acrylic hybrid pressure-sensitive adhesive is provided in ethyl acetate, the composition contains a continuous, acrylic external phase and a discontinuous, silicone internal phase. After evaporating the solvent in which the silicone acrylic hybrid pressure-sensitive adhesive is provided, the phase arrangement of the resulting pressure-sensitive adhesive film or layer corresponds to the phase arrangement of the solvent-containing adhesive coating composition. For example, in the absence of any substance that may induce an inversion of the phase arrangement in a silicone acrylic hybrid pressure sensitive adhesive composition, a pressure-sensitive adhesive layer prepared from a silicone acrylic hybrid pressure-sensitive adhesive in n-heptane provides a continuous, silicone external phase and a discontinuous, acrylic internal phase, a pressure-sensitive adhesive layer prepared from a silicone acrylic hybrid pressure-sensitive adhesive in ethyl acetate provides a continuous, acrylic external phase and a discontinuous, silicone internal phase. The phase arrangement of the compositions can, for example, be determined in peel force tests with pressure-sensitive adhesive films or layers prepared from the silicone acrylic hybrid PSA compositions which are attached to a siliconized release liner. The pressure-sensitive adhesive film contains a continuous, silicone external phase if the siliconized release liner cannot or can only hardly be removed from the pressure-sensitive adhesive film (laminated to a backing film) due to the blocking of the two silicone surfaces. Blocking results from the adherence of two silicone layers which comprise a similar surface energy. The silicone adhesive shows a good spreading on the siliconized liner and therefore can create a good adhesion to the liner. If the siliconized release liner can easily be removed the pressure-sensitive adhesive film contains a continuous, acrylic external phase. The acrylic adhesive has no good spreading due to the different surface energies and thus has a low or almost no adhesion to the siliconized liner.

According to a preferred embodiment of the invention, the silicone acrylic hybrid polymer is a silicone acrylic hybrid pressure-sensitive adhesive obtainable from a silicon-containing pressure-sensitive adhesive composition comprising acrylate or methacrylate functionality. It is to be understood that the silicon-containing pressure-sensitive adhesive composition comprising acrylate or methacrylate functionality can include only acrylate functionality, only methacrylate functionality, or both acrylate functionality and methacrylate functionality.

According to certain embodiments of the invention, the silicone acrylic hybrid pressure-sensitive adhesive comprises the reaction product of (a) a silicon-containing pressure-sensitive adhesive composition comprising acrylate or methacrylate functionality, (b) an ethylenically unsaturated monomer, and (c) an initiator. That is, the silicone acrylic hybrid pressure-sensitive adhesive is the product of the chemical reaction between these reactants ((a), (b), and (c)). In particular, the silicone acrylic hybrid pressure-sensitive adhesive includes the reaction product of (a) a silicon-containing pressure-sensitive adhesive composition comprising acrylate or methacrylate functionality, (b) a (meth)acrylate monomer, and (c) an initiator (i.e., in the presence of the initiator). That is, the silicone acrylic hybrid pressure-sensitive adhesive includes the product of the chemical reaction between these reactants ((a), (b), and (c)).

The reaction product of (a) a silicon-containing pressure-sensitive adhesive composition comprising acrylate or methacrylate functionality, (b) an ethylenically unsaturated monomer, and (c) an initiator may contain a continuous, silicone external phase and a discontinuous, acrylic internal phase or the reaction product of (a), (b), and (c) may contain a continuous, acrylic external phase and a discontinuous, silicone internal phase.

The silicon-containing pressure-sensitive adhesive composition comprising acrylate or methacrylate functionality (a) is typically present in the silicone acrylic hybrid pressure-sensitive adhesive in an amount of from 5 to 95, more typically 25 to 75, parts by weight based on 100 parts by weight of the hybrid pressure-sensitive adhesive.

The ethylenically unsaturated monomer (b) is typically present in the silicone acrylic hybrid pressure-sensitive adhesive in an amount of from 5 to 95, more typically 25 to 75, parts by weight based on 100 parts by weight of the hybrid pressure-sensitive adhesive.

The initiator (c) is typically present in the silicone acrylic hybrid pressure-sensitive adhesive in an amount of from 0.005 to 3, more typically from 0.01 to 2, parts by weight based on 100 parts by weight of the hybrid pressure-sensitive adhesive.

According to certain embodiments of the invention the silicon-containing pressure-sensitive adhesive composition comprising acrylate or methacrylate functionality (a) comprises the condensation reaction product of (a1) a silicone resin, (a2) a silicone polymer, and (a3) a silicon-containing capping agent which provides said acrylate or methacrylate functionality. The silicone resin (a1) may also be referred to as silicate resin or silica resin. Preferably, the silicone polymer (a2) is a polysiloxane, preferably polydimethylsiloxane. It is to be understood that (a1) and (a2) form a silicone-based pressure sensitive adhesive by polycondensation, and that the acrylate or methacrylate functionality is introduced by reaction with (a3).

According to certain embodiments of the invention the silicon-containing pressure-sensitive adhesive composition comprising acrylate or methacrylate functionality (a) comprises the condensation reaction product of:

(a1) a silicone resin,

(a2) a silicone polymer, and

(a3) a silicon-containing capping agent which provides said acrylate or methacrylate functionality, wherein said silicon-containing capping agent is of the general formula $XYR'_bSiZ_{3-b}$, wherein

X is a monovalent radical of the general formula AE-
where E is -O- or -NH- and A is an acryl group or a methacryl group,
Y is a divalent alkylene radical having from 1 to 6 carbon atoms,
R' is a methyl or a phenyl radical,
Z is a monovalent hydrolyzable organic radical or a halogen, and
b is 0 or 1;

wherein the silicone resin and silicone polymer are reacted to form a pressure-sensitive adhesive, wherein the silicon-containing capping agent is introduced prior to, during, or after the silicone resin and silicone polymer are reacted, and wherein:

the silicon-containing capping agent reacts with the pressure-sensitive adhesive after the silicone resin and silicone polymer have been condensation reacted to form the pressure-sensitive adhesive; or
the silicon-containing capping agent reacts *in-situ* with the silicone resin and silicone polymer.

According to certain embodiments of the invention the silicon-containing pressure-sensitive adhesive composition comprising acrylate or methacrylate functionality comprises the condensation reaction product of a pressure sensitive adhesive and a silicon-containing capping agent which provides said acrylate or methacrylate functionality. That is, the silicon-containing pressure sensitive adhesive composition comprising acrylate or methacrylate functionality is essentially a pressure sensitive adhesive that has been capped or end blocked with the silicon-containing capping agent which provides said acrylate or methacrylate functionality, wherein the pressure sensitive adhesive comprises the condensation reaction product of the silicone resin and the silicone polymer. Preferably, the silicone resin reacts in an amount of from 30 to 80 parts by weight to form the pressure sensitive adhesive, and the silicone polymer reacts in an amount of from 20 to 70 parts by weight to form the pressure sensitive adhesive. Both of these parts by weight are based on 100 parts by weight of the pressure sensitive adhesive. Although not required, the pressure sensitive adhesive may comprise a catalytic amount of a condensation catalyst. A wide array of silicone resins and silicone polymers are suitable to make up the pressure sensitive adhesive.

According to certain embodiments of the invention the silicone acrylic hybrid pressure-sensitive adhesive is the reaction product of:

(a) a silicon-containing pressure-sensitive adhesive composition comprising acrylate or methacrylate functionality that comprises the condensation reaction product of:

(a1) a silicone resin,
(a2) a silicone polymer, and
(a3) a silicon-containing capping agent which provides said acrylate or methacrylate functionality, wherein said silicon-containing capping agent is of the general formula $XYR'_bSiZ_{3-b}$, wherein

X is a monovalent radical of the general formula AE-
where E is -O- or -NH- and A is an acryl group or a methacryl group,
Y is a divalent alkylene radical having from 1 to 6 carbon atoms,
R' is a methyl or a phenyl radical,
Z is a monovalent hydrolyzable organic radical or a halogen, and
b is 0 or 1;

wherein the silicone resin and silicone polymer are reacted to form a pressure-sensitive adhesive, wherein the silicon-containing capping agent is introduced prior to, during, or after the silicone resin and silicone polymer are reacted, and wherein:

the silicon-containing capping agent reacts with the pressure-sensitive adhesive after the silicone resin and silicone polymer have been condensation reacted to form the pressure-sensitive adhesive; or
the silicon-containing capping agent reacts in-situ with the silicone resin and silicone polymer;

(b) an ethylenically unsaturated monomer; and

(c) an initiator.

The silicone acrylic hybrid composition used in the present invention may be described by being prepared by a method comprising the steps of:

(i) providing a silicon-containing pressure-sensitive adhesive composition comprising acrylate or methacrylate functionality that comprises the condensation reaction product of:

a silicone resin,
a silicone polymer, and
a silicon-containing capping agent which provides said acrylate or methacrylate functionality,
wherein said silicon-containing capping agent is of the general formula $XYR'_bSiZ_{3-b}$, wherein

X is a monovalent radical of the general formula AE-
where E is -O- or -NH- and A is an acryl group or a methacryl group,
Y is a divalent alkylene radical having from 1 to 6 carbon atoms,
R' is a methyl or a phenyl radical,
Z is a monovalent hydrolyzable organic radical or a halogen, and
b is 0 or 1;

wherein the silicone resin and silicone polymer are reacted to form a pressure-sensitive adhesive, wherein the silicon-containing capping agent is introduced prior to, during, or after the silicone resin and silicone polymer are reacted, and wherein:

the silicon-containing capping agent reacts with the pressure-sensitive adhesive after the silicone resin and silicone polymer have been condensation reacted to form the pressure-sensitive adhesive; or
the silicon-containing capping agent reacts in-situ with the silicone resin and silicone polymer;

(ii) polymerizing an ethylenically unsaturated monomer and the silicon-containing pressure-sensitive adhesive composition comprising acrylate or methacrylate functionality of step (i) in the presence of an initiator to form a silicone acrylic hybrid composition, optionally at a temperature of from 50 °C to 100 °C, or from 65 °C to 90 °C.

During the polymerization of the ethylenically unsaturated monomer and the silicon-containing pressure-sensitive adhesive composition comprising acrylate or methacrylate functionality, the silicone to acrylic ratio can be controlled and optimized as desired. The silicone to acrylic ratio can be controlled by a wide variety of mechanisms in and during the method. An illustrative example of one such mechanism is the rate controlled addition of the ethylenically unsaturated monomer or monomers to the silicon-containing pressure-sensitive adhesive composition comprising acrylate or methacrylate functionality. In certain applications, it may be desirable to have the silicone-based sub-species, or the overall silicone content, to exceed the acrylate-based sub-species, or the overall acrylic content. In other applications, it may be desirable for the opposite to be true. Independent of the end application, it is generally preferred, as already described above, that the silicon-containing pressure-sensitive adhesive composition comprising acrylate or methacrylate functionality is preferably present in the silicone acrylic hybrid composition in an amount of from about 5 to about 95 parts by weight, more preferably from about 25 to about 75 parts by weight, and still more preferably from about 40 to about 60 parts by weight based on 100 parts by weight of the silicone acrylic hybrid composition.

According to a certain embodiment of the invention, the silicone acrylic hybrid composition used in the present invention may be described by being prepared by a method comprising the steps of:

(i) providing a silicon-containing pressure-sensitive adhesive composition comprising acrylate or methacrylate functionality that comprises the condensation reaction product of:

a silicone resin,
a silicone polymer, and
a silicon-containing capping agent which provides said acrylate or methacrylate functionality,
wherein said silicon-containing capping agent is of the general formula $XYR'_bSiZ_{3-d}$, wherein

X is a monovalent radical of the general formula AE-
where E is -O- or -NH- and A is an acryl group or a methacryl group,
Y is a divalent alkylene radical having from 1 to 6 carbon atoms,

R' is a methyl or a phenyl radical,
Z is a monovalent hydrolyzable organic radical or a halogen, and
b is 0 or 1;

wherein the silicone resin and silicone polymer are reacted to form a pressure-sensitive adhesive, wherein the silicon-containing capping agent is introduced prior to, during, or after the silicone resin and silicone polymer are reacted, and wherein:

the silicon-containing capping agent reacts with the pressure-sensitive adhesive after the silicone resin and silicone polymer have been condensation reacted to form the pressure-sensitive adhesive; or
the silicon-containing capping agent reacts in-situ with the silicone resin and silicone polymer;

(ii) polymerizing an ethylenically unsaturated monomer and the silicon-containing pressure-sensitive adhesive composition comprising acrylate or methacrylate functionality of step (i) in a first solvent in the presence of an initiator at a temperature of from 50 °C to 100 °C to form a silicone acrylic hybrid composition;
(iii) removing the first solvent; and
(iv) adding a second solvent to form the silicone acrylic hybrid composition, wherein the phase arrangement of the silicone acrylic hybrid composition is selectively controlled by selection of the second solvent.

The silicone acrylic hybrid PSA composition used in the present invention may also be described by being prepared by a method comprising the steps of:

(i) providing a silicon-containing pressure-sensitive adhesive composition comprising acrylate or methacrylate functionality that comprises the condensation reaction product of:

a silicone resin,
a silicone polymer, and
a silicon-containing capping agent which provides said acrylate or methacrylate functionality,
wherein said silicon-containing capping agent is of the general formula $XYR'_bSiZ_{3-d}$, wherein

X is a monovalent radical of the general formula AE-
where E is -O- or -NH- and A is an acryl group or a methacryl group,
Y is a divalent alkylene radical having from 1 to 6 carbon atoms,
R' is a methyl or a phenyl radical,
Z is a monovalent hydrolyzable organic radical or a halogen, and
b is 0 or 1;

wherein the silicone resin and silicone polymer are reacted to form a pressure-sensitive adhesive, wherein the silicon-containing capping agent is introduced prior to, during, or after the silicone resin and silicone polymer are reacted, and wherein:

the silicon-containing capping agent reacts with the pressure-sensitive adhesive after the silicone resin and silicone polymer have been condensation reacted to form the pressure-sensitive adhesive; or
the silicon-containing capping agent reacts in-situ with the silicone resin and silicone polymer;

(ii) polymerizing an ethylenically unsaturated monomer and the silicon-containing pressure-sensitive adhesive composition comprising acrylate or methacrylate functionality of step (i) in a first solvent in the presence of an initiator at a temperature of from 50 °C to 100 °C to form a silicone acrylic hybrid composition;
(iii) adding a processing solvent, wherein the processing solvent has a higher boiling point than the first solvent, and
(iv) applying heat at a temperature of from 70 °C to 150 °C such that a majority of the first solvent is selectively removed;
(v) removing the processing solvent; and.
(vi) adding a second solvent to form the silicone acrylic hybrid composition, wherein the phase arrangement of the silicone acrylic hybrid composition is selectively controlled by selection of the second solvent.

The silicone resin according to the previous paragraphs may contain a copolymer comprising triorganosiloxy units of the formula $R^X{}_3SiO_{1/2}$ and tetrafunctional siloxy units of the formula $SiO_{4/2}$ in a ratio of from 0.1 to 0.9, preferably of about 0.6 to 0.9, triorganosiloxy units for each tetrafunctional siloxy unit. Preferably, each $R^X$ independently denotes a

monovalent hydrocarbon radical having from 1 to 6 carbon atoms, vinyl, hydroxyl or phenyl groups.

The silicone polymer according to the previous paragraphs may comprise at least one polydiorganosiloxane and is preferably end-capped (end-blocked) with a functional group selected from the group consisting of hydroxyl groups, alkoxy groups, hydride groups, vinyl groups, or mixtures thereof . The diorganosubstituent may be selected from the group consisting of dimethyl, methylvinyl, methylphenyl, diphenyl, methylethyl, (3,3,3-trifluoropropyl)methyl and mixtures thereof. Preferably, the diorganosubstituents contain only methyl groups. The molecular weight of polydiorganosiloxane will typically range from about 50,000 to about 1,000,000, preferably, from about 80,000 to about 300,000. Preferably, the polydiorganosiloxane comprises $AR^XSiO$ units terminated with endblocking $TR^XASiO_{1/2}$ units, wherein the poly-diorganosiloxane has a viscosity of from about 100 centipoise to about 30,000,000 centipoise at 25 °C, each A radical is independently selected from $R^X$ or halohydrocarbon radicals having from 1 to 6 carbon atoms, each T radical is independently selected from the group consisting of $R^X$, OH, H or $OR^Y$, and each $R^Y$ is independently an alkyl radical having from 1 to 4 carbon atoms.

As an example using forms of the preferred silicone resin and the preferred silicone polymer, one type of pressure sensitive adhesive is made by: mixing (i) from 30 to 80 inclusive parts by weight of at least one resin copolymer containing silicon-bonded hydroxyl radicals and consisting essentially of $R^X_3SiO_{1/2}$ units and $SiO_{4/2}$ units in a mole ratio of 0.6 to 0.9 $R^X_3SiO_{1/2}$ units for each $SiO_{4/2}$ unit present, (ii) between about 20 and about 70 parts by weight of at least one polydiorganosiloxane comprising $AR^XSiO$ units terminated with endblocking $TR^XASiO_{1/2}$ units, wherein the polydiorganosiloxane has a viscosity of from about 100 centipoise to about 30,000,000 mPa.s at 25 °C and each $R^X$ is a monovalent organic radical selected from the group consisting of hydrocarbon radicals of from 1 to 6 inclusive carbon atoms, each A radical is independently selected from $R^X$ or halohydrocarbon radicals having from 1 to 6 inclusive carbon atoms, each T radical is independently selected from the group consisting of $R^X$, OH, H or $OR^Y$, and each $R^Y$ is independently an alkyl radical of from 1 to 4 inclusive carbon atoms; a sufficient amount of (iii) at least one of the silicon-containing capping agents, also referred to throughout as endblocking agents, described below and capable of providing a silanol content, or concentration, in the range of 5,000 to 15,000, more typically 8,000 to 13,000, ppm, when desirable an additional catalytic amount of (iv) a mild silanol condensation catalyst in the event that none is provided by (ii), and when necessary, an effective amount of (v) an organic solvent which is inert with respect to (i), (ii), (iii) and (iv) to reduce the viscosity of a mixture of (i), (ii), (iii), and (iv), and condensing the mixture of (i), (ii), (iii) and (iv) at least until a substantial amount of the silicon-containing capping agent or agents have reacted with the silicon-bonded hydroxyl radicals and T radicals of (i) and (ii). Additional organosilicon endblocking agents can be used in conjunction with the silicon-containing capping agent or agents (iii) of the present invention.

The silicon-containing capping agent according to the previous paragraphs may be selected from the group of acrylate functional silanes, acrylate functional silazanes, acrylate functional disilazanes, acrylate functional disiloxanes, methacrylate functional silanes, methacrylate functional silazanes, methacrylate functional disilazanes, meth-acrylate functional disiloxanes, and combinations thereof and may be described as to be of the general formula $XYR'_bSiZ_{3-d}$, wherein X is a monovalent radical of the general formula AE- where E is -O- or -NH- and A is an acryl group or a methacryl group, Y is a divalent alkylene radical having from 1 to 6 carbon atoms, R' is a methyl or a phenyl radical, Z is a monovalent hydrolyzable organic radical or a halogen, and b is 0, 1 or 2. Preferably, the monovalent hydrolyzable organic radical is of the general formula R"0 - where R" is an alkylene radical. Most preferably, this particular endblocking agent is selected from the group of 3-methacryloxypropyldimethylchlorosilane, 3- methacryloxypropyldichlorosilane, 3-methacryloxypropyltrichlorosilane, 3-methacryloxypropyldimethylmethoxysilane, 3-methacryloxypropylmethyldimethoxysilane, 3-methacryloxypropyltrimethoxysilane, 3-methacryloxypropyldimethylethoxysilane, 3-methacryloxypropylmethyldiethoxysilane, 3-methacryloxypropyltriethoxysilane, (methacryloxymethyl)dimethylmethoxysilane, (methacryloxymethyl)methyldimethoxysilane, (methacryloxymethyl)trimethoxysilane, (methacryloxymethyl)dimethylethoxysilane, (methacryloxymethyl)methyldiethoxysilane, methacryloxymethyltriethoxysilane, methacryloxy-propyltriisopropoxysilane, 3-methacryloxypropyldimethylsilazane, 3-acryloxy-propyldimethylchlorosilane, 3-acryloxypropyldichlorosilane, 3-acryloxypropyltrichlorosilane, 3-acryloxypropyldimethylmethoxysilane, 3-acryloxy-propylmethyldimethoxysilane, 3-acryloxypropyltrimethoxysilane, 3-acryloxypropyl-dimethylsilazane, and combinations thereof.

The ethylenically unsaturated monomer according to the previous paragraphs can be any monomer having at least one carbon-carbon double bond. Preferably, the ethylenically unsaturated monomer according to the previous paragraphs may be a compound selected from the group consisting of aliphatic acrylates, aliphatic methacrylates, cycloaliphatic acrylates, cycloaliphatic methacrylates, and combinations thereof. It is to be understood that each of the compounds, the aliphatic acrylates, the aliphatic methacrylates, the cycloaliphatic acrylates, and the cycloaliphatic methacrylates, include an alkyl radical. The alkyl radicals of these compounds can include up to 20 carbon atoms. The aliphatic acrylates that may be selected as one of the ethylenically unsaturated monomers are selected from the group consisting of methyl acrylate, ethyl acrylate, propyl acrylate, n-butyl acrylate, iso-butyl acrylate, tert-butyl acrylate, hexyl acrylate, 2-ethylhexyl acrylate, iso-octyl acrylate, iso-nonyl acrylate, iso-pentyl acrylate, tridecyl acrylate, stearyl acrylate, lauryl acrylate, and mixtures thereof. The aliphatic methacrylates that may be selected as one of the ethylenically unsaturated monomers are selected from the group consisting of methyl methacrylate, ethyl methacrylate, propyl

methacrylate, n-butyl methacrylate, iso-butyl meth-acrylate, tert-butyl methacrylate, hexyl methacrylate, 2-eth-ylhexyl methacrylate, iso-octyl methacrylate, iso-nonyl methacrylate, iso-pentyl methacrylate, tridecyl methacrylate, stearyl methacrylate, lauryl methacrylate, and mixtures thereof. The cycloaliphatic acrylate that may be selected as one of the ethylenically unsaturated monomers is cyclohexyl acrylate, and the cycloaliphatic methacrylate that may be selected as one of the ethylenically unsaturated monomers is cyclohexyl methacrylate.

It is to be understood that the ethylenically unsaturated monomer used for preparing the silicone acrylic hybrid pressure sensitive adhesive may be more than one ethylenically unsaturated monomer. That is, a combination of ethylenically unsaturated monomers may be polymerized, more specifically co-polymerized, along with the silicon-containing pressure sensitive adhesive composition comprising acrylate or methacrylate functionality and the initiator. According to a certain embodiment of the invention, the silicone acrylic hybrid pressure-sensitive adhesive is prepared by using at least two different ethylenically unsaturated monomers, preferably selected from the group of 2-ethylhexyl acrylate and methyl acrylate, more preferably in a ratio of 50 % 2-ethylhexyl acrylate and 50 % methyl acrylate, or in a ratio of 60 % 2-ethylhexyl acrylate and 40 % methyl acrylate as the acrylic monomer.

The initiator according to the previous paragraphs may be any substance that is suitable to initiate the polymerization of the silicon-containing pressure sensitive adhesive composition comprising acrylate or methacrylate functionality and the ethylenically unsaturated monomer to form the silicone acrylic hybrid. For example, free radical initiators selected from the group of peroxides, azo compounds, redox initiators, and photo-initiators may be used.

Further suitable silicone resins, silicone polymers, silicon-containing capping agents, ethylenically unsaturated monomers, and initiators that can be used in accordance with the previous paragraphs are detailed in WO 2007/145996, EP 2 599 847 A1, and WO 2016/130408.

According to a certain embodiment of the invention, the silicone acrylic hybrid polymer comprises a reaction product of a silicone polymer, a silicone resin and an acrylic polymer, wherein the acrylic polymer is covalently self-crosslinked and covalently bound to the silicone polymer and/or the silicone resin.

According to a certain other embodiment of the invention, the silicone acrylic hybrid polymer comprises a reaction product of a silicone polymer, a silicone resin and an acrylic polymer, wherein the silicone resin contains triorganosiloxy units $R_3SiO_{1/2}$ where R is an organic group, and tetrafunctional siloxy units $SiO_{4/2}$ in a mole ratio of from 0.1 to 0.9 $R_3SiO_{1/2}$ units for each $SiO_{4/2}$.

The acrylic polymer may comprise at least an alkoxysilyl functional monomer, polysiloxane-containing monomer, halosilyl functional monomer or alkoxy halosilyl functional monomer. Preferably, the acrylic polymer is prepared from alkoxysilyl functional monomers selected from the group consisting of trialkoxylsilyl (meth)acrylates, dialkoxyalkylsilyl (meth)acrylates, and mixtures thereof, or comprises end-capped alkoxysilyl functional groups. The alkoxysilyl functional groups may preferably be selected from the group consisting of trimethoxylsilyl groups, dimethoxymethylsilyl groups, triethoxylsilyl, diethoxymethylsilyl groups and mixtures thereof.

The acrylic polymer may also be prepared from a mixture comprising polysiloxane-containing monomers, preferably from a mixture comprising polydimethylsiloxane mono (meth)acrylate.

The silyl functional monomers will typically be used in amounts of from 0.2 to 20 % by weight of the acrylic polymer, more preferably the amount of silyl functional monomers will range from about 1.5 to about 5 % by weight of the acrylic polymer.

The amount of polysiloxane-containing monomer will typically be used in amounts of from 1.5 to 50 % by weight of the acrylic polymer, more preferably the amount of polysiloxane-containing monomers will range from 5 to 15 % by weight of the acrylic polymer.

Alternatively, the acrylic polymer comprises a block or grafted copolymer of acrylic and polysiloxane. An example of a polysiloxane block copolymer is polydimethylsiloxane-acrylic block copolymer. The preferred amount of siloxane block is 10 to 50 % weight of the whole block polymer.

The acrylic polymer comprises alkyl (meth)acrylate monomers. Preferred alkyl (meth)acrylates which may be used have up to about 18 carbon atoms in the alkyl group, preferably from 1 to about 12 carbon atoms in the alkyl group. Preferred low glass transition temperature (Tg) alkyl acrylate with a homopolymer Tg of less than about 0 °C have from about 4 to about 10 carbon atoms in the alkyl group and include butyl acrylate, amyl acrylate, hexyl acrylate, 2-ethylhexyl acrylate, octyl acrylate, isooctyl acrylate, decyl acrylate, isomers thereof, and combinations thereof. Particularly preferred are butyl acrylate, 2-ethylhexyl acrylate and isooctyl acrylate. The acrylic polymer components may further comprise (meth)acrylate monomers having a high Tg such as methyl acrylate, ethyl acrylate, methyl methacrylate and isobutyl methacrylate.

The acrylic polymer component may further comprise a polyisobutylene group to improve cold flow properties of the resultant adhesive.

The acrylic polymer components may comprise nitrogen-containing polar monomers. Examples include N-vinyl pyrrolidone, N-vinyl caprolactam, N-tertiary octyl acrylamide, dimethyl acrylamide, diacetone acrylamide, N-tertiary butyl acrylamide, N-isopropyl acrylamide, cyanoethylacrylate, N-vinyl acetamide and N-vinyl formamide.

The acrylic polymer component may comprise one or more hydroxyl containing monomers such as 2-hydroxyethyl acrylate, 2-hydroxyethyl methacrylate, hydroxypropyl acrylate and/or hydroxypropyl methacrylate.

The acrylic polymer components may, if desired, comprise carboxylic acid containing monomers. Useful carboxylic acids preferably contain from about 3 to about 6 carbon atoms and include, among others, acrylic acid, methacrylic acid, itaconic acid, β-carboxyethyl acrylate and the like. Acrylic acid is particularly preferred.

Other useful, well known co-monomers include vinyl acetate, styrene, cyclohexyl acrylate, alkyl di(meth) acrylates, glycidyl methacrylate and allyl glycidyl ether, as well as macromers such as, for example, poly(styryl) methacrylate.

One acrylic polymer component that can be used in the practice of the invention is an acrylic polymer that comprises from about 90 to about 99.5 % by weight of butyl acrylate and from about 0.5 to about 10 % by weight dimethoxymethylsilyl methacrylate.

According to a certain embodiment of the invention, the silicone acrylic hybrid polymer may be prepared by a) reacting silicone polymer with silicone resin to form a resultant product, b) reacting the resultant product of a) with an acrylic polymer containing reactive functionality, wherein the components are reacted in an organic solvent.

According to a certain embodiment of the invention, the silicone acrylic hybrid polymer may be prepared by a) reacting a silicone resin with an acrylic polymer containing reactive functionality to form a resultant product, b) reacting the resultant product of a) with silicone polymer, wherein the components are reacted in an organic solvent.

According to a certain embodiment of the invention, the silicone acrylic hybrid polymer may be prepared by a) reacting a silicone polymer with an acrylic polymer containing reactive functionality to form a resultant product, b) reacting the resultant product of a) with silicone resin, wherein the components are reacted in an organic solvent.

Further suitable acrylic polymers, silicone resins, and silicone polymers that can be used for chemically reacting together a silicone polymer, a silicone resin and an acrylic polymer to provide a silicone acrylic hybrid polymer in accordance with the previous paragraphs are detailed in WO 2010/124187.

According to certain embodiments of the invention, the silicone acrylic hybrid polymer used in the TTS is blended with one or more non-hybrid polymers, preferably the silicone acrylic hybrid polymer is blended with one or more non-hybrid pressure sensitive adhesives (e.g. pressure-sensitive adhesives based on polysiloxane or acrylates).

SILICONE-BASED POLYMER (NON HYBRID)

As indicated above, the TTS according to the present invention comprises at least one silicone-containing polymer in the active agent-containing layer. In a preferred embodiment, the at least one silicone-containing polymer is a silicone acrylic hybrid polymer, a silicone-based polymer, or a combination thereof. The silicone-based polymer is described in further detail hereinafter.

As used herein, the silicone-based polymer is a non-hybrid polymer, i.e. a polymer, which does not include a hybrid species. Silicone-based polymers are based on polysiloxanes. They may therefore also be referred to as polymers based on polysiloxanes. Preferably, the silicone-based polymers are silicone-based pressure sensitive adhesives, i.e. pressure sensitive adhesives based on polysiloxanes.

As the silicone-based polymer is preferably a non-curing polymer, it is typically supplied and used in solvents, such as n-heptane and ethyl acetate. The solids content is usually between 30 % and 80 %.

Suitable silicone-based polymers are commercially available under the brand names BIO-PSAs (pressure sensitive adhesives based on polysiloxanes).

Pressure-sensitive adhesives based on polysiloxanes provide for suitable tack and for quick bonding to various skin types, including wet skin, suitable adhesive and cohesive qualities, long lasting adhesion to the skin, a high degree of flexibility, a permeability to moisture, and compatibility to many actives and film-substrates. It is possible to provide the pressure-sensitive adhesives based on polysiloxanes with sufficient amine resistance and therefore enhanced stability in the presence of amines. Such pressure-sensitive adhesives are based on a resin-in-polymer concept wherein, by condensation reaction of silanol end blocked polydimethylsiloxane with a silica resin (also referred to as silicate resin), a pressure-sensitive adhesive based on polysiloxane is prepared wherein for amine stability the residual silanol functionality is additionally capped with trimethylsiloxy groups. The silanol end blocked polydimethylsiloxane content contributes to the viscous component of the visco-elastic behavior, and impacts the wetting and the spreadability properties of the adhesive. The resin acts as a tackifying and reinforcing agent, and participates in the elastic component. The correct balance between silanol end blocked polydimethylsiloxane and resin provides for the correct adhesive properties.

In view of the above, silicone-based polymers, and in particular silicone-based pressure sensitive adhesives, are generally obtainable by polycondensation of silanol endblocked polydimethylsiloxane with a silicate resin. Amine-compatible silicone-based polymers, and in particular amine-compatible silicone-based pressure sensitive adhesives, can be obtained by reacting the silicone-based polymer, in particular the silicone-based pressure sensitive adhesive, with trimethylsilyl (e.g. hexamethyldisilazane) in order to reduce the silanol content of the polymer. As a result, the residual silanol functionality is at least partly, preferably mostly or fully capped with trimethylsiloxy groups.

As indicated above, the tackiness of the silicone-based polymer may be modified by the resin-to-polymer ratio, i.e. the ratio of the silanol endblocked polydimethylsiloxane to the silicate resin, which is preferably in the range of from 70:30 to 50:50, preferably from 65:35 to 55:45. The tackiness will be increased with increasing amounts of the polydimethylsiloxane relative to the resin. High tack silicone-based polymers preferably have a resin-to-polymer ratio of 55:45, medium tack silicone-based polymers preferably have a resin-to-polymer ratio of 60:40, and low tack silicone-based polymers preferably have a resin-to-polymer ratio of 65:35. High tack silicone-based polymers preferably have a complex viscosity at 0.01 rad/s and 30 °C of about 5 x 10e5 Pa.s medium tack silicone-based polymers preferably have a complex viscosity at 0.01 rad/s and 30 °C of about 5 x 10e6 Pa.s and low tack silicone-based polymers preferably have a complex viscosity at 0.01 rad/s and 30 °C of about 5 x 10e7 Pa.s. High tack amine-compatible silicone-based polymers preferably have a complex viscosity at 0.01 rad/s and 30 °C of about 5 x 10e5 Pa.s, medium tack amine-compatible silicone-based polymers preferably have a complex viscosity at 0.01 rad/s and 30 °C of about 5 x 10e7 Pa.s, and low tack amine-compatible silicone-based polymers preferably have a complex viscosity at 0.01 rad/s and 30 °C of about 5 x 10e8 Pa.s.

Examples of silicone-based PSA compositions which are commercially available include the standard BIO-PSA series (7-4400,7-4500 and 7-4600 series), the amine compatible (endcapped) BIO-PSA series (7-4100, 7-4200 and 7-4300 series) and the Soft Skin Adhesives series (7-9800) manufactured and typically supplied in n-heptane or ethyl acetate by Dow Corning. For example, BIO-PSA 7-4201 is characterized by a solution viscosity at 25 °C and about 60 % solids content in heptane of 450 mPa s and a complex viscosity at 0.01 rad/s at 30 °C of 1× 10e7 Pa.s. BIO-PSA 7-4301 has a solution viscosity at 25 °C and about 60 % solids content in heptane of 500 mPa s and a complex viscosity at 0.01 rad/s at 30 °C of 5× 10e5 Pa.s.

The pressure-sensitive adhesives based on polysiloxanes are supplied and used in solvents like n-heptane, ethyl acetate or other volatile silicone fluids. The solids content of pressure-sensitive adhesives based on polysiloxanes in solvents is usually between 60 and 85 %, preferably between 70 and 80 % or between 60 and 75 %. The skilled person is aware that the solids content may be modified by adding a suitable amount of solvent.

Pressure-sensitive adhesives based on polysiloxanes, which are, e.g., available from Dow Corning, may be obtained according to the following scheme:

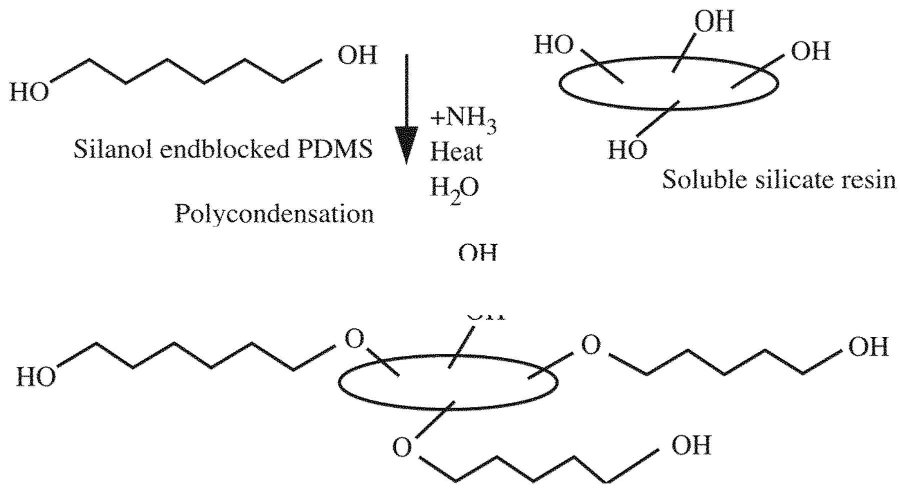


Such pressure-sensitive adhesives based on polysiloxanes are available from Dow Corning, e.g., under the tradenames BIO-PSA 7-4401, BIO-PSA-7-4501, or BIO-PSA 7-4601, which are provided in the solvent n-heptane (indicated by the code "01"), or under the tradenames BIO-PSA 7-4402, BIO-PSA 7-4502, and BIO 7-4602, which are provided in the solvent ethyl acetate (indicated by the code "02"). Typical solids contents in the solvent are in the range of from 60 to 75 %. The code "44" indicates a resin-to-polymer ratio of 65:35 resulting in a low tackiness, the code "45" indicates a resin-to-polymer ratio of 60:40 resulting in medium tackiness, the code "46" indicates a resin-to-polymer ratio of 55:45 resulting in high tackiness.

Amine-compatible pressure-sensitive adhesives based on polysiloxanes, which are, e.g., available from Dow Corning may be obtained according to the following scheme:

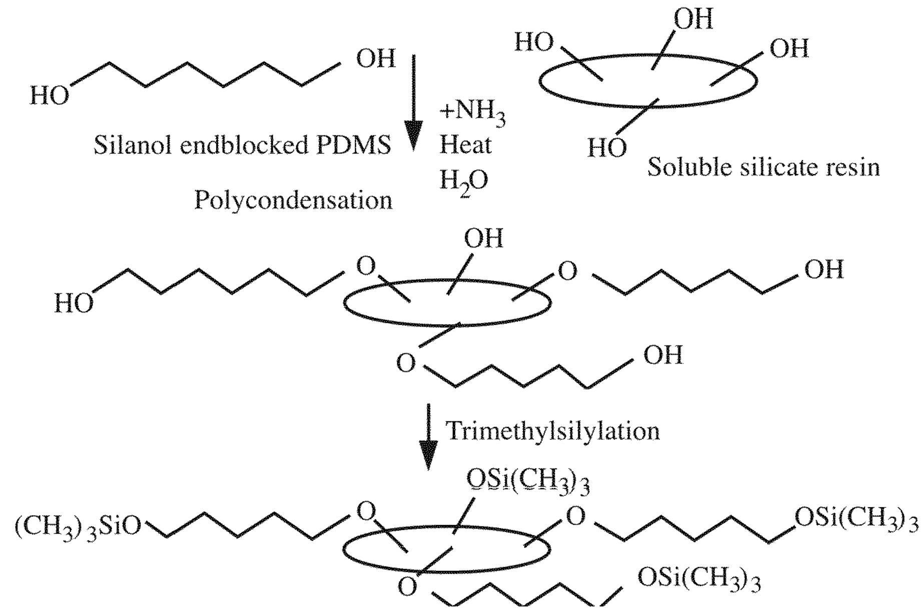


Such amine-compatible pressure-sensitive adhesives based on polysiloxanes are available from Dow Corning, e.g., under the tradenames BIO-PSA 7-4101, BIO-PSA-7-4201, or BIO-PSA 7-4301, which are provided in the solvent n-heptane (indicated by the code "01"), or under the tradenames BIO-PSA 7-4102, BIO-PSA 7-4202, and BIO 7-4302, which are provided in the solvent ethyl acetate (indicated by the code "02"). Typical solids contents in the solvent are in the range of from 60 to 75 %. The code "41" indicates a resin-to-polymer ratio of 65:35 resulting in a low tackiness, the code "42" indicates a resin-to-polymer ratio of 60:40 resulting in medium tackiness, the code "43" indicates a resin-to-polymer ratio of 55:45 resulting in high tackiness.

The preferred pressure-sensitive adhesives based on polysiloxanes in accordance with the invention are characterized by a solution viscosity at 25 °C and 60 % solids content in n-heptane of more than about 150 mPa s, or from about 200 mPa s to about 700 mPa s, preferably as measured using a Brookfield RVT viscometer equipped with a spindle number 5 at 50 rpm. Theses may also be characterized by a complex viscosity at 0.01 rad/s at 30 °C of less than about 1 x 10e8 Pa.s or from about 1 x 10e4 to about 9 x 10e7 Pa.s.

## FURTHER NON-HYBRID POLYMERS

As indicated above, the TTS according to the present invention may additionally comprise at least one further non-hybrid polymer in the active agent-containing layer. Such non-hybrid polymers preferably include non-hybrid polymers (e.g. non-hybrid pressure-sensitive adhesives) based on acrylates, polyisobutylenes, or styrene-isoprene-styrene block copolymers, preferably based on acrylates.

Non-hybrid pressure-sensitive adhesives are usually supplied and used in solvents like n-heptane and ethyl acetate. The solids content of the pressure-sensitive adhesives is usually between 30 % and 80 %.

Suitable non-hybrid polymers, which may additionally be present in the active agent-containing layer are commercially available e.g. under the brand names Oppanol™ (polyisobutylenes), JSR-SIS (a styrene-isoprene-styrene copolymer) or Duro-Tak™ (acrylic polymers).

Suitable polyisobutylenes according to the invention are available under the tradename Oppanol®. Combinations of high-molecular weight polyisobutylenes (B100/B80) and low-molecular weight polyisobutylenes (B10, B11, B12, B13) may be used. Suitable ratios of low-molecular weight polyisobutylene to high-molecular weight polyisobutylene are in the range of from 100:1 to 1:100, preferably from 95:5 to 40:60, more preferably from 90:10 to 80:20. A preferred example for a polyisobutylene combination is B10/B100 in a ratio of 85/15. Oppanol® B100 has a viscosity average molecular weight $M_v$ of 1,110,000, and a weight average molecular weight $M_w$ of 1,550,000, and an average molecular weight distribution $M_w/M_n$ of 2.9. Oppanol® B10 has a viscosity average molecular weight $M_y$ of 40,000, and a weight average molecular weight $M_w$ of 53,000, and an average molecular weight distribution $M_w/M_n$ of 3.2. In certain embodiments, polybutene may be added to the polyisobutylenes. The solids content of polyisobutylenes in solvents is usually between 30 and 50 %, preferably between 35 and 40 %. The skilled person is aware that the solids content may be modified by adding a suitable amount of solvent.

Pressure-sensitive adhesives based on acrylates may also be referred to as acrylate-based pressure-sensitive adhesives, or acrylate pressure-sensitive adhesives. Pressure-sensitive adhesives based on acrylates may have a solids

content preferably between 30 % and 60 %. Such acrylate-based pressure-sensitive adhesives may or may not comprise functional groups such as hydroxy groups, carboxylic acid groups, neutralized carboxylic acid groups and mixtures thereof. Thus, the term "functional groups" in particular refers to hydroxy- and carboxylic acid groups, and deprotonated carboxylic acid groups.

Corresponding commercial products are available e.g. from Henkel under the tradename Duro Tak®. Such acrylate-based pressure-sensitive adhesives are based on monomers selected from one or more of acrylic acid, butylacrylate, 2-ethylhexylacrylate, glycidylmethacrylate, 2-hydroxyethylacrylate, methylacrylate, methylmethacrylate, butylmethacrylate, t-octylacrylamide and vinylacetate, and are provided in ethyl acetate, heptane, n-heptane, hexane, methanol, ethanol, isopropanol, 2,4-pentanedione, toluene or xylene or mixtures thereof.

Specific acrylate-based pressure-sensitive adhesives are available as:

- Duro-Tak™ 387-2287 or Duro-Tak™ 87-2287 (a copolymer based on vinyl acetate, 2-ethylhexyl-acrylate, 2-hydroxyethyl-acrylate and glycidyl-methacrylate provided as a solution in ethyl acetate without cross-linking agent),
- Duro-Tak™ 387-2516 or Duro-Tak™ 87-2516 (a copolymer based on vinyl acetate, 2-ethylhexyl-acrylate, 2-hydroxyethyl-acrylate and glycidyl-methacrylate provided as a solution in ethyl acetate, ethanol, n-heptane and methanol with a titanium cross-linking agent),
- Duro-Tak™ 387-2051 or Duro-Tak™ 87-2051 (a copolymer based on acrylic acid, butylacrylate, 2-ethylhexylacrylate and vinyl acetate, provided as a solution in ethyl acetate and heptane),
- Duro-Tak™ 387-2353 or Duro-Tak™ 87-2353 (a copolymer based on acrylic acid, 2-ethylhexylacrylate, glycidylmethacrylate and methylacrylate, provided as a solution in ethyl acetate and hexane),
- Duro-Tak™ 87-4098 (a copolymer based on 2-ethylhexyl-acrylate and vinyl acetate, provided as a solution in ethyl acetate).

Additional polymers may also be added to enhance cohesion and/or adhesion.

Certain polymers in particular reduce the cold flow and are thus in particular suitable as additional polymer. A polymeric matrix may show a cold flow, since such polymer compositions often exhibit, despite a very high viscosity, the ability to flow very slowly. Thus, during storage, the matrix may flow to a certain extent over the edges of the backing layer. This is a problem with storage stability and can be prevented by the addition of certain polymers. A basic acrylate polymer (e.g. Eudragit® E100) may e.g. be used to reduce the cold flow. Thus, in certain embodiments, the matrix layer composition comprises additionally a basic polymer, in particular an amine-functional acrylate as e.g. Eudragit® E100. Eudragit® E100 is a cationic copolymer based on dimethylaminoethyl methacrylate, butyl methacrylate, and methyl methacrylate with a ratio of 2:1:1. The monomers are randomly distributed along the copolymer chain. Based on SEC method, the weight average molar mass (Mw) of Eudragit® E100 is approximately 47,000 g/mol. Further, polymers such as Plastoid B, acrylic polymers such as Eudragits, Chitosan, celluloses and derivatives thereof, and polystyrene may be useful to increase the dryness of the adhesive (e.g. the matrix layer).

## SILICONE GEL ADHESIVE

As indicated above, the TTS according to the present invention comprises a skin contact layer, which is an adhesive layer comprising a silicone gel adhesive. The silicone gel adhesive is an elastic, jelly-like solid material formed by lightly crosslinking silicone polymers. Thus, in contrast to the silicone-containing polymers as used herein, the silicone gel adhesive is based on a curable gel producing composition. The silicone gel adhesive provides for the adhesiveness of the TTS to the skin, while at the same time reducing the problem of skin irritation. Furthermore, the drug delivery of the TTS is not negatively affected.

Silicone gel adhesives are also referred to as silicone gels and, e.g., described in WO 2011/022199 A2

The silicone gel adhesive is generally formed from linear or branched silicones having reactive groups thereon. Such reactive groups undergo a crosslinking during curing. Examples of crosslinking reactions include the hydrosilylation reaction in which a silicone having an Si-H group reacts with a silicone having an aliphatic unsaturated reactive group in the presence of a hydrosilylation catalyst. These materials are described, for example in US 5,656,279, US 5,891,076, EP 0 322 118 and US 4,991,574. An alternative reaction is the condensation cure in which an alkoxy and/or hydroxy containing siloxanes are cured with a catalyst as described in US 4,831,070.

Typically, the silicone gel adhesive is obtainable by reacting a gel producing composition comprising (i) at least one alkenyl-substituted polydiorganosiloxane, (ii) at least one organosiloxane, which contains silicone-bonded hydrogen atoms, and (iii) at least one catalyst for the reaction of the SiH groups with the Si-alkenyl groups. These compositions cure at normal ambient temperatures, but curing can be expedited by heating to elevated temperatures, e.g., from 40 to 140 °C, or by applying UV light.

Suitable alkenyl groups contain from 2 carbon to about 6 carbon atoms and are exemplified by, but not limited to, vinyl, allyl, and hexenyl. The alkenyl groups in this component may be located at terminal, pendant (non-terminal), or both

terminal and pendant positions. The remaining silicon-bonded organic groups in the alkenyl-substituted polydiorganosiloxane are independently selected from the group consisting of monovalent hydrocarbon and monovalent halogenated hydrocarbon groups free of aliphatic unsaturation. These groups typically contain from 1 carbon to about 20 carbon atoms, alternatively from 1 carbon to 8 carbon atoms and are exemplified by, but not limited to, alkyl such as methyl, ethyl, propyl, and butyl; aryl such as phenyl; and halogenated alkyl such as 3,3,3-trifluoropropyl. Typically, at least 50 percent of the organic groups in the alkenyl-substituted polydiorganosiloxane are methyl. The structure of the alkenyl-substituted polydiorganosiloxane is typically linear, however, it may contain some branching due to the presence of trifunctional siloxane units. The viscosity of the alkenyl-substituted polydiorganosiloxane can be any desired. For example, it can be >0 $mm^2/s$ to 100,000 $mm^2/s$, alternatively 50 $mm^2/s$ to 80,000 $mm^2/s$, alternatively 300 $mm^2/s$ - 3,000 $mm^2/s$.

Methods for preparing the alkenyl-substituted polydiorganosiloxanes (i) of the present invention, such as condensation of the corresponding halosilanes or equilibration of cyclic polydiorganosiloxanes, are well known in the art.

The alkenyl-substituted polydiorganosiloxanes can be used in the gel producing composition in an amount of 10 wt. % - 90 wt. % based on the weight of the composition, alternatively 40 wt. % - 90 wt. %, alternatively 50 wt. % - 80 wt.%. The amount of alkenyl groups present in the alkenyl-substituted polydiorganosiloxane is typically in the range of 0.05 wt. % - 1 % wt%, alternatively 0.05 wt. % to 1 wt. % based on the weight of the alkenyl-substituted polydiorganosiloxane.

The organosiloxane containing silicon-bonded hydrogen atoms (ii) are also known in the art as described, for example in US patent number 3,983,298. The hydrogen atoms in this component may be located at terminal, pendant (non-terminal), or both terminal and pendant positions. The remaining silicon-bonded organic groups in this component are independently selected from the group consisting of monovalent hydrocarbon and monovalent halogenated hydrocarbon groups free of aliphatic unsaturation. These groups typically contain from 1 carbon to about 20 carbon atoms, alternatively from 1 carbon to 8 carbon atoms, and are exemplified by, but not limited to, alkyl such as methyl, ethyl, propyl, and butyl; aryl such as phenyl; and halogenated alkyl such as 3,3,3-trifluoropropyl. In one embodiment of the invention, at least 50 percent of the organic groups in the organosiloxane containing silicon-bonded hydrogen atoms are methyl.

The structure of the organosiloxane containing silicon-bonded hydrogen atoms is typically linear however; it may contain some branching due to the presence of trifunctional siloxane units. The viscosity of the organosiloxane containing silicon-bonded hydrogen atoms can be any desired. For example, it can be >0 $mm^2/s$ to 100,000 $mm^2/s$, alternatively, 5 $mm^2/s$ to 500 $mm^2/s$.

The organosiloxanes containing silicon-bonded hydrogen atoms can be used in the gel producing composition in an amount of 1 wt. % - 30 wt. % based on the weight of the composition, alternatively 5 wt. % - 20 wt %, and alternatively 5 wt. % - 15 wt. %. In one embodiment, the amount of hydrogen group present in the organosiloxane containing silicon-bonded hydrogen atoms is between 0.05 wt. % - 1.44 wt% based on the weight of the organosiloxane containing silicon-bonded hydrogen atoms.

Methods of preparing the organosiloxane containing silicon-bonded hydrogen atoms of the present invention by co-hydrolysis of the appropriate chlorosilanes are known in the art; U.S. Patent No. 2,877,255 to Clark; Japanese Laid Open Patent Application (KOKAI) SHO 62(1987)-39660 to Mogi et al.; and U.S. Patent Nos. 5,446,185 and U.S. No. 5,493,040 to Cobb et al.

In the gel producing compositions (i) and (ii) are present such that the ratio of (H as SiH):(Alkenyl as Si-Alkenyl) is generally in the range of 0.1: 1 to 10:1.

The hydrosilylation catalyst (iii) promotes the addition reaction of the alkenyl-substituted polydiorganosiloxane with the organosiloxane containing silicon-bonded hydrogen. The hydrosilylation catalyst can be any of the well known hydrosilylation catalysts comprising a platinum group metal, a compound containing a platinum group metal, or a microencapsulated platinum group metal or compound containing same. These platinum group metals include platinum, rhodium, ruthenium, palladium, osmium and iridium. Platinum and platinum compounds are preferred catalysts based on their high activity level in hydrosilylation reactions. One class of platinum catalysts is the complexes of chloroplatinic acid with certain vinyl-containing organosiloxane compounds disclosed by Willig in US. Pat. No. 3,419 593. A specific catalyst of this type is the reaction product of chloroplatinic acid and 1,3-diethenyl-1,1,3,3-tetramethyldisiloxane.

The hydrosilylation catalyst is present in an amount sufficient to cure the composition of the present invention. Typically, the concentration of the catalyst is sufficient to provide from 0.1 ppm to 500 ppm (part per million), alternatively from 1 ppm to 100 ppm, alternatively from 1 ppm to 50 ppm of a platinum group metal, based on the weight of (i) and (ii).

An optional ingredient is a hydroxy substituted silicone resin as described in US. Patent Application No. 2007-0202245. The resin is typically comprised of groups having the formula $R^3{}_3SiO_{1/2}$ ("M" groups) and groups having the formula $SiO_{4/2}$ ("Q" groups) where $R^3$ is a alkyl group having 1 carbon to 6 carbon atoms or alkylene group having 1 carbon to 6 carbon atoms, typically methyl or vinyl.

If an alkenyl group is present in the resin, typically the mol-% of R groups present as alkenyl groups is < 10 mol-%, alternatively 5 mol-%.

The number ratio of M groups to Q groups is typically in the range of 0.6:1 to 4:1, alternatively 0.6:1 to 1.0:1. The silicone resin typically contains 0.1 wt % to 5 wt %, alternatively 1.0 wt % to 5 wt % silicone-bonded hydroxy groups.

The resin can be used in the gel producing composition in an amount of 2 wt % to 45 wt %, based on the weight of

the gel producing composition and resin; alternatively 5 wt % to 40 wt %, alternatively 10 wt % to 35 wt %.

The silicone gel adhesive layer can be made by processes known in the art. For example, the gel may be preformed (e.g. as a sheet) by molding, calendaring, extruding, spraying, brushing, applying by hand, casting or coating on a substrate such as a liner. Or the silicone gel layer can be made by applying the gel producing composition to a substrate by spraying, coating, bar coating, etc. Once applied to the substrate the gel producing composition is cured to produce the silicone gel adhesive on the substrate.

In view of the above, in a preferred embodiment of the invention, the silicone gel adhesive is obtainable by reacting a gel producing composition comprising (i) a copolymer of vinylmethylsiloxane and dimethylsiloxane with (ii) methylhydrogen polysiloxane with trimethylsilyl endgroups in the presence of (iii) a platinum catalyst. Preferably, (i) and (ii) are present such that the ratio of (H as SiH):(Alkenyl as Si-Alkenyl) is generally in the range of 0.1:1 to 10:1.

Further, in certain preferred embodiments, the silicone gel adhesive is a silicate resin-reinforced silicone gel adhesive that contains from about 2 to about 45 % by weight of at least one hydroxyl substituted silicate resin.

## FURTHER ADDITIVES

The TTS according to the invention, and in particular the active agent-containing layer may further comprise at least one additive or excipient. Said additives or excipients are preferably selected from the group consisting of crystallization inhibitors, solubilizers, fillers, substances for skincare, pH regulators, preservatives, tackifiers, softeners, stabilizers, and permeation enhancers, in particular from crystallization inhibitors, substances for skincare, tackifiers, softeners, stabilizers, and permeation enhancers. More preferably, said additives are selected from the group consisting of crystallization inhibitors, solubilizers, fillers, substances for skincare, pH regulators, preservatives, tackifiers, softeners, stabilizers, and permeation enhancers, in particular from substances for skincare, tackifiers, softeners, and stabilizers. Such additives may be present in the active agent-containing layer in an amount of from 0.001 % to 15 % by weight, e.g. from 1 to 10 % by weight or from 0.01 to 5 % by weight, based on the total weight of the active agent-containing layer.

It should be noted that in pharmaceutical formulations, the formulation components are categorized according to their physicochemical and physiological properties, and in accordance with their function. This means in particular that a substance or a compound falling into one category is not excluded from falling into another category of formulation component. E.g. a certain polymer can be a crystallization inhibitor but also a tackifier. Some substances may e.g. be a typical softener but at the same time act as a permeation enhancer. The skilled person is able to determine based on his general knowledge in which category or categories of formulation component a certain substance or compound belongs to. In the following, details on the excipients and additives are provided which are, however, not to be understood as being exclusive. Other substances not explicitly listed in the present description may be as well used in accordance with the present invention, and substances and/or compounds explicitly listed for one category of formulation component are not excluded from being used as another formulation component in the sense of the present invention.

In one embodiment, the active agent-containing layer further comprises a crystallization inhibitor. In some embodiments, the crystallization inhibitor can be present in an amount of from 0.5 to 10 % by weight based on the total weight of the active agent-containing layer. Suitable examples of crystallization inhibitors include polyvinylpyrrolidone, vinyl acetate/vinylpyrrolidone copolymer and cellulose derivatives. The crystallization inhibitor is preferably polyvinylpyrrolidone, more preferably soluble polyvinylpyrrolidone. The crystallization inhibitor may increase the solubility of the active agent or inhibit the crystallization of the active agent, e.g., if the active agent is used in the form of a salt.

In one embodiment, the active agent-containing layer further comprises a stabilizer, wherein the stabilizer is preferably selected from tocopherol and ester derivatives thereof and ascorbic acid and ester derivatives thereof. In some embodiments, the stabilizer can be present in an amount of from 0.001 to 2.0 %, preferably from 0.01 to 1.0 %, by weight based on the total weight of the active agent-containing layer. In some embodiments, preferred stabilizers include sodium metabisulfite, ascorbyl esters of fatty acids such as ascorbyl palmitate, ascorbic acid, butylated hydroxytoluene, tocopherol, tocopheryl acetate and tocopheryl linoleate. Preferred stabilizers include ascorbyl esters of fatty acids, ascorbic acid, tocopherol, tocopheryl acetate and tocopheryl linoleate. Particularly preferred is tocopherol. Also particularly preferred is a combination of tocopherol and ascorbyl palmitate.

In one embodiment, the active agent-containing layer further comprises a softener/ plasticizer. Exemplary softeners/plasticizers include linear or branched, saturated or unsaturated alcohols having 6 to 20 carbon atoms, triglycerides and polyethylene glycols.

In one embodiment, the active agent-containing layer further comprises a solubilizer. The solubilizer preferably improves the solubility of the active agent in the active agent-containing layer. Preferred solubilizers include, e.g., glycerol-, polyglycerol-, propylene glycol- and polyoxyethylene-esters of medium chain and/or long chain fatty acids, such as glyceryl monolinoleate, medium chain glycerides and medium chain triglycerides, non-ionic solubilisers made by reacting castor oil with ethylene oxide, and any mixtures thereof which may further contain fatty acids or fatty alcohols; cellulose and methylcellulose and derivatives thereof such as hydroxypropylcellulose and hypromellose acetate succinate; various cyclodextrins and derivatives thereof; non-ionic tri-block copolymers having a central hydrophobic chain of

polyoxypropylene flanked by two hydrophilic chains of polyoxyethylene known as poloxamers; water-soluble derivatives of vitamin E; pharmaceutical graded or agglomerated spherical isomalt; a polyethylene glycol, polyvinyl acetate and polyvinylcaprolactame-based graft copolymer, also abbreviated as PVAc-PVCap- PEG and known as Soluplus®; purified grades of naturally derived castor oil, of polyethylene glycol 400, of polyoxyethylene sorbitan monooleate (such as polysorbate 80) or of propylene glycols; diethylene glycol monoethyl ether; glucono-delta-lactone; maize and potato starch; as well as any of the below mentioned soluble polyvinylpyrrolidones, but also insoluble / cross-linked polyvinylpyrrolidones such as crospovidones.

However, also the permeation enhancers mentioned below can act as solubilizers. Furthermore, also crystallization inhibitors may act as solubilizers.

In one embodiment, the active agent-containing layer further comprises a pH regulator. Suitable pH regulators include mild acids and bases including amine derivatives, inorganic alkali derivatives, and polymers with basic or acidic functionality.

In one embodiment, the active agent-containing layer further comprises a preservative. Suitable preservatives include parabens, formaldehyde releasers, isothiazolinones, phenoxyethanol, and organic acids such as benzoic acid, sorbic acid, levulinic acid and anisic acid.

In one embodiment, the active agent-containing layer further comprises a substance for skincare. Such substances may be used to avoid or reduce skin irritation as detectable by the dermal response score. Suitable substances for skincare include sterol compounds such as cholesterol, dexpanthenol, alpha-bisabolol, and antihistamines. Substances for skincare are preferably used in amounts of from 1 to 10 % by weight based on the total weight of the active agent-containing layer.

If the active agent-containing layer is required to have self-adhesive properties and one or more polymers is/are selected, which does/do not provide sufficient self-adhesive properties, a tackifier is added. Preferred tackifiers include Miglyol, which is a liquid wax ester based on long-chain, unsaturated, even-numbered fatty acids and long-chain, unsaturated, even-numbered fatty alcohols of vegetable origin, and polyethyleneglycols. In particular, the tackifier may be selected from polyvinylpyrrolidone (which, due to its ability to absorb water, is able to maintain the adhesive properties of the matrix layer and thus can be regarded as a tackifier in a broad sense), triglycerides, polyethylene glycols, dipropylene glycol, resins, resin esters, terpenes and derivatives thereof, ethylene vinyl acetate adhesives, dimethylpolysiloxanes and polybutenes, preferably polyvinylpyrrolidone and more preferably soluble polyvinylpyrrolidone. Preferably, the tackifier may be selected from polyvinylpyrrolidone, triglycerides, dipropylene glycol, resins, resin esters, terpenes and derivatives thereof, ethylene vinyl acetate adhesives, dimethylpolysiloxanes and polybutenes, preferably polyvinylpyrrolidone and more preferably soluble polyvinylpyrrolidone. In some embodiments, the tackifier can be present in an amount of from 5 to 15 % by weight based on the total weight of the active agent-containing layer.

The term "soluble polyvinylpyrrolidone" refers to polyvinylpyrrolidone, also known as povidone, which is soluble with more than 10 % in at least ethanol, preferably also in water, diethylene glycol, methanol, n-propanol, 2 propanol, n-butanol, chloroform, methylene chloride, 2-pyrrolidone, macrogol 400, 1,2 propylene glycol, 1,4 butanediol, glycerol, triethanolamine, propionic acid and acetic acid. Examples of polyvinylpyrrolidones which are commercially available include Kollidon® 12 PF, Kollidon® 17 PF, Kollidon® 25, Kollidon® 30 and Kollidon® 90 F supplied by BASF, or povidone K90F. The different grades of Kollidon® are defined in terms of the K-Value reflecting the average molecular weight of the polyvinylpyrrolidone grades. Kollidon® 12 PF is characterized by a K-Value range of 10.2 to 13.8, corresponding to a nominal K-Value of 12. Kollidon® 17 PF is characterized by a K-Value range of 15.3 to 18.4, corresponding to a nominal K-Value of 17. Kollidon® 25 is characterized by a K-Value range of 22.5 to 27.0, corresponding to a nominal K-Value of 25, Kollidon® 30 is characterized by a K-Value range of 27.0 to 32.4, corresponding to a nominal K-Value of 30. Kollidon® 90 F is characterized by a K-Value range of 81.0 to 97.2, corresponding to a nominal K-Value of 90. Preferred Kollidon® grades are Kollidon® 12 PF, Kollidon® 30 and Kollidon® 90 F.

Within the meaning of this invention, the term "K-Value" refers to a value calculated from the relative viscosity of polyvinylpyrrolidone in water according to the European Pharmacopoeia (Ph.Eur.) and USP monographs for "Povidone".

Fillers such as silica gels, titanium dioxide and zinc oxide may be used in conjunction with the polymer in order to influence certain physical parameters, such as cohesion and bond strength, in the desired way.

In one embodiment, the active agent-containing layer further comprises a permeation enhancer. Permeation enhancers are substances, which influence the barrier properties of the stratum corneum in the sense of increasing the active agent permeability. Some examples of permeation enhancers are polyhydric alcohols such as dipropylene glycol, propylene glycol, and polyethylene glycol; oils such as olive oil, squalene, and lanolin; fatty ethers such as cetyl ether and oleyl ether; fatty acid esters such as isopropyl myristate; urea and urea derivatives such as allantoin; polar solvents such as dimethyldecylphosphoxide, methylcetylsulfoxide, dimethylaurylamine, dodecyl pyrrolidone, isosorbitol, dimethylacetonide, dimethylsulfoxide, decylmethylsulfoxide, and dimethylformamide; salicylic acid; amino acids; benzyl nicotinate; and higher molecular weight aliphatic surfactants such as lauryl sulfate salts. Other agents include oleic and linoleic acids, ascorbic acid, panthenol, butylated hydroxytoluene, tocopherol, tocopheryl acetate, tocopheryl linoleate, propyl oleate, and isopropyl palmitate.

If the active agent-containing layer further comprises a permeation enhancer, the permeation enhancer is preferably selected from diethylene glycol monoethyl ether (transcutol), diisopropyl adipate, isopropyl myristate, isopropyl palmitate, lauryl lactate, and dimethylpropylene urea.

It has been found that the TTS provides sufficient permeability of the active agent even if no permeation enhancer is present. Therefore, in certain embodiments of the invention, the active agent-containing layer does not comprise a permeation enhancer or solubilizer.

## RELEASE CHARACTERISTICS

It has been found that the TTS according to the present invention is suitable for the transdermal administration of an active agent, in particular rivastigmine or buprenorphine, to the systemic circulation, while at the same time providing good adhesive properties and reduced skin irritation in comparison to other TTS known in the art.

The TTS according to the present invention is suitable for the transdermal administration of an active agent, in particular rivastigmine or buprenorphine, to the systemic circulation for a predefined extended period of time, preferably for at least 24 hours or at least 72 hours.

Preferably, the TTS provides therapeutically effective plasma concentrations of the active agent, preferably rivastigmine or buprenorphine, within less than 8 hours, preferably less than 6 hours, more preferably less than 4 hours after application of the TTS to the skin.

Preferably, the TTS provides, after a steady state of the plasma concentration is reached, a therapeutically effective steady state plasma concentration of the active agent, preferably rivastigmine or buprenorphine, for at least 12 hours, preferably at least 18 hours, more preferably at least 20 hours, provided that the TTS is administered to the skin for a sufficient time, e.g., for at least 24 hours, so that the steady state can be reached and maintained.

In one embodiment, the TTS provides by transdermal delivery a mean release rate of from 150 to 3500 $\mu g/cm^2$*day, preferably from 200 to 3000 $\mu g/cm^2$*day rivastigmine over about 24 hours of administration.

In another embodiment, the TTS provides a cumulative permeated amount of rivastigmine as measured in a Franz diffusion cell with an EVA membrane of about 600 to 1200 $\mu g/cm^2$over a time period of about 24 hours.

In one embodiment, the TTS provides by transdermal delivery a mean release rate of from 2 to 25 $\mu$g/h, preferably from 5 to 20 $\mu$g/h buprenorphine over at least 48 hours, preferably at least 72 hours of administration.

In another embodiment, the TTS provides a cumulative permeated amount of buprenorphine as measured in a Franz diffusion cell with human skin of about 60 to 130 $\mu g/cm^2$over a time period of about 72 hours.

## METHOD OF TREATMENT / MEDICAL USE

The TTS according to the present invention is suitable for use in a method of treatment. If the active agent is rivastigmine, the TTS is suitable for use in a method of preventing, treating, or delaying of progression of Alzheimer's disease, dementia associated with Parkinson's disease, and/or symptoms of traumatic brain injury. Furthermore, the TTS is suitable for use in a method of treating mild to moderate dementia caused by Alzheimer's or Parkinson's disease. If the active agent is buprenorphine, the TTS is suitable for use in the treatment of pain.

In one embodiment, the TTS according to the invention is for use in a method of treatment, wherein the transdermal therapeutic system is preferably applied to the skin of the patient for at least 24 hours or at least 72 hours. In another embodiment, the present invention relates to a method of treatment comprising applying a transdermal therapeutic system according to the invention to the skin of a patient, preferably for at least 24 hours or at least 72 hours.

In one embodiment, the TTS comprises rivastigmine as active agent and is for use in a method of preventing, treating, or delaying of progression of Alzheimer's disease, dementia associated with Parkinson's disease, and/or symptoms of traumatic brain injury. In a preferred embodiment, the TTS is applied to the skin of the patient for a dosing interval of at least 24 hours, preferably about 24 hours. In another embodiment, the present invention relates to a method of preventing, treating, or delaying of progression of Alzheimer's disease, dementia associated with Parkinson's disease, and/or symptoms of traumatic brain injury comprising applying a transdermal therapeutic system according to the invention comprising rivastigmine to the skin of a patient, preferably for at least 24 hours, in particular about 24 hours.

In one embodiment, the TTS comprises buprenorphine as active agent and is for use in a method of treating pain. In a preferred embodiment, the TTS is applied to the skin of the patient for a dosing interval of at least 72 hours, preferably at least 84 hours. In another embodiment, the present invention relates to a method of treating pain comprising applying a transdermal therapeutic system according to the invention comprising buprenorphine to the skin of a patient, preferably for at least 72 hours, in particular at least 84 hours.

In connection with the above uses and methods of treatment, the TTS according to the invention is preferably applied to at least one body surface on the subject selected from the upper outer art, upper chest, upper back or the side of the chest for the defined dosing intervals.

The preferred application time of a TTS according to the invention is at least 24 hours, preferably about 24 hours

(1 day) or about 84 hours (3.5 days). After this time, the TTS may be removed, and optionally a new TTS may be applied, so as to allow an around-the-clock treatment.

**PROCESS OF MANUFACTURE**

The invention further relates to a process of manufacture of an active agent-containing layer structure for use in a transdermal therapeutic system.

In accordance with the invention, the process for manufacturing the active agent-containing layer for use in a transdermal therapeutic system according to the present invention comprises the steps of

1.1) coating a coating composition comprising

- an active agent; and
- at least one silicone-containing polymer

on a first foil;
1.2) drying the coated coating composition to form the active agent-containing layer;
1.3) laminating the active agent-containing layer with a backing layer.

As explained above, the silicone-containing polymer is preferably non-curing and therefore typically applied by a solvent-based process. Accordingly, the at least one silicone-containing polymer is preferably provided in a solvent, wherein the solids content in the solvent is preferably from 40 to 75 % by weight. The solvent is preferably selected from alcoholic solvents, in particular methanol, ethanol, isopropanol and mixtures thereof, and from non-alcoholic solvents, in particular ethyl acetate, hexane, heptane, petroleum ether, toluene, and mixtures thereof, and is more preferably selected from non-alcoholic solvents, and is most preferably ethyl acetate or n-heptane. The active agent is preferably homogeneously dissolved or dispersed in the coating composition.

The coated coating composition is solidified by drying. Drying is preferably performed at a temperature of from 20 to 90 °C, more preferably from 40 to 70 °C.

The process for manufacturing the skin contact layer for use in a transdermal therapeutic system according to the present invention comprises the steps of

2.1) coating a gel producing composition comprising

(i) at least one alkenyl-substituted polydiorganosiloxane,
(ii) at least one organosiloxane, which contains silicone-bonded hydrogen atoms, and
(iii) at least one catalyst for the reaction of the SiH groups with the Si-alkenyl groups, on a second foil;

2.2) crosslinking the gel producing composition at a temperature of from 50 °C to 150 °C or by applying UV light to form the skin contact layer;
2.3) laminating the skin contact layer with a release liner.

As explained above, the gel producing composition forms the silicone gel adhesive of the skin contact layer upon curing, i.e. crosslinking of the reactive groups of the silicone polymers.

Crosslinking is preferably performed at a temperature of from 40 °C to 140 °C.

The active agent-containing layer and the skin contact layer are preferably prepared separately as indicated above, and then laminated together by removing the foils and then laminating the open sides of the two layers together, so as to give an active agent-containing layer structure.

Accordingly, the process for manufacturing an active agent-containing layer structure for use in a transdermal therapeutic system according to the present invention comprises the steps of:

1.1) coating a coating composition comprising

- an active agent; and
- at least one silicone-containing polymer

on a first foil;
1.2) drying the coated coating composition to form the active agent-containing layer;
1.3) laminating the active agent-containing layer with a backing layer;

2.1) coating a gel producing composition comprising

(i) at least one alkenyl-substituted polydiorganosiloxane,
(ii) at least one organosiloxane, which contains silicone-bonded hydrogen atoms, and
(iii) at least one catalyst for the reaction of the SiH groups with the Si-alkenyl groups, on a second foil;

2.2) crosslinking the gel producing composition at a temperature of from 50 °C to 150 °C or by applying UV light to form the skin contact layer;
2.3) laminating the skin contact layer with a release liner;
3.1) removing the foils from the active agent-containing layer and the skin contact layer; and
3.2) laminating the open side of the active agent-containing layer onto the open side of the skin contact layer to obtain an active agent-containing layer structure.

The preparation of the active-agent containing layer may be performed before or after the preparation of the skin contact layer, or the preparation of the two layers may be performed in parallel.

The present invention also relates to a transdermal therapeutic system obtainable by the above described process.

## EXAMPLES

The present invention will now be more fully described with reference to the accompanying examples. It should be understood, however, that the following description is illustrative only and should not be taken in any way as a restriction of the invention. Numerical values provided in the examples regarding the amount of ingredients in the composition or the area weight may vary slightly due to manufacturing variability.

## EXAMPLES 1A AND 1B

### Active-containing coating composition

The formulations of the active-containing coating compositions of Examples 1A and 1B are summarized in Table 1.1 below. The solids %-values refer to the amounts (Amt) in % by weight.

Table 1.1

| **Ingredient (Trade Name)** | **Ex. 1A** | | **Ex. 1B** | |
|---|---|---|---|---|
| | **Amt [g]** | **Solids [%]** | **Amt [g]** | **Solids [%]** |
| Rivastigmine base | 10 | 20.0 | 10 | 20.0 |
| Silicone acrylic hybrid pressure sensitive adhesive in ethyl acetate Solids content of 50 % by weight (PSA SilAc 7-6101 from Dow Corning Healthcare) | 40 | 80.0 | 40 | 80.0 |
| Total | 50 | 100.0 | 50 | 100.0 |
| Area Weight [$g/m^2$] | 90 | | 90 | |
| Loading API [$\mu g/cm^2$] | 1800 | | 1800 | |

### Preparation of the active-containing coating composition

A beaker was loaded with the silicone acrylic hybrid pressure-sensitive adhesive having a solids content of about 50 % by weight (PSA SilAc 7-6101 from Dow Corning Healthcare). The rivastigmine base was added under stirring. The mixture was stirred at about 800 rpm until a homogenous mixture was obtained (at least 20 min).

### Coating of the active-containing coating composition

The resulting active-containing coating composition was coated within less than 24 h on an abhesively equipped foil (Scotchpak 9755 AB1F) using hand over knife lab coating equipment, using an erichson coater. The solvent was

removed by drying in a first step at about room temperature (23 ± 2 °C) for about 10 min, followed by a second drying step at about 60 °C for about 20 min.

The coating thickness was chosen such that removal of the solution results in an area weight of the active-containing layer of about 90.0 g/m$^2$.

The resulting active-containing layer was then laminated with a backing layer (FO PET 23 μm transparent).

**Active-free coating composition**

The formulation of the active-free coating composition of Examples 1A and 1B are summarized in Table 1.2 below. The solids %-values refer to the amounts (Amt) in % by weight.

Table 1.2

| Ingredient (Trade Name) | Ex. 1A | | Ex. 1B | |
|---|---|---|---|---|
| | Amt [g] | Solids [%] | Amt [g] | Solids [%] |
| Component A of 2 component Soft Skin Adhesive from Dow Corning (MG 7-9900 (A)) | 15 | 50.0 | 15 | 50.0 |
| Component B of 2 component Soft Skin Adhesive from Dow Corning (MG 7-9900 (B)) | 15 | 50.0 | 15 | 50.0 |
| Total | 30 | 100.0 | 30 | 100.0 |
| Area Weight [g/m$^2$] | 40 | | 85 | |
| Layer thickness [μm] | 50 | | 100 | |

**Preparation of the active-free coating composition**

Both components were weighed separately into a suitable vessel, e.g. a glass vessel. Subsequently, component A was added to the mixing vessel followed by component B. Then the mixture was mixed at approx. 200 rpm for approx. 5 min until a homogeneous mixture of Component A and Component B was obtained.

**Coating of the active-free coating composition**

Within a time frame of approx. 30 min, the resulting active-free coating composition was coated on an abhesively equipped foil using hand over knife lab coating equipment, e.g. an erichson coater. The coating temperature was set to 120 °C. The resulting active-free layer was heated at this temperature for approx. 5 min.

The coating thickness was chosen such that removal of the solvents resulted in a layer thickness of the active-free (skin contact) layer of approx. 50 μm (for Ex. 1A) and 100 μm (for Ex. 1B).

The resulting active-free (skin contact) layer was laminated with a release liner (FEP, fluorinated ethylene propylene, 100 μm).

**Lamination of the active-containing layer and the active-free (skin contact) layer**

The active-free (skin contact) layer was then laminated with the active-containing layer. For this purpose, the abhesively equipped foils used for the coating and drying of the layers were removed and the resulting open sides of the active-containing layer and the active-free (skin contact) layer were laminated together resulting in an active-containing self-adhesive layer structure comprising the backing layer, the active-containing layer, and the active-free (skin contact) layer, wherein the active-containing layer is attached to the backing layer, and the active-free (skin contact) layer is attached to the active-containing layer, and wherein the structure is closed by a release liner, which is attached to the active-free (skin contact layer).

**Preparation of the TTS**

The individual systems (TTS) were punched out from the active-containing self-adhesive layer structure obtained as described above. Then, the TTS were sealed into pouches of the primary packaging material.

### Measurement of the skin permeation

The permeated amounts of the TTS prepared according to Examples 1A and 1B were determined by experiments in accordance with the EMA Guideline on quality of transdermal patches (adopted October 23, 2014) carried out with a 10.0 ml Franz diffusion cell, wherein an EVA-membrane (9 % vinyl acetate; Nitroderm TTS K-Membrane 343 mm from PetroplastVinora AG) having a thickness of 50 μm was used. Diecuts with an area of release of 1.118 $cm^2$ were punched from the TTS. The permeated amount of rivastigmine in the receptor medium of the Franz diffusion cell (phosphate buffer solution pH 5.5 with 0.1 % sodium azide as antibacteriological agent) at a temperature of 32 ± 1 °C was measured.

The results are shown in Table 1.3 and Figure 1.

Table 1.3

| Cumulative permeated amount with SD [μg/cm²] | | | | |
|---|---|---|---|---|
| **Elapsed time [h]** | **Ex. 1A (n = 3)** | | **Ex. 1B (n = 3)** | |
| | **Amount** | **SD** | **Amount** | **SD** |
| **2** | 106 | 2.0 | 107 | 4.16 |
| **4** | 213 | 7.55 | 193 | 31.9 |
| **6** | 318 | 13.4 | 307 | 24.6 |
| **8** | 412 | 18.3 | 406 | 22.7 |
| **24** | 965 | 42.4 | 968 | 15.6 |

### EXAMPLES 2A AND 2B

### Active-containing coating composition

The formulations of the active-containing coating compositions of Examples 2A and 2B are summarized in Table 2.1 below. The solids %-values refer to the amounts (Amt) in % by weight.

Table 2.1

| **Ingredient (Trade Name)** | **Ex. 2A** | | **Ex. 2B** | |
|---|---|---|---|---|
| | **Amt [g]** | **Solids [%]** | **Amt [g]** | **Solids [%]** |
| Rivastigmine base | 10 | 20.0 | 10 | 20.0 |
| Silicone acrylic hybrid pressure sensitive adhesive in ethyl acetate Solids content of 50 % by weight (PSA SilAc 7-6101 from Dow Corning Healthcare) | 40 | 80.0 | 40 | 80.0 |
| Total | 50 | 100.0 | 50 | 100.0 |
| Area Weight [g/m²] | 90 | | 90 | |
| Loading API [μg/cm²] | 1800 | | 1800 | |

### Preparation of the active-containing coating composition

A beaker was loaded with the silicone acrylic hybrid pressure-sensitive adhesive having a solids content of about 50 % by weight (PSA SilAc 7-6101 from Dow Corning Healthcare). The rivastigmine base was added under stirring. The mixture was stirred at about 800 rpm until a homogenous mixture was obtained (at least 20 min).

### Coating of the active-containing coating composition

The resulting active-containing coating composition was coated within less than 24 h on an abhesively equipped foil (Scotchpak 9755 AB1F) using hand over knife lab coating equipment, using an erichson coater. The solvent was

removed by drying in a first step at about room temperature (23 ± 2 °C) for about 10 min, followed by a second drying step at about 60 °C for about 20 min.

The coating thickness was chosen such that removal of the solution results in an area weight of the active-containing layer of about 90.0 g/$m^2$.

The resulting active-containing layer was then laminated with a backing layer (FO PET 23 μm transparent).

**Active-free coating composition**

The formulation of the active-free coating composition of Examples 2A and 2B are summarized in Table 2.2 below. The solids %-values refer to the amounts (Amt) in % by weight.

Table 2.2

| Ingredient (Trade Name) | Ex. 2A | | Ex. 2B | |
|---|---|---|---|---|
| | Amt [g] | Solids [%] | Amt [g] | Solids [%] |
| Component A of 2 component Soft Skin Adhesive from Dow Corning (MG 7-1010 (A)) | 20 | 50.0 | 20 | 50.0 |
| Component B of 2 component Soft Skin Adhesive from Dow Corning (MG 7-1010 (B)) | 20 | 50.0 | 20 | 50.0 |
| Total | 40 | 100.0 | 40 | 100.0 |
| Area Weight [g/$m^2$] | 49 | | 68 | |
| Layer thickness [μm] | 50 | | 100 | |

**Preparation of the active-free coating composition**

Both components were weighed separately into a suitable vessel, e.g. a glass vessel. Subsequently, component A was added to the mixing vessel followed by component B. Then the mixture was mixed at approx. 200 rpm for approx. 5 min until a homogeneous mixture of Component A and Component B was obtained.

**Coating of the active-free coating composition**

Within a time frame of approx. 30 min, the resulting active-free coating composition was coated on an abhesively equipped foil using hand over knife lab coating equipment, e.g. an erichson coater. The coating temperature was set to 120 °C. The resulting active-free layer was heated at this temperature for approx. 5 min.

The coating thickness was chosen such that removal of the solvents resulted in a layer thickness of the active-free (skin contact) layer of approx. 50 μm (for Ex. 2A) and 100 μm (for Ex. 2B).

The resulting active-free (skin contact) layer was laminated with a Scotchpak 9709 release liner (fluorosilicone coated polyester film).

**Lamination of the active-containing layer and the active-free (skin contact) layer**

The active-free (skin contact) layer was then laminated with the active-containing layer. For this purpose, the abhesively equipped foils used for the coating and drying of the layers were removed and the resulting open sides of the active-containing layer and the active-free (skin contact) layer were laminated together resulting in an active-containing self-adhesive layer structure comprising the backing layer, the active-containing layer, and the active-free (skin contact) layer, wherein the active-containing layer is attached to the backing layer, and the active-free (skin contact) layer is attached to the active-containing layer, and wherein the structure is closed by a release liner, which is attached to the active-free (skin contact layer).

**Preparation of the TTS**

The individual systems (TTS) were punched out from the active-containing self-adhesive layer structure obtained as described above. Then, the TTS were sealed into pouches of the primary packaging material.

**Measurement of the skin permeation**

The permeated amounts of the TTS prepared according to Examples 2A and 2B were determined as described for Examples 1A and 1B.

The results are shown in Table 2.3 and in Figure 1.

Table 2.3

| Cumulative permeated amount with SD [μg/cm²] | | | | |
|---|---|---|---|---|
| Elapsed time [h] | Ex. 2A (n = 3) | | Ex. 2B (n = 3) | |
| | Amount | SD | Amount | SD |
| **2** | 93 | 3.35 | 85 | 3.11 |
| **4** | 178 | 8.63 | 172 | 4.27 |
| **6** | 275 | 9.60 | 255 | 3.90 |
| **8** | 361 | 9.75 | 325 | 18.3 |
| **24** | 860 | 12.6 | 785 | 17.3 |

**EXAMPLES 3A AND 3B**

**Active-containing coating composition**

The formulations of the active-containing coating compositions of Examples 3A and 3B are summarized in Table 3.1 below. The solids %-values refer to the amounts (Amt) in % by weight.

Table 3.1

| Ingredient (Trade Name) | Ex. 3A | | Ex. 3B | |
|---|---|---|---|---|
| | Amt [g] | Solids [%] | Amt [g] | Solids [%] |
| Rivastigmine base | 10 | 20.0 | 10 | 20.0 |
| Acrylate vinylacetate adhesive in ethyl acetate Solids content of 40 % by weight (DURO-TAK 87-4098 from Henkel) | 5 | 10.0 | 5 | 10.0 |
| Silicone acrylic hybrid pressure sensitive adhesive in ethyl acetate Solids content of 50 % by weight (PSA SilAc 7-6302 from Dow Corning Healthcare) | 35 | 70.0 | 35 | 70.0 |
| Total | 50 | 100.0 | 50 | 100.0 |
| Area Weight [g/m²] | 90 | | 90 | |
| Loading API [μg/cm²] | 1800 | | 1800 | |

**Preparation of the active-containing coating composition**

A beaker was loaded with the silicone acrylic hybrid pressure-sensitive adhesive having a solids content of about 50 % by weight (PSA SilAc 7-6302 from Dow Corning Healthcare). The acrylate vinylacetate adhesive with a silds content of about 40 % by weight (DURO-TAK 87-4098 from Henkel) and the rivastigmine base were added under stirring. The mixture was stirred at about 800 rpm until a homogenous mixture was obtained (at least 20 min).

**Coating of the active-containing coating composition**

The resulting active-containing coating composition was coated within less than 24 h on an abhesively equipped foil (Scotchpak 9755 AB1F) using hand over knife lab coating equipment, using an erichson coater. The solvent was removed by drying in a first step at about room temperature (23 ± 2 °C) for about 10 min, followed by a second drying step at about 60 °C for about 20 min.

The coating thickness was chosen such that removal of the solution results in an area weight of the active-containing layer of about 90.0 g/m$^2$.

The resulting active-containing layer was then laminated with a backing layer (FO PET 23 μm transparent).

**Active-free coating composition**

The formulation of the active-free coating composition of Examples 3A and 3B are summarized in Table 3.2 below. The solids %-values refer to the amounts (Amt) in % by weight.

Table 3.2

| **Ingredient (Trade Name)** | **Ex. 3A** | | **Ex. 3B** | |
|---|---|---|---|---|
| | **Amt [g]** | **Solids [%]** | **Amt [g]** | **Solids [%]** |
| Component A of 2 component Soft Skin Adhesive from Dow Corning (MG 7-9900 (A)) | 15 | 50.0 | 15 | 50.0 |
| Component B of 2 component Soft Skin Adhesive from Dow Corning (MG 7-9900 (B)) | 15 | 50.0 | 15 | 50.0 |
| Total | 30 | 100.0 | 30 | 100.0 |
| Area Weight [g/m$^2$] | 40 | | 85 | |
| Layer thickness [μm] | 50 | | 100 | |

**Preparation of the active-free coating composition**

Both components were weighed separately into a suitable vessel, e.g. a glass vessel. Subsequently, component A was added to the mixing vessel followed by component B. Then the mixture was mixed at approx. 200 rpm for approx. 5 min until a homogeneous mixture of Component A and Component B was obtained.

**Coating of the active-free coating composition**

Within a time frame of approx. 30 min, the resulting active-free coating composition was coated on an abhesively equipped foil using hand over knife lab coating equipment, e.g. an erichson coater. The coating temperature was set to 120°C. The resulting active-free layer was heated at this temperature for approx. 5 min.

The coating thickness was chosen such that removal of the solvents resulted in a layer thickness of the active-free (skin contact) layer of approx. 50 μm (for Ex. 3A) and 100 μm (for Ex. 3B).

The resulting active-free (skin contact) layer was laminated with a release liner (FEP, 100 μm).

**Lamination of the active-containing layer and the active-free (skin contact) layer**

The active-free (skin contact) layer was then laminated with the active-containing layer. For this purpose, the abhesively equipped foils used for the coating and drying of the layers were removed and the resulting open sides of the active-containing layer and the active-free (skin contact) layer were laminated together resulting in an active-containing self-adhesive layer structure comprising the backing layer, the active-containing layer, and the active-free (skin contact) layer, wherein the active-containing layer is attached to the backing layer, and the active-free (skin contact) layer is attached to the active-containing layer, and wherein the structure is closed by a release liner, which is attached to the active-free (skin contact layer).

**Preparation of the TTS**

The individual systems (TTS) were punched out from the active-containing self-adhesive layer structure obtained as described above. Then, the TTS were sealed into pouches of the primary packaging material.

**Measurement of the skin permeation**

The permeated amounts of the TTS prepared according to Examples 3A and 3B were determined as described

for Examples 1A and 1B.

The results are shown in Table 3.3 and in Figure 2.

Table 3.3

| Cumulative permeated amount with SD [μg/cm²] | | | | |
|---|---|---|---|---|
| **Elapsed time [h]** | **Ex. 3A (n = 3)** | | **Ex. 3B (n = 3)** | |
| | **Amount** | **SD** | **Amount** | **SD** |
| **2** | 116 | 1.53 | 116 | 4.93 |
| **4** | 239 | 3.61 | 237 | 8.14 |
| **6** | 359 | 4.93 | 356 | 10.6 |
| **8** | 467 | 6.11 | 463 | 12.5 |
| **24** | 1063 | 8.50 | 1045 | 20.5 |

**EXAMPLES 4A AND 4B**

**Active-containing coating composition**

The formulations of the active-containing coating compositions of Examples 4A and 4B are summarized in Table 4.1 below. The solids %-values refer to the amounts (Amt) in % by weight.

Table 4.1

| **Ingredient (Trade Name)** | **Ex. 4A** | | **Ex. 4B** | |
|---|---|---|---|---|
| | **Amt [g]** | **Solids [%]** | **Amt [g]** | **Solids [%]** |
| Rivastigmine base | 10 | 20.0 | 10 | 20.0 |
| Acrylate vinylacetate adhesive in ethyl acetate<br>Solids content of 40 % by weight (DURO-TAK 87-4098 from Henkel) | 5 | 10.0 | 5 | 10.0 |
| Silicone acrylic hybrid pressure sensitive adhesive in ethyl acetate<br>Solids content of 50 % by weight (PSA SilAc 7-6302 from Dow Corning Healthcare) | 35 | 70.0 | 35 | 70.0 |
| Total | 50 | 100.0 | 50 | 100.0 |
| Area Weight [g/m²] | 90 | | 90 | |
| Loading API [μg/cm²] | 1800 | | 1800 | |

**Preparation of the active-containing coating composition**

A beaker was loaded with the silicone acrylic hybrid pressure-sensitive adhesive having a solids content of about 50 % by weight (PSA SilAc 7-6302 from Dow Corning Healthcare). The acrylate vinylacetate adhesive with a solds content of 40 % by weight (DURO-TAK 87-4098 from Henkel) and the rivastigmine base were added under stirring. The mixture was stirred at about 800 rpm until a homogenous mixture was obtained (at least 20 min).

**Coating of the active-containing coating composition**

The resulting active-containing coating composition was coated within less than 24 h on an abhesively equipped foil (Scotchpak 9755 AB1F) using hand over knife lab coating equipment, using an erichson coater. The solvent was removed by drying in a first step at about room temperature (23 ± 2 °C) for about 10 min, followed by a second drying step at about 60 °C for about 20 min.

The coating thickness was chosen such that removal of the solution results in an area weight of the active-containing layer of about 90.0 g/m².

The resulting active-containing layer was then laminated with a backing layer (FO PET 23 μm transparent).

**Active-free coating composition**

The formulation of the active-free coating composition of Examples 4A and 4B are summarized in Table 4.2 below. The solids %-values refer to the amounts (Amt) in % by weight.

Table 4.2

| Ingredient (Trade Name) | Ex. 4A | | Ex. 4B | |
|---|---|---|---|---|
| | Amt [g] | Solids [%] | Amt [g] | Solids [%] |
| Component A of 2 component Soft Skin Adhesive from Dow Corning (MG 7-1010 (A)) | 25 | 50.0 | 25 | 50.0 |
| Component B of 2 component Soft Skin Adhesive from Dow Corning (MG 7-1010 (B)) | 25 | 50.0 | 25 | 50.0 |
| Total | 50 | 100.0 | 50 | 100.0 |
| Area Weight [g/m$^2$] | 45 | | 61 | |
| Layer thickness [$\mu$m] | 50 | | 100 | |

**Preparation of the active-free coating composition**

Both components were weighed separately into a suitable vessel, e.g. a glass vessel. Subsequently, component A was added to the mixing vessel followed by component B. Then the mixture was mixed at approx. 200 rpm for approx. 5 min until a homogeneous mixture of Component A and Component B was obtained.

**Coating of the active-free coating composition**

Within a time frame of approx. 30 min, the resulting active-free coating composition was coated on an abhesively equipped foil using hand over knife lab coating equipment, e.g. an erichson coater. The coating temperature was set to 120 °C. The resulting active-free layer was heated at this temperature for approx. 5 min.

The coating thickness was chosen such that removal of the solvents resulted in a layer thickness of the active-free (skin contact) layer of approx. 50 $\mu$m (for Ex. 4A) and 100 $\mu$m (for Ex. 4B).

The resulting active-free (skin contact) layer was laminated with a Scotchpak 9709 release liner (fluorosilicone coated polyester film).

**Lamination of the active-containing layer and the active-free (skin contact) layer**

The active-free (skin contact) layer was then laminated with the active-containing layer. For this purpose, the abhesively equipped foils used for the coating and drying of the layers were removed and the resulting open sides of the active-containing layer and the active-free (skin contact) layer were laminated together resulting in an active-containing self-adhesive layer structure comprising the backing layer, the active-containing layer, and the active-free (skin contact) layer, wherein the active-containing layer is attached to the backing layer, and the active-free (skin contact) layer is attached to the active-containing layer, and wherein the structure is closed by a release liner, which is attached to the active-free (skin contact layer).

**Preparation of the TTS**

The individual systems (TTS) were punched out from the active-containing self-adhesive layer structure obtained as described above. Then, the TTS were sealed into pouches of the primary packaging material.

**Measurement of the skin permeation**

The permeated amounts of the TTS prepared according to Examples 4A and 4B were determined as described for Examples 1A and 1B.

The results are shown in Table 4.3 and in Figure 2.

Table 4.3

| Cumulative permeated amount with SD [$\mu$g/cm$^2$] | | | | |
|---|---|---|---|---|
| **Elapsed time [h]** | **Ex. 4A (n = 3)** | | **Ex. 4B (n = 3)** | |
| | **Amount** | **SD** | **Amount** | **SD** |
| **2** | 96 | 2.40 | 90.1 | 1.07 |
| **4** | 199 | 1.60 | 185 | 4.24 |
| **6** | 299 | 2.01 | 276 | 7.49 |
| **8** | 388 | 4.23 | 359 | 9.90 |
| **24** | 891 | 18.9 | 827 | 23.5 |

## EXAMPLE 5

### Active-containing coating composition

The formulation of the active-containing coating composition of Example 5 is summarized in Table 5.1 below. The solids %-values refer to the amounts (Amt) in % by weight.

Table 5.1

| Ingredient (Trade Name) | Ex. 5 | |
|---|---|---|
| | **Amt [g]** | **Solids [%]** |
| Buprenorphine base | 1.368 | 10.0 |
| Levulinic acid | 0.958 | 7.0 |
| Ethanol | 1.938 | - |
| Polyvinylpyrrolidone (provided in the form of a 25 % PVP pre-solution in ethanol) | 0.342 | 2.5 |
| Ascorbyl palmitate | 0.027 | 0.2 |
| Silicone-based polymer in n-heptane Solids content of 73 % by weight (BIO-PSA Q7-4201 from Dow Corning) | 15.048 | 80.3 |
| n-Heptane | 0.319 | - |
| Total | 20 | 100.0 |
| Area Weight [g/m$^2$] | 90 | |

### Preparation of the active-containing coating composition

In a 10 L vessel, 1.00 kg of polyvinylpyrrolidone and 3.00 kg of ethanol were dissolved to form a 25 % PVP pre-solution. In a homogenizing/mixing vessel (Becomix Lab mixer RW 30 Ex), 1.368 kg of the PVP pre-solution, 0.958 kg levulinic acid, 0.027 kg ascorbyl palmitate and a first part of the ethanol (0.912 kg) were suspended by stirring. The prescribed amount of the buprenorphine base was weighed and added to the homogenizing/mixing vessel followed by rinsing the weighing container used for buprenorphine with the remaining part of ethanol. The mixture was kept under stirring for at least 1h until a buprenorphine base-containing solution was formed. 15.048 kg of the silicone-based polymer in the form of a solution in n-heptane having a solids content of 73 % by weight and 0.319 kg of n-heptane were added to the mixing/homogenizing vessel. The mixture was stirred for at least 2 h until a buprenorphine base-containing adhesive mixture with 6.8 % by weight of buprenorphine, with a solids content of 6 % was formed. Afterwards, this mixture was homogenized using a rotor-stator device using homogenizing unit at approx. 2250 rpm.

### Coating of the active-containing coating composition

Within 24 hours the resulting active-containing coating composition was coated on a polyethylene terephthalate foil (Scotchpak from 3M) using a pilot plant roll coater including a drying tunnel, several drying sections, an unwinding and

laminating station. The solvent was removed by drying at approximately 30-50 °C. The layer remained within the drying tunnel at approx. 8 minutes.

The coating thickness was chosen such that removal of the solvents results in an area weight of the active-containing layer of about 90.0 g/m$^2$. This results in 10 % by weight of buprenorphine, 7 % by weight of levulinic acid, 2.5 % by weight of polyvinylpyrrolidone (PVP), 0.2 % by weight of ascorbyl palmitate, and 80.3 % by weight of silicone-based polymer in this layer.

The resulting active-containing layer was then laminated with a backing layer (e.g. polyethylenterephthalate (PET) foil, 19 $\mu$m).

**Active-free coating composition**

The formulation of the active-free coating composition of Example 5 is summarized in Table 5.2 below. The solids %-values refer to the amounts (Amt) in % by weight.

Table 5.2

| Ingredient (Trade Name) | Ex. 5 | |
|---|---|---|
| | Amt [g] | Solids [%] |
| Component A of 2 component Soft Skin Adhesive from Dow Corning (MG 7-9900 (A)) | 25.0 | 50.0 |
| Component B of 2 component Soft Skin Adhesive from Dow Corning (MG 7-9900 (B)) | 25.0 | 50.0 |
| Total | 50.0 | 100.0 |
| Area Weight [g/m$^2$] | 20 | |

**Preparation of the active-free coating composition**

Both components were weighed separately into a suitable vessel, e.g. a glass vessel. Subsequently, component A was added to the mixing vessel followed by component B. Then the mixture was mixed at approx. 200 rpm for approx. 5 min until a homogeneous mixture of Component A and Component B was obtained.

**Coating of the active-free coating composition**

Within a time frame of approx. 30 min, the resulting active-free coating composition was coated on an abhesively equipped foil using hand over knife lab coating equipment, e.g. an erichson coater. The coating temperature was set to 120 °C. The resulting active-free layer was heated at this temperature for approx. 5 min.

The coating thickness was chosen such that removal of the solvents results in an area weight of the skin contact layer of about 20 g/m$^2$. This results in 100 % by weight of Soft Skin Adhesive in the active-free (skin contact) layer.

The resulting active-free (skin contact) layer was laminated with a release liner (FEP, 100 $\mu$m).

**Lamination of the active-containing layer and the active-free (skin contact) layer**

The active-free (skin contact) layer was then laminated with the active-containing layer. For this purpose, the abhesively equipped foils used for the coating and drying of the layers were removed and the resulting open sides of the active-containing layer and the active-free (skin contact) layer were laminated together resulting in an active-containing self-adhesive layer structure comprising the backing layer, the active-containing layer, and the active-free (skin contact) layer, wherein the active-containing layer is attached to the backing layer, and the active-free (skin contact) layer is attached to the active-containing layer, and wherein the structure is closed by a release liner, which is attached to the active-free (skin contact layer).

**Preparation of the TTS**

The individual systems (TTS) were punched out from the active-containing self-adhesive layer structure obtained as described above.

**Measurement of the skin permeation**

The permeated amounts of the TTS prepared according to Example 5 were determined by experiments in

accordance with the OECD Guideline (adopted April 13, 2004) carried out with a 9.0 ml Franz diffusion cell. Split thickness human skin from cosmetic surgeries (female abdomen, date of birth 1988) was used. A dermatome was used to prepare skin of a thickness of 800 μm with an intact epidermis for all TTS. Due to the prolonged test (168 hours) 800 μm skin is used instead of the recommended 200 to 400 μm skin. Diecuts with an area of release of 1.191 $cm^2$ were punched from the TTS. The permeated amount of buprenorphine in the receptor medium of the Franz diffusion cell (phosphate buffer solution pH 5.5 with 0.1 % sodium azide as antibacteriological agent) at a temperature of 32 ± 1 °C was measured.

The results are shown in Table 5.3 and in Figure 3.

Table 5.3

| **Cumulative permeated amount with SD [μg/cm²]** | | |
|---|---|---|
| **Elapsed time [h]** | **Ex. 5 (n = 3)** | |
| | **Amount** | **SD** |
| **8** | 0.00 | 0.00 |
| **24** | 6.69 | 1.80 |
| **32** | 14.63 | 3.30 |
| **48** | 37.87 | 7.38 |
| **72** | 89.30 | 15.84 |
| **144** | 275.00 | 27.51 |
| **168** | 341.00 | 28.00 |

**Measurement of the in-vitro release rate**

The amount of buprenorphine released from TTS prepared according to Example 5 was determined by in vitro dissolution experiments using the rotating cylinder method according to USP 724 and Pharmacopeia 2.9.4. Acetate buffer pH 4.5 ± 0.1 was used as release medium. To this end, it was filled into the dissolution vessel with a volume of 600 ml and heated to 32 °C ± 0.5 °C. Diecuts of 10 $cm^2$ were punched out of the TTS, reinforced on the back side with a polyester film (Hostaphan RN 100, Kalle) and laminated with double-sided adhesive tape (Duplocoll 3205, Lohmann) on one side. The back side of the diecut was fixed to the cylinder using double-sided adhesive tape (Duplocoll 3205, Lohmann). Subsequently, the release liner was removed from the TTS. The cylinder was immersed into the release medium and immediately set to rotate with a speed of 50 rpm. Samples of 10 ml were drawn from the release medium after 0.5, 2, 8, 24 and 48 hours. The sample volume was not replaced by new release medium. The samples were analyzed for their buprenorphine content by HPLC using a stainless steel column (150 mm x 4 mm internal diameter packed with ODS phase HyperSil ODS, Thermo Corp., VDS Optilab), an acetonitrile/0.05 M $KH_2PO_4$ solution (50:50 (v/v), pH 3.5) as mobile phase (flow rate 1.0 mL/min, injection volume 20 μl) and UV-detection at 220 nm.

The results are shown in Table 5.4.

Table 5.4

| **Cumulative amount released with SD [mg]** | | |
|---|---|---|
| **Elapsed time [h]** | **Ex. 5 (n = 3)** | |
| | **Amount** | **SD** |
| **0.5** | 0.32 | 0.08 |
| **2** | 1.02 | 0.11 |
| **8** | 2.55 | 0.09 |
| **24** | 5.22 | 0.10 |
| **48** | 7.92 | 0.07 |

**Measurement of adhesion force**

Adhesion force tests were performed with the TTS using a tensile strength testing machine. Prior to testing, the

samples were equilibrated 24 hours under controlled conditions at approx. room temperature (23 ± 2 °C) and approx. 50 % rh (relative humidity). Further, the samples were cut into pieces with a fixed width of 25 mm and a suitable length. The first millimeters of the abhesively equipped foil was pulled off and a splicing tape is applied to the opened adhesive side of the buprenorphine-containing layer structure. Then, the abhesively equipped foil was totally removed and the sample was placed with the adhesive surface in longitudinal direction onto the center of the cleaned testing plate (aluminum). The testing plate was fixed to the lower clamp of the tensile strength machine. The machine was adjusted to zero, the splicing tape was gripped into the upper clamp of the machine. The pull angle was set to 90°. After measurement of the adhesion force of three samples, the mean value of the adhesion force was calculated. The measurement value is based on units "N/sample width" [N/25mm].

The results are shown in Table 5.5 below.

Table 5.5

| **Adhesion force** [N/25 mm] |
|---|
| **Ex. 5 (n = 3)** |
| 0.8 |

**Measurement of tack**

Tack (the force which is required to separate an object from an adhesive surface after a short time of contact) tests were performed with the TTS in accordance with the Standard Test Method for Pressure-Sensitive Tack of Adhesives Using an Inverted Probe Machine (ASTM D 2979 - 01; Reapproved 2009) using a probe tack tester PT-1000 (ChemInstruments). Prior to testing, the samples were equilibrated 24 hours under controlled conditions at approx. room temperature (23 ± 2 °C) and approx. 50 % rh. For determining the tack, the tip of a cleaned probe with a diameter of 5 mm was brought into contact with the adhesive surface of the buprenorphine-containing adhesive matrix layer for 1 second, at a defined rate (10 ± 0.1 mm/s), under defined pressure (9.79 ± 0.10 kPa), at a given temperature (23 ± 2 °C) and the bond formed between probe and the adhesive was subsequently broken at the same rate. Tack was measured as the maximum force required to break the adhesion bond (see ASTM D2979 - 01; Reapproved 2009). After finalization the mean value from the individual results of three associated samples were calculated and the mean tack value reported in [N].

The results are shown in Table 5.6 below.

Table 5.6

| **Tack** [N] |
|---|
| **Ex. 5 (n = 3)** |
| 0.77 |

**EXAMPLES 6A AND 6B**

**Active-containing coating composition**

The formulations of the active-containing coating compositions of Examples 6A and 6B are summarized in Table 6.1 below. The solids %-values refer to the amounts (Amt) in % by weight.

Table 6.1

| **Ingredient (Trade Name)** | **Ex. 6A** | | **Ex. 6B** | |
|---|---|---|---|---|
| | **Amt [g]** | **Solids [%]** | **Amt [g]** | **Solids [%]** |
| Buprenorphine base | 9.00 | 10 | 9.20 | 10 |
| Levulinic acid | 6.30 | 7 | 6.44 | 7 |
| Ethanol | 8.67 | - | 6.13 | - |
| Polyvinylpyrrolidone (provided in the form of a 25 % PVP pre-solution in ethanol) | - | - | 9.20 | 2.5 |

(continued)

| Ingredient (Trade Name) | Ex. 6A | | Ex. 6B | |
|---|---|---|---|---|
| | Amt [g] | Solids [%] | Amt [g] | Solids [%] |
| Ascorbyl palmitate | 0.18 | 0.2 | 0.18 | 0.2 |
| Silicone acrylic hybrid pressure sensitive adhesive in n-heptane Solids content of 50 % by weight (PSA SilAc 7-6101 from Dow Corning) | 149.04 | 82.8 | - | - |
| Silicone acrylic hybrid pressure sensitive adhesive in ethyl acetate Solids content of 50 % by weight (PSA SilAc 7-6102 from Dow Corning) | - | - | 147.75 | 80.3 |
| n-Heptane | 0.22 | - | 0.08 | - |
| Total | 175.3 | 100.0 | 179.0 | 100.0 |
| Area Weight [g/m$^2$] | 90 | | 90 | |

**Preparation of the active-containing coating composition**

In a 250 mL wide-neck glass, the buprenorphine base was suspended in levulinic acid, ethanol, if applicable the PVP solution, and ascorbyl palmitate, and the mixture was stirred until complete dissolution of buprenorphine. The silicone acrylic hybrid pressure-sensitive adhesive in the form of a mixture in n-heptane or ethyl acetate having a solids content of 50 % by weight and n-heptane to adjust the solids content were added. The mixture was stirred until homogeneous to give a buprenorphine-containing coating composition with 5.19 % (Ex. 6A) and 5.14 % (Ex. 6B) by weight of buprenorphine, with a solids content of 51.9 % (Ex. 6A) and 51.4 % (Ex. 6B), respectively.

**Coating of the active-containing coating composition**

Within 24 hours the resulting active-containing coating composition was coated on an abhesively equipped foil (Scotchpak from 3M) using hand over knife lab coating equipment, using an erichson coater. The solvent was removed by drying in a first step at approx. room temperature (23 ± 2 °C) for approx. 10 min, followed by a second drying step at approx. 75 °C for approx. 10 min.

The coating thickness was chosen such that removal of the solvents results in an area weight of the active-containing layer of about 90.0 g/m$^2$.

The resulting active-containing layer was then laminated with a backing layer (e.g. polyethylenterephthalate (PET) foil, 19 μm).

**Active-free coating composition**

The formulations of the active-free coating compositions of Examples 6A and 6B are summarized in Table 6.2 below. The solids %-values refer to the amounts (Amt) in % by weight.

Table 6.2

| Ingredient (Trade Name) | Ex. 6A | | Ex. 6B | |
|---|---|---|---|---|
| | Amt [g] | Solids [%] | Amt [g] | Solids [%] |
| Component A of 2 component Soft Skin Adhesive from Dow Corning (MG 7-1010 (A)) | 25.0 | 50.0 | 25.0 | 50.0 |
| Component B of 2 component Soft Skin Adhesive from Dow Corning (MG 7-1010 (B)) | 25.0 | 50.0 | 25.0 | 50.0 |
| Total | 50.0 | 100.0 | 50.0 | 100.0 |
| Area Weight [g/m$^2$] | 20 | | 20 | |

**Preparation of the active-free coating composition**

Both components were weighed separately into a suitable vessel, e.g. a glass vessel. Subsequently, component A was added to the mixing vessel followed by component B. Then the mixture was mixed at approx. 200 rpm for approx. 5 min until a homogeneous mixture of Component A and Component B was obtained.

**Coating of the active-free coating composition**

Within a time frame of approx. 30 min, the resulting active-free coating composition was coated on an abhesively equipped foil using hand over knife lab coating equipment, e.g. an erichson coater. The coating temperature was set to 120 °C. The resulting active-free layer was heated at this temperature for approx. 5 min.

The coating thickness was chosen such that removal of the solvents results in an area weight of the skin contact layer of about 20 g/m$^2$. This results in 100 % by weight of Soft Skin Adhesive in the active-free (skin contact) layer.

The resulting active-free (skin contact) layer was laminated with a release liner (FEP, 100 µm).

**Lamination of the active-containing layer and the active-free (skin contact) layer**

The active-free (skin contact) layer was then laminated with the active-containing layer. For this purpose, the abhesively equipped foils used for the coating and drying of the layers were removed and the resulting open sides of the active-containing layer and the active-free (skin contact) layer were laminated together resulting in an active-containing self-adhesive layer structure comprising the backing layer, the active-containing layer, and the active-free (skin contact) layer, wherein the active-containing layer is attached to the backing layer, and the active-free (skin contact) layer is attached to the active-containing layer, and wherein the structure is closed by a release liner, which is attached to the active-free (skin contact layer).

**Preparation of the TTS**

The individual systems (TTS) were punched out from the active-containing self-adhesive layer structure obtained as described above.

**Measurement of the skin permeation**

The permeated amounts of the TTS prepared according to Examples 6A and 6B were determined as described for Example 5.

The results are shown in Table 6.3 and in Figure 4.

Table 6.3

| Cumulative permeated amount with SD [µg/cm$^2$] | | | | |
|---|---|---|---|---|
| **Elapsed time [h]** | **Ex. 6A (n = 3)** | | **Ex. 6B (n = 3)** | |
| | **Amount** | **SD** | **Amount** | **SD** |
| **8** | 0.65 | 1.13 | 2.18 | 1.12 |
| **24** | 14.14 | 8.99 | 24.60 | 8.32 |
| **32** | 25.33 | 14.08 | 40.53 | 11.75 |
| **48** | 51.27 | 21.84 | 73.20 | 17.86 |
| **72** | 85.90 | 29.90 | 113.33 | 22.74 |
| **144** | 212.67 | 43.02 | 254.00 | 23.52 |
| **168** | 254.00 | 44.14 | 298.33 | 20.23 |

**Measurement of the in-vitro release rate**

The amounts of buprenorphine released from TTS prepared according to according to Examples 6A and 6B were determined as described for Example 5.

The results are shown in Table 6.4.

Table 6.4

| Cumulative amount released with SD [mg] | | | | |
|---|---|---|---|---|
| **Elapsed time [h]** | **Ex. 6A (n = 3)** | | **Ex. 6B (n = 3)** | |
| | **Amount** | **SD** | **Amount** | **SD** |
| **0.5** | 0.14 | 0.00 | 0.23 | 0.01 |
| **2** | 0.37 | 0.01 | 0.50 | 0.02 |
| **8** | 1.08 | 0.03 | 1.35 | 0.04 |
| **24** | 2.43 | 0.08 | 2.83 | 0.09 |
| **48** | 4.14 | 0.10 | 4.67 | 0.09 |

**Measurement of adhesion force**

Adhesion force tests were performed with the TTS according to Examples 6A and 6B as described for Example 5.
The results are shown in Table 6.5 below.

Table 6.5

| **Adhesion force** [N/25 mm] | |
|---|---|
| **Ex. 6A (n = 3)** | **Ex. 6B (n = 3)** |
| 4.2 | complete break |

**Measurement of tack**

Tack tests were performed with the TTS according to Examples 6A and 6B as described for Example 5.
The results are shown in Table 6.6 below.

Table 6.6

| **Tack** [N] | |
|---|---|
| **Ex. 6A (n = 3)** | **Ex. 6B (n = 3)** |
| 0.89 | 0.73 |

**COMPARATIVE EXAMPLE 1**

**TTS**

Comparative Example 1 (Comp. 1) is the commercially available rivastigmine-containing TTS product Exelon®, having a rivastigmine-containing acrylic based layer (60 g/$m^2$) and a rivastigmine-free silicone based skin contact layer (30 g/$m^2$), which is available from Novartis Pharma.

**Measurement of the skin permeation**

The permeated amount of the commercially available Exelon® TTS (Comp. 1) was determined by experiments in accordance with the EMA Guideline on quality of transdermal patches (adopted October 23, 2014) carried out with a 10.0 ml Franz diffusion cell, wherein an EVA-membrane (9 % vinyl acetate; Nitroderm TTS K-Membrane 343 mm from PetroplastVinora AG) having a thickness of 50 $\mu$m was used. Diecuts with an area of release of 1.118 $cm^2$ were punched from the TTS. The permeated amount of rivastigmine in the receptor medium of the Franz diffusion cell (phosphate buffer solution pH 5.5 with 0.1 % sodium azide as antibacteriological agent) at a temperature of 32 ± 1 °C was measured.
The results are shown in Table 7 and in Figures 1 and 2.

Table 7

| Cumulative permeated amount with SD [μg/cm²] | | |
|---|---|---|
| Elapsed time [h] | Comp. 1 (n=3) | |
| | Amount | SD |
| **2** | 90 | 1.37 |
| **4** | 184 | 1.18 |
| **6** | 272 | 0.81 |
| **8** | 350 | 1.15 |
| **24** | 816 | 3.61 |

## COMPARATIVE EXAMPLE 2

### Active-containing coating composition

The formulation of the active-containing coating composition of Comparative Example 2 (Comp. 2) is summarized in Table 8.1 below. The solids %-values refer to the amounts (Amt) in % by weight.

Table 8.1

| Ingredient (Trade Name) | Comp. 2 | |
|---|---|---|
| | Amt [g] | Solids [%] |
| Buprenorphine base | 1.368 | 10.0 |
| Levulinic acid | 0.958 | 7.0 |
| Ethanol | 1.938 | - |
| Polyvinylpyrrolidone (provided in the form of a 25 % PVP pre-solution in ethanol) | 0.342 | 2.5 |
| Ascorbyl palmitate | 0.027 | 0.2 |
| Silicone-based polymer in n-heptane Solids content of 73 % by weight (BIO-PSA Q7-4201 from Dow Corning) | 15.048 | 80.3 |
| n-Heptane | 0.319 | - |
| Total | 20 | 100.0 |
| Area Weight [g/m²] | 90 | |

### Preparation of the active-containing coating composition

In a 10 L vessel, 1.00 kg of polyvinylpyrrolidone and 3.00 kg of ethanol were dissolved to form a 25 % PVP pre-solution. In a homogenizing/mixing vessel (Becomix Lab mixer RW 30 Ex), 1.368 kg of the PVP pre-solution, 0.958 kg levulinic acid, 0.027 kg ascorbyl palmitate and a first part of the ethanol (0.912 kg) were suspended by stirring. The prescribed amount of the buprenorphine base was weighed and added to the homogenizing/mixing vessel followed by rinsing the weighing container used for buprenorphine with the remaining part of ethanol. The mixture was kept under stirring for at least 1h until a buprenorphine base-containing solution was formed. 15.048 kg of the silicone-based polymer in the form of a solution in n-heptane having a solids content of 73 % by weight and 0.319 kg of n-heptane were added to the mixing/homogenizing vessel. The mixture was stirred for at least 2 h until a buprenorphine base-containing adhesive mixture with 6.8 % by weight of buprenorphine, with a solids content of 6 % was formed. Afterwards, this mixture was homogenized using a rotor-stator device using homogenizing unit at approx. 2250 rpm.

### Coating of the active-containing coating composition

Within 24 hours the resulting active-containing coating composition was coated on a polyethylene terephthalate foil (Scotchpak from 3M) using a pilot plant roll coater including a drying tunnel, several drying sections, an unwinding and

laminating station. The solvent was removed by drying at approximately 30-50 °C. The layer remained within the drying tunnel at approx. 8 minutes.

The coating thickness was chosen such that removal of the solvents results in an area weight of the active-containing layer of about 90.0 g/$m^2$. This results in 10 % by weight of buprenorphine, 7 % by weight of levulinic acid, 2.5 % by weight of polyvinylpyrrolidone (PVP), 0.2 % by weight of ascorbyl palmitate, and 80.3 % by weight of silicone-based polymer in this layer.

The resulting active-containing layer was then laminated with a backing layer (e.g. polyethylenterephthalate (PET) foil, 19 μm).

**Preparation of the TTS**

The individual systems (TTS) were then punched from the buprenorphine-containing self-adhesive layer structure obtained as described above.

**Measurement of the skin permeation**

The permeated amounts of the TTS prepared according to Comp. 2 were determined as described for Example 5.

The results are shown in Table 8.2 and in Figure 3.

Table 8.2

| Cumulative permeated amount with SD [μg/$cm^2$] | | |
|---|---|---|
| **Elapsed time [h]** | **Comp. 2 (n = 3)** | |
| | **Amount** | **SD** |
| **8** | 1.11 | 0.28 |
| **24** | 22.17 | 3.61 |
| **32** | 39.27 | 6.03 |
| **48** | 77.57 | 10.42 |
| **72** | 140.33 | 15.95 |
| **144** | 320.67 | 29.01 |
| **168** | 380.67 | 30.57 |

**Measurement of the in-vitro release rate**

The amount of buprenorphine released from TTS prepared according to Comp. 2 was determined as described for Example 5.

The results are shown in Table 8.3.

Table 8.3

| Cumulative amount released with SD [mg] | | |
|---|---|---|
| **Elapsed time [h]** | **Comp. 2 (n = 3)** | |
| | **Amount** | **SD** |
| **0.5** | 0.49 | 0.02 |
| **2** | 1.08 | 0.03 |
| **8** | 2.24 | 0.04 |
| **24** | 4.50 | 0.08 |
| **48** | 7.17 | 0.08 |

**Measurement of adhesion force**

Adhesion force tests were performed with the TTS according to Comp. 2 as described for Example 5.

The results are shown in Table 8.4 below.

Table 8.4

| **Adhesion force** [N/25 mm] |
|---|
| **Comp. 2 (n = 3)** |
| 3.1 |

**Measurement of tack**

Tack tests were performed with the TTS according to Comp. 2 as described for Example 5.

The results are shown in Table 8.5 below.

Table 8.5

| **Tack** [N] |
|---|
| **Comp. 2 (n = 3)** |
| 0.76 |

**COMPARATIVE EXAMPLES 3A AND 3B**

**Active-containing coating composition**

The formulations of the active-containing coating compositions of Comparative Examples 3A and 3B (Comp. 3A and 3B) are summarized in Table 9.1 below. The solids %-values refer to the amounts (Amt) in % by weight.

Table 9.1

| **Ingredient (Trade Name)** | **Comp. 3A** | | **Comp. 3B** | |
|---|---|---|---|---|
| | **Amt [g]** | **Solids [%]** | **Amt [g]** | **Solids [%]** |
| Buprenorphine base | 9.00 | 10 | 9.20 | 10 |
| Levulinic acid | 6.30 | 7 | 6.44 | 7 |
| Ethanol | 8.67 | - | 6.13 | - |
| Polyvinylpyrrolidone (provided in the form of a 25 % PVP pre-solution in ethanol) | - | - | 9.20 | 2.5 |
| Ascorbyl palmitate | 0.18 | 0.2 | 0.18 | 0.2 |
| Silicone acrylic hybrid pressure sensitive adhesive in n-heptane Solids content of 50 % by weight (PSA SilAc 7-6101 from Dow Corning) | 149.04 | 82.8 | - | - |
| Silicone acrylic hybrid pressure sensitive adhesive in ethyl acetate Solids content of 50 % by weight (PSA SilAc 7-6102 from Dow Corning) | - | - | 147.75 | 80.3 |
| n-Heptane | 0.22 | - | 0.08 | - |
| Total | 175.3 | 100.0 | 179.0 | 100.0 |
| Area Weight [g/m$^2$] | 90 | | 90 | |

**Preparation of the active-containing coating composition**

In a 250 mL wide-neck glass, the buprenorphine base was suspended in levulinic acid, ethanol, if applicable the PVP solution, and ascorbyl palmitate, and the mixture was stirred until complete dissolution of buprenorphine. The silicone acrylic hybrid pressure-sensitive adhesive in the form of a mixture in n-heptane or ethyl acetate having a solids content of 50 % by weight and n-heptane to adjust the solids content were added. The mixture was stirred until homogeneous to give a buprenorphine-containing coating composition with 5.19 % (Comp. 3A) and 5.14 % (Comp. 3B) by weight of buprenorphine, with a solids content of 51.9 % (Comp. 3A) and 51.4 % (Comp. 3B), respectively.

**Coating of the active-containing coating composition**

Within 24 hours the resulting active-containing coating composition was coated on an abhesively equipped foil (Scotchpak from 3M) using hand over knife lab coating equipment, using an erichson coater. The solvent was removed by drying in a first step at approx. room temperature (23 ± 2 °C) for approx. 10 min, followed by a second drying step at approx. 75 °C for approx. 10 min.

The coating thickness was chosen such that removal of the solvents results in an area weight of the active-containing layer of about 90.0 g/m$^2$.

The resulting active-containing layer was then laminated with a backing layer (e.g. polyethylenterephthalate (PET) foil, 19 μm).

**Preparation of the TTS**

The individual systems (TTS) were then punched from the buprenorphine-containing self-adhesive layer structure obtained as described above.

**Measurement of the skin permeation**

The permeated amounts of the TTS prepared according to Comp. 3A and 3B were determined as described for Example 5.

The results are shown in Table 9.2 and in Figure 4.

Table 9.2

| Cumulative permeated amount with SD [μg/cm$^2$] | | | | |
|---|---|---|---|---|
| **Elapsed time [h]** | **Comp. 3A (n = 3)** | | **Comp. 3B (n = 3)** | |
| | **Amount** | **SD** | **Amount** | **SD** |
| **8** | 0.36 | 0.62 | 0.76 | 0.76 |
| **24** | 9.91 | 5.15 | 12.26 | 6.79 |
| **32** | 18.03 | 7.43 | 21.80 | 10.81 |
| **48** | 37.33 | 11.56 | 43.13 | 18.07 |
| **72** | 72.13 | 14.36 | 72.10 | 26.22 |
| **144** | 178.67 | 11.59 | 183.33 | 36.83 |
| **168** | 213.67 | 7.64 | 224.00 | 37.00 |

**Measurement of the in-vitro release rate**

The amount of buprenorphine released from TTS prepared according to Comp. 3A and 3B were determined as described for Example 5.

The results are shown in Table 9.3.

Table 9.3

| Cumulative amount released with SD [mg] | | | | |
|---|---|---|---|---|
| **Elapsed time [h]** | **Comp. 3A (n = 3)** | | **Comp. 3B (n = 3)** | |
| | **Amount** | **SD** | **Amount** | **SD** |
| **0.5** | 0.61 | 0.01 | 0.894 | 0.019 |
| **2** | 0.92 | 0.01 | 1.471 | 0.069 |
| **8** | 1.63 | 0.01 | 2.450 | 0.083 |
| **24** | 2.86 | 0.01 | 4.179 | 0.097 |
| **48** | 4.44 | 0.03 | 6.158 | 0.102 |

**Measurement of adhesion force**

Adhesion force tests were performed with the TTS according to Comp. 3A and 3B as described for Example 5.

The results are shown in Table 9.4 below.

Table 9.4

| **Adhesion force** [N/25 mm] | |
|---|---|
| **Comp. 3A (n = 3)** | **Comp. 3B (n = 3)** |
| 2.3 | complete break |

**Measurement of tack**

Tack tests were performed with the TTS according to Comp. 3A and 3B as described for Example 5.

The results are shown in Table 9.5 below.

Table 9.5

| **Tack [N]** | |
|---|---|
| **Comp. 3A (n = 3)** | **Comp. 3B (n = 3)** |
| 1.43 | 1.89 |

## Claims

1. Transdermal therapeutic system for the transdermal administration of an active agent comprising an active agent-containing layer structure, said active agent-containing layer structure comprising:

   A) a backing layer;
   B) an active agent-containing layer comprising at least one silicone-containing polymer; and
   C) a skin contact layer;
   wherein the skin contact layer is an adhesive layer comprising a silicone gel adhesive, which is directly attached to the active agent-containing layer,
   wherein the active agent is rivastigmine or buprenorphine.

2. Transdermal therapeutic system according to claim 1,
   wherein the active agent-containing layer comprises

   - a silicone acrylic hybrid polymer, wherein preferably the silicone acrylic hybrid polymer comprises a silicone phase and an acrylate phase in a weight ratio of from 20:80 to 90: 10, or
   - a polymer blend comprising a silicone-based polymer and an acrylic polymer, wherein the silicone-based polymer is present in an amount of at least 20 % by weight based on the total weight of the polymer blend, or

- a polymer blend comprising a silicone acrylic hybrid polymer and a silicone-based polymer, or
- a polymer blend comprising a silicone acrylic hybrid polymer and an acrylic polymer.

**3.** Transdermal therapeutic system according to claim 2,
wherein the acrylic polymer is obtainable from one or more monomers selected from acrylic acid, butylacrylate, 2-ethylhexylacrylate, glycidylmethacrylate, 2-hydroxyethylacrylate, methyl acrylate, methylmethacrylate, butylmethacrylate, t-octylacrylamide, and vinylacetate, preferably from one or more monomers selected from ethylhexylacrylate, glycidylmethacrylate, 2-hydroxyethylacrylate, and vinylacetate.

**4.** Transdermal therapeutic system according to claim 2 or 3,
wherein the silicone-based polymer is obtainable by polycondensation of silanol endblocked polydimethylsiloxane with a silicate resin.

**5.** Transdermal therapeutic system according to any one of claims 2 to 4,
wherein the silicone acrylic hybrid polymer comprises a reaction product of a silicone polymer, a silicone resin and an acrylic polymer, wherein the acrylic polymer is covalently self-crosslinked and covalently bound to the silicone polymer and/or the silicone resin.

**6.** Transdermal therapeutic system according to any one of claims 2 to 4,
wherein the silicone acrylic hybrid polymer is a silicone acrylic hybrid pressure-sensitive adhesive obtainable from

(a) a silicone-containing pressure-sensitive adhesive composition comprising acrylate or methacrylate functionality.

**7.** Transdermal therapeutic system according to any one of claims 2 to 4 or 6,
wherein the silicone acrylic hybrid polymer is a silicone acrylic hybrid pressure-sensitive adhesive comprising the reaction product of

(a) a silicone-containing pressure-sensitive adhesive composition comprising acrylate or methacrylate functionality;
(b) an ethylenically unsaturated monomer; and
(c) an initiator.

**8.** Transdermal therapeutic system according to claim 6 or 7,
wherein the silicone-containing pressure-sensitive adhesive composition comprising acrylate or methacrylate functionality comprises the condensation reaction product of

(a1) a silicone resin, and
(a2) a silicone polymer, and
(a3) a silicone-containing capping agent comprising acrylate or methacrylate functionality.

**9.** Transdermal therapeutic system according to claim 7 or 8,
wherein the ethylenically unsaturated monomer is selected from the group consisting of aliphatic acrylates, aliphatic methacrylates, cycloaliphatic acrylates, cycloaliphatic methacrylates, and combinations thereof, each of said compounds having up to 20 carbon atoms in the alkyl radical, and wherein the ethylenically unsaturated monomer is preferably a combination of 2-ethylhexyl acrylate and methyl acrylate, particularly preferably in a ratio of from 40:60 to 70:30.

**10.** Transdermal therapeutic system according to any one of claims 2 to 9,
wherein the silicone acrylic hybrid polymer contains a continuous, silicone external phase and a discontinuous, acrylic internal phase, or a continuous, acrylic external phase and a discontinuous, silicone internal phase.

**11.** Transdermal therapeutic system according to any one of claims 1 to 10,
wherein the silicone gel adhesive is obtainable by reacting a gel producing composition comprising (i) at least one alkenyl-substituted polydiorganosiloxane, (ii) at least one organosiloxane, which contains silicone-bonded hydrogen atoms, and (iii) at least one catalyst for the reaction of the SiH groups with the Si-alkenyl groups.

**12.** Transdermal therapeutic system according to any one of claims 1 to 11,

wherein the silicone gel adhesive is obtainable by reacting a gel producing composition comprising (i) a copolymer of vinylmethylsiloxane and dimethylsiloxane with (ii) methylhydrogen polysiloxane with trimethylsilyl endgroups in the presence of (iii) a platinum catalyst.

**13.** Transdermal therapeutic system according to any one of claims 1 to 12,
wherein the silicone gel adhesive is a silicate resin-reinforced silicone gel adhesive that contains from about 2 to about 45 % by weight of at least one hydroxyl substituted silicate resin.

**14.** Transdermal therapeutic system according to any one of claims 1 to 13, wherein the transdermal therapeutic system further comprises a release liner.

**15.** Transdermal therapeutic system according to any one of claims 1 to 14,
for use in a method of treatment, wherein the transdermal therapeutic system is preferably applied to the skin of the patient for at least 24 hours or at least 72 hours.

**16.** A process for manufacturing an active agent-containing layer structure for use in a transdermal therapeutic system according to any one of claims 1 to 14 comprising the steps of:

1.1) coating a coating composition comprising

- an active agent; and
- at least one silicone-containing polymer

on a first foil;
1.2) drying the coated coating composition to form the active agent-containing layer;
1.3) laminating the active agent-containing layer with a backing layer;
2.1) coating a gel producing composition comprising

(i) at least one alkenyl-substituted polydiorganosiloxane,
(ii) at least one organosiloxane, which contains silicone-bonded hydrogen atoms, and
(iii) at least one catalyst for the reaction of the SiH groups with the Si-alkenyl groups, on a second foil;

2.2) crosslinking the gel producing composition at a temperature of from 50 °C to 150 °C or by applying UV light to form the skin contact layer;
2.3) laminating the skin contact layer with a release liner;
3.1) removing the foils from the active agent-containing layer and the skin contact layer; and
3.2) laminating the open side of the active agent-containing layer onto the open side of the skin contact layer to obtain an active agent-containing layer structure.

**Patentansprüche**

**1.** Transdermales therapeutisches System zur transdermalen Verabreichung eines Wirkstoffs, umfassend eine wirkstoffhaltige Schichtstruktur, wobei die wirkstoffhaltige Schichtstruktur umfasst:

A) eine Trägerschicht
B) eine wirkstoffhaltige Schicht, die mindestens ein silikonhaltiges Polymer umfasst; und
C) eine Hautkontaktschicht;
wobei die Hautkontaktschicht eine Klebeschicht ist, die einen Silikongelklebstoff umfasst, der direkt an der wirkstoffhaltigen Schicht angebracht ist,
wobei der Wirkstoff Rivastigmin oder Buprenorphin ist.

**2.** Transdermales therapeutisches System nach Anspruch 1,
wobei die wirkstoffhaltige Schicht umfasst

- ein Silicon-Acryl-Hybridpolymer, wobei das Silicon-Acryl-Hybridpolymer vorzugsweise eine Siliconphase und eine Acrylatphase in einem Gewichtsverhältnis von 20:80 bis 90:10 umfasst, oder
- eine Polymermischung, die ein Polymer auf Silikonbasis und ein Acrylpolymer umfasst, wobei das Polymer auf

Silikonbasis in einer Menge von mindestens 20 Gew.-%, bezogen auf das Gesamtgewicht der Polymermischung, vorhanden ist, oder
- eine Polymermischung, die ein Silikon-Acryl-Hybridpolymer und ein Polymer auf Silikonbasis umfasst, oder
- eine Polymermischung, die ein Silikon-Acryl-Hybridpolymer und ein Acrylpolymer umfasst.

**3.** Transdermales therapeutisches System nach Anspruch 2,
wobei das Acrylpolymer aus einem oder mehreren Monomeren erhältlich ist, die aus Acrylsäure, Butylacrylat, 2-Ethylhexylacrylat, Glycidylmethacrylat, 2-Hydroxyethylacrylat, Methylacrylat, Methylmethacrylat, Butylmethacrylat, t-Octylacrylamid und Vinylacetat, vorzugsweise aus einem oder mehreren Monomeren, ausgewählt aus Ethylhexylacrylat, Glycidylmethacrylat, 2-Hydroxyethylacrylat und Vinylacetat.

**4.** Transdermales therapeutisches System nach Anspruch 2 oder 3,
wobei das Polymer auf Silikonbasis durch Polykondensation von Polydimethylsiloxan mit Silanol-Endgruppen mit einem Silikatharz erhältlich ist.

**5.** Transdermales therapeutisches System nach einem der Ansprüche 2 bis 4,
wobei das Silikon-Acryl-Hybridpolymer ein Reaktionsprodukt aus einem Silikonpolymer, einem Silikonharz und einem Acrylpolymer umfasst, wobei das Acrylpolymer kovalent selbstvernetzt und kovalent an das Silikonpolymer und/oder das Silikonharz gebunden ist.

**6.** Transdermales therapeutisches System nach einem der Ansprüche 2 bis 4,
wobei das Silikon-Acryl-Hybridpolymer ein Silikon-Acryl-Hybrid-Haftkleber ist, der erhältlich ist aus

(a) einer silikonhaltigen Haftklebstoffzusammensetzung umfassend Acrylat- oder Methacrylatfunktionalität.

**7.** Transdermales therapeutisches System nach einem der Ansprüche 2 bis 4 oder 6,
wobei das Silikon-Acryl-Hybrid-Polymer ein Silikon-Acryl-Hybrid-Haftkleber ist, umfassend das Reaktionsprodukt von

(a) einer silikonhaltigen Haftklebstoffzusammensetzung umfassend Acrylat- oder Methacrylatfunktionalität;
(b) ein ethylenisch ungesättigtes Monomer; und
(c) ein Initiator.

**8.** Transdermales therapeutisches System nach Anspruch 6 oder 7,
wobei die silikonhaltige Haftklebstoffzusammensetzung umfassend Acrylat- oder Methacrylatfunktionalität das Kondensationsreaktionsprodukt umfasst von

(a1) ein Silikonharz, und
(a2) ein Silikonpolymer und
(a3) ein silikonhaltiges Verkappungsmittel umfassend Acrylat- oder Methacrylatfunktionalität.

**9.** Transdermales therapeutisches System nach Anspruch 7 oder 8,
wobei das ethylenisch ungesättigte Monomer ausgewählt ist aus der Gruppe bestehend aus aliphatischen Acrylaten, aliphatischen Methacrylaten, cycloaliphatischen Acrylaten, cycloaliphatischen Methacrylaten und Kombinationen davon, wobei jede dieser Verbindungen bis zu 20 Kohlenstoffatome im Alkylrest aufweist, und wobei das ethylenisch ungesättigte Monomer vorzugsweise eine Kombination aus 2-Ethylhexylacrylat und Methylacrylat ist, besonders bevorzugt in einem Verhältnis von 40:60 bis 70:30.

**10.** Transdermales therapeutisches System nach einem der Ansprüche 2 bis 9,
wobei das Silikon-Acryl-Hybridpolymer eine kontinuierliche äußere Silikonphase und eine diskontinuierliche innere Acrylphase oder eine kontinuierliche äußere Acrylphase und eine diskontinuierliche innere Silikonphase enthält.

**11.** Transdermales therapeutisches System nach einem der Ansprüche 1 bis 10,
wobei der Silikongelklebstoff erhältlich ist durch Umsetzen einer gelbildenden Zusammensetzung, umfassend (i) mindestens ein alkenylsubstituiertes Polydiorganosiloxan, (ii) mindestens ein Organosiloxan, das silikongebundene Wasserstoffatome enthält, und (iii) mindestens einen Katalysator für die Reaktion der SiH-Gruppen mit den Si-Alkenylgruppen.

**12.** Transdermales therapeutisches System nach einem der Ansprüche 1 bis 11,
wobei der Silikongelklebstoff erhältlich ist durch Umsetzen einer gelherstellenden Zusammensetzung, die (i) ein Copolymer aus Vinylmethylsiloxan und Dimethylsiloxan mit (ii) Methylhydrogenpolysiloxan mit Trimethylsilyl-Endgruppen in Gegenwart von (iii) einem Platinkatalysator umfasst.

**13.** Transdermales therapeutisches System nach einem der Ansprüche 1 bis 12,
wobei der Silikongelklebstoff ein silikatharzverstärkter Silikongelklebstoff ist, der etwa 2 bis etwa 45 Gew.-% mindestens eines hydroxylsubstituierten Silikatharzes enthält.

**14.** Transdermales therapeutisches System nach einem der Ansprüche 1 bis 13, wobei das transdermale therapeutische System zusätzlich eine Trennfolie umfasst.

**15.** Transdermales therapeutisches System nach einem der Ansprüche 1 bis 14,
zur Verwendung in einem Behandlungsverfahren, wobei das transdermale therapeutische System vorzugsweise für mindestens 24 Stunden oder mindestens 72 Stunden auf die Haut des Patienten aufgetragen wird.

**16.** Verfahren zur Herstellung einer wirkstoffhaltigen Schichtstruktur zur Verwendung in einem transdermalen therapeutischen System nach einem der Ansprüche 1 bis 14, umfassend die folgenden Schritte:

1.1) Beschichten einer Beschichtungszusammensetzung, umfassend

- einen Wirkstoff; und
- mindestens ein silikonhaltiges Polymer

auf einer ersten Folie;
1.2) Trocknen der beschichteten Beschichtungszusammensetzung zur Bildung der wirkstoffhaltigen Schicht;
1.3) Laminieren der wirkstoffhaltigen Schicht mit einer Trägerschicht;
2.1) Beschichten einer gelbildenden Zusammensetzung, umfassend

(i) mindestens ein alkenylsubstituiertes Polydiorganosiloxan,
(ii) mindestens ein Organosiloxan, das silikongebundene Wasserstoffatome enthält, und
(iii) mindestens einen Katalysator für die Reaktion der SiH-Gruppen mit den Si-Alkenylgruppen,

auf einer zweiten Folie;
2.2) Vernetzen der gelbildenden Zusammensetzung bei einer Temperatur von 50 °C bis 150 °C oder durch Anwendung von UV-Licht zur Bildung der Hautkontaktschicht;
2.3) Laminieren der Hautkontaktschicht mit einer Trennfolie;
3.1) Entfernen der Folien von der wirkstoffhaltigen Schicht und der Hautkontaktschicht; und
3.2) Laminieren der offenen Seite der wirkstoffhaltigen Schicht auf die offene Seite der Hautkontaktschicht, um eine wirkstoffhaltige Schichtstruktur zu erhalten.

## Revendications

**1.** Système thérapeutique transdermique pour l'administration transdermique d'un agent actif comprenant une structure de couche contenant un agent actif, ladite structure de couche contenant un agent actif comprenant :

A) une couche de support ;
B) une couche contenant un agent actif comprenant au moins un polymère contenant du silicone ; et
C) une couche de contact avec la peau ;
dans lequel la couche de contact avec la peau est une couche adhésive comprenant un adhésif en gel de silicone, qui est directement attaché à la couche contenant l'agent actif,
dans lequel l'agent actif est la rivastigmine ou la buprénorphine.

**2.** Système thérapeutique transdermique selon la revendication l, dans lequel la couche contenant un agent actif comprend

- un polymère hybride acrylique de silicone, dans lequel de préférence le polymère hybride acrylique de silicone

comprend une phase silicone et une phase acrylate dans un rapport pondéral de 20/80 à 90/10, ou
- un mélange de polymères comprenant un polymère à base de silicone et un polymère acrylique, dans lequel le polymère à base de silicone est présent en une proportion d'au moins 20 % en poids par rapport au poids total du mélange de polymères, ou
- un mélange de polymères comprenant un polymère hybride acrylique de silicone et un polymère à base de silicone, ou
- un mélange de polymères comprenant un polymère hybride acrylique de silicone et un polymère acrylique.

**3.** Système thérapeutique transdermique selon la revendication 2, dans lequel le polymère acrylique peut être obtenu à partir de un ou plusieurs monomères choisis parmi l'acide acrylique, le butylacrylate, le 2-éthylhexylacrylate, le glycidylméthacrylate, le 2-hydroxyéthylacrylate, le méthylacrylate, le méthylméthacrylate, le butylméthacrylate, le t-octylacrylamide et l'acétate de vinyle, de préférence à partir d'un ou plusieurs monomères choisis parmi l'éthylhexylacrylate, le glycidylméthacrylate, le 2-hydroxyéthylacrylate et l'acétate de vinyle.

**4.** Système thérapeutique transdermique selon la revendication 2 ou 3,
dans lequel le polymère à base de silicone peut être obtenu par polycondensation de polydiméthylsiloxane à extrémité bloquée par le silanol avec une résine de silicate.

**5.** Système thérapeutique transdermique selon l'une quelconque des revendications 2 à 4,
dans lequel le polymère hybride acrylique de silicone comprend un produit de la réaction d'un polymère de silicone, d'une résine de silicone et d'un polymère acrylique, dans lequel le polymère acrylique est auto-réticulé de manière covalente et lié de manière covalente au polymère de silicone et/ou à la résine de silicone.

**6.** Système thérapeutique transdermique selon l'une quelconque des revendications 2 à 4,
dans lequel le polymère hybride acrylique de silicone est un adhésif hybride acrylique de silicone sensible à la pression qui peut être obtenu à partir de

(a) une composition adhésive sensible à la pression contenant du silicone et comprenant une fonctionnalité acrylate ou méthacrylate.

**7.** Système thérapeutique transdermique selon l'une quelconque des revendications 2 à 4 ou 6,
dans lequel le polymère hybride acrylique de silicone est un adhésif hybride acrylique de silicone sensible à la pression comprenant le produit de réaction de

(a) une composition adhésive sensible à la pression contenant du silicone comprenant une fonctionnalité acrylate ou méthacrylate ;
(b) un monomère éthyléniquement insaturé ; et
(c) un initiateur.

**8.** Système thérapeutique transdermique selon la revendication 6 ou 7,
dans lequel la composition adhésive sensible à la pression contenant du silicone comprenant une fonctionnalité acrylate ou méthacrylate comprend le produit de la réaction de condensation

a1) une résine de silicone, et
a2) un polymère de silicone ;
a3) Agent de bouchage contenant du silicone comprenant une fonctionnalité acrylate ou méthacrylate.

**9.** Système thérapeutique transdermique selon la revendication 7 ou 8,
dans lequel le monomère éthyléniquement insaturé est choisi dans le groupe constitué par les acrylates aliphatiques, les méthacrylates aliphatiques, les acrylates cycloaliphatiques, les méthacrylates cycloaliphatiques et leurs combinaisons, chacun desdits composés ayant jusqu'à 20 atomes de carbone dans le radical alkyle, et dans lequel le monomère éthyléniquement insaturé est de préférence une combinaison d'acrylate de 2-éthylhexyle et d'acrylate de méthyle, en particulier de préférence dans un rapport de 40/60 à 70/30.

**10.** Système thérapeutique transdermique selon l'une quelconque des revendications 2 à 9,
dans lequel le polymère hybride acrylique de silicone contient une phase externe de silicone continue et une phase interne acrylique discontinue, ou une phase externe acrylique continue et une phase interne de silicone discontinue.

**11.** Système thérapeutique transdermique selon l'une quelconque des revendications 1 à 10,
dans lequel l'adhésif en gel de silicone peut être obtenu en faisant réagir une composition productrice de gel comprenant (i) au moins un polydiorganosiloxane substitué par un radical alcényle, (ii) au moins un organosiloxane, qui contient des atomes d'hydrogène liés au silicone, et (iii) au moins un catalyseur pour la réaction des groupes SiH avec les groupes Si-alcényle.

**12.** Système thérapeutique transdermique selon l'une quelconque des revendications 1 à 11,
dans lequel l'adhésif en gel de silicone peut être obtenu en faisant réagir une composition produisant un gel comprenant (i) un copolymère de vinylméthylsiloxane et de diméthylsiloxane avec (ii) du méthylhydrogène polysiloxane avec des groupes d'extrémités triméthylsilyle en présence (iii) d'un catalyseur de platine.

**13.** Système thérapeutique transdermique selon l'une quelconque des revendications 1 à 12,
dans lequel l'adhésif en gel de silicone est un adhésif en gel de silicone renforcé de résine de silicate qui contient d'environ 2 à environ 45% en poids d'au moins une résine de silicate substituée par un hydroxyle.

**14.** Système thérapeutique transdermique selon l'une quelconque des revendications 1 à 13, dans lequel le système thérapeutique transdermique comprend en outre un revêtement antiadhésif.

**15.** Système thérapeutique transdermique selon l'une quelconque des revendications 1 à 14, destiné à être utilisé dans un procédé de traitement, dans lequel le système thérapeutique transdermique est appliqué de préférence sur la peau du patient pendant au moins 24 heures ou au moins 72 heures.

**16.** Un procédé de fabrication d'une structure de couche contenant un agent actif destinée à être utilisée dans un système thérapeutique transdermique selon l'une quelconque des revendications 1 à 14, comprenant les étapes consistant à :

1.1) revêtir un premier feuil d'une composition de revêtement comprenant

- un agent actif ; et
- au moins un polymère contenant du silicone ;

1.2) séchage de la composition de revêtement enduite pour former la couche contenant l'agent actif ;
1.3) stratification de la couche contenant l'agent actif avec une couche de support ;
2.1) revêtement d'une composition produisant un gel comprenant

(i) au moins un polydiorganosiloxane substitué par un groupe alcényle,
(ii) au moins un organosiloxane, qui contient des atomes d'hydrogène liés au silicone, et
(iii) au moins un catalyseur pour la réaction des groupes SiH avec les groupes Si-alcényle, sur un second feuil ;

2.2) réticulation de la composition productrice de gel à une température de 50°C à 150°C ou en appliquant une lumière UV pour former la couche de contact avec la peau ;
2.3) stratifier la couche de contact avec la peau d'un revêtement antiadhésif ;
3.1) retirer les feuils de la couche contenant l'agent actif et de la couche en contact avec la peau ; et
3.2) stratifier le côté ouvert de la couche contenant un agent actif sur le côté ouvert de la couche de contact avec la peau pour obtenir une structure de couche contenant un agent actif.

FIG. 1

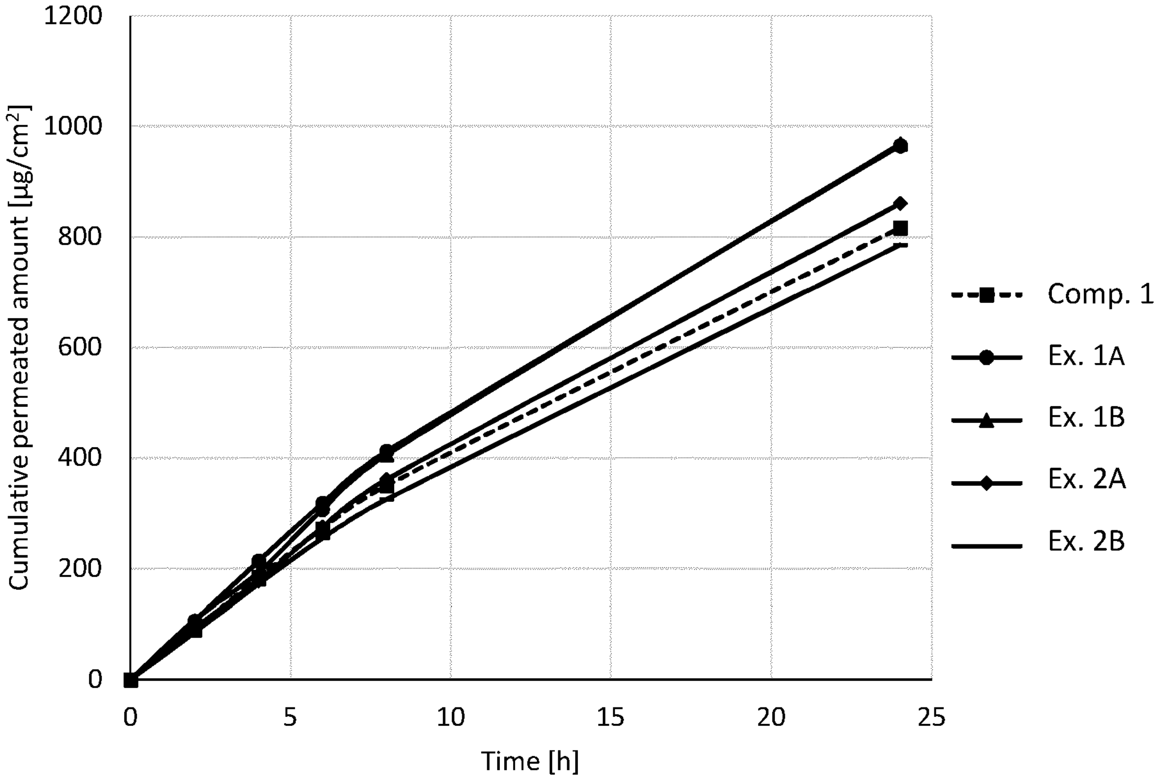
1200
1000
800
600
400
200
0
Cumulative permeated amount [µg/cm2]
0
5
10
15
20
25
Time [h]
Comp. 1
Ex. 1A
Ex. 1B
Ex. 2A
Ex. 2B

FIG. 2

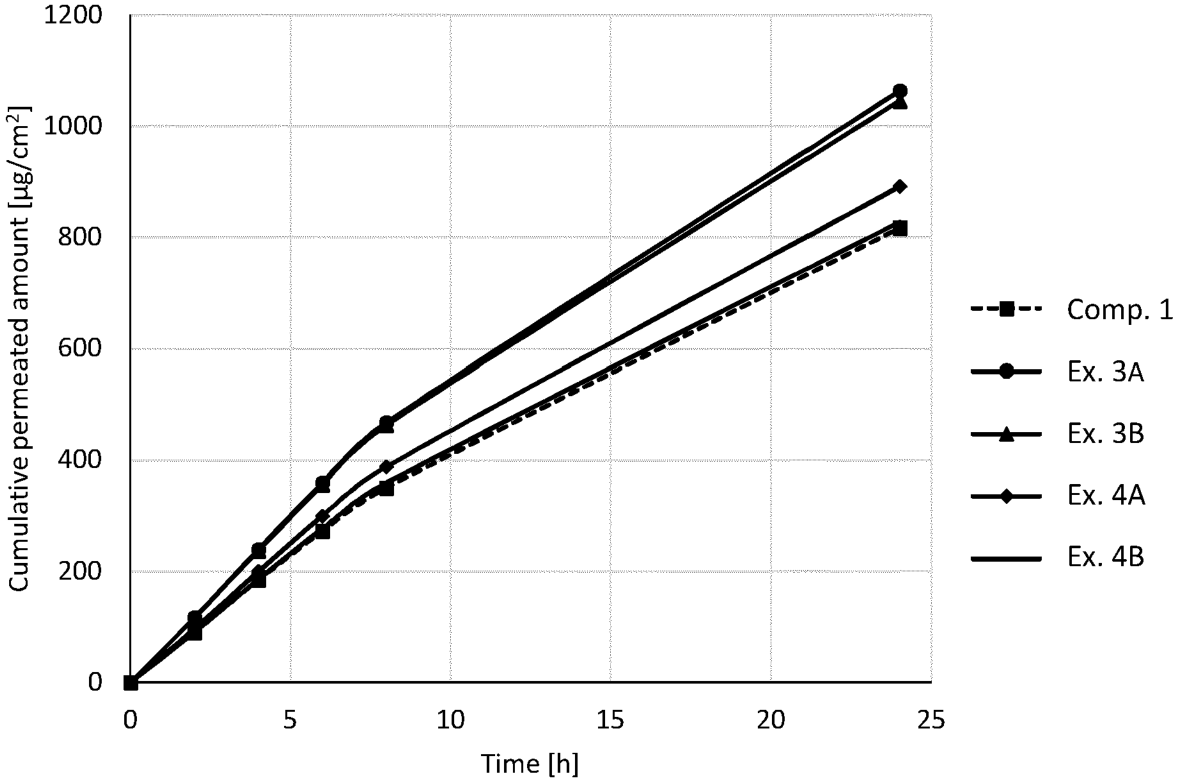
1200
1000
800
600
400
200
0
Cumulative permeated amount [µg/cm2]
0
5
10
15
20
25
Time [h]
Comp. 1
Ex. 3A
Ex. 3B
Ex. 4A
Ex. 4B

FIG. 3

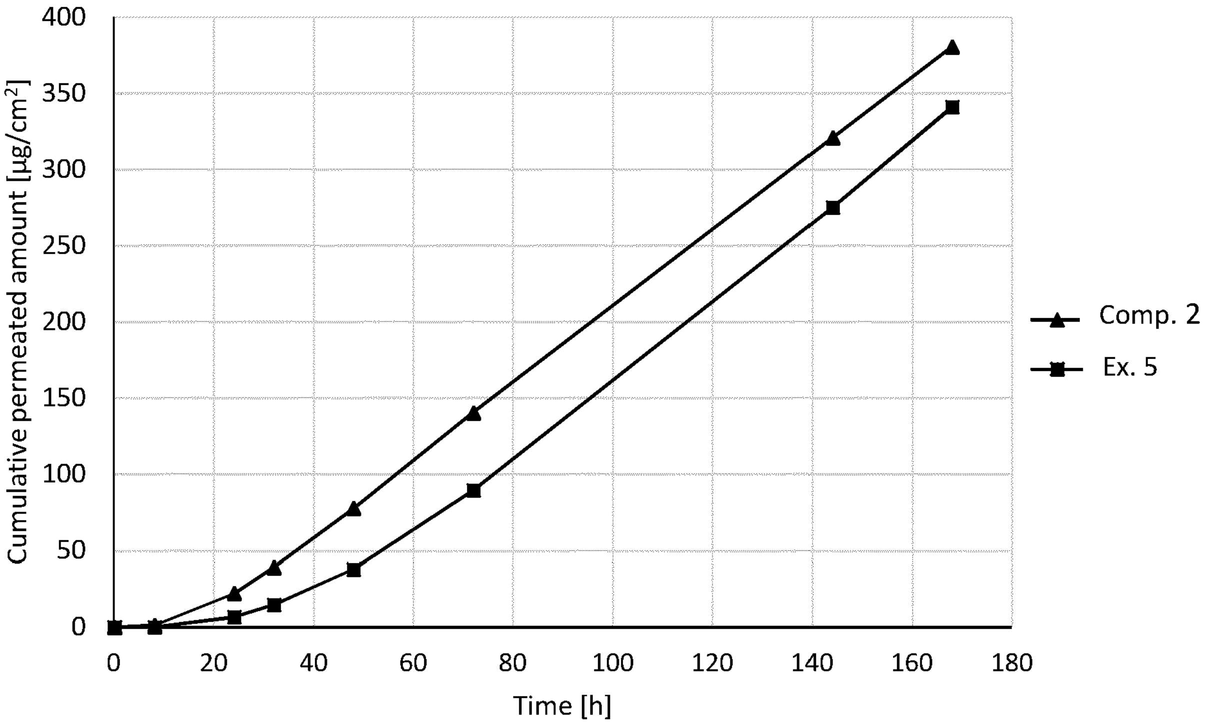
400
350
300
250
200
150
100
50
0
Cumulative permeated amount [µg/cm2]
0
20
40
60
80
100
120
140
160
180
Time [h]
Comp. 2
Ex. 5

FIG. 4

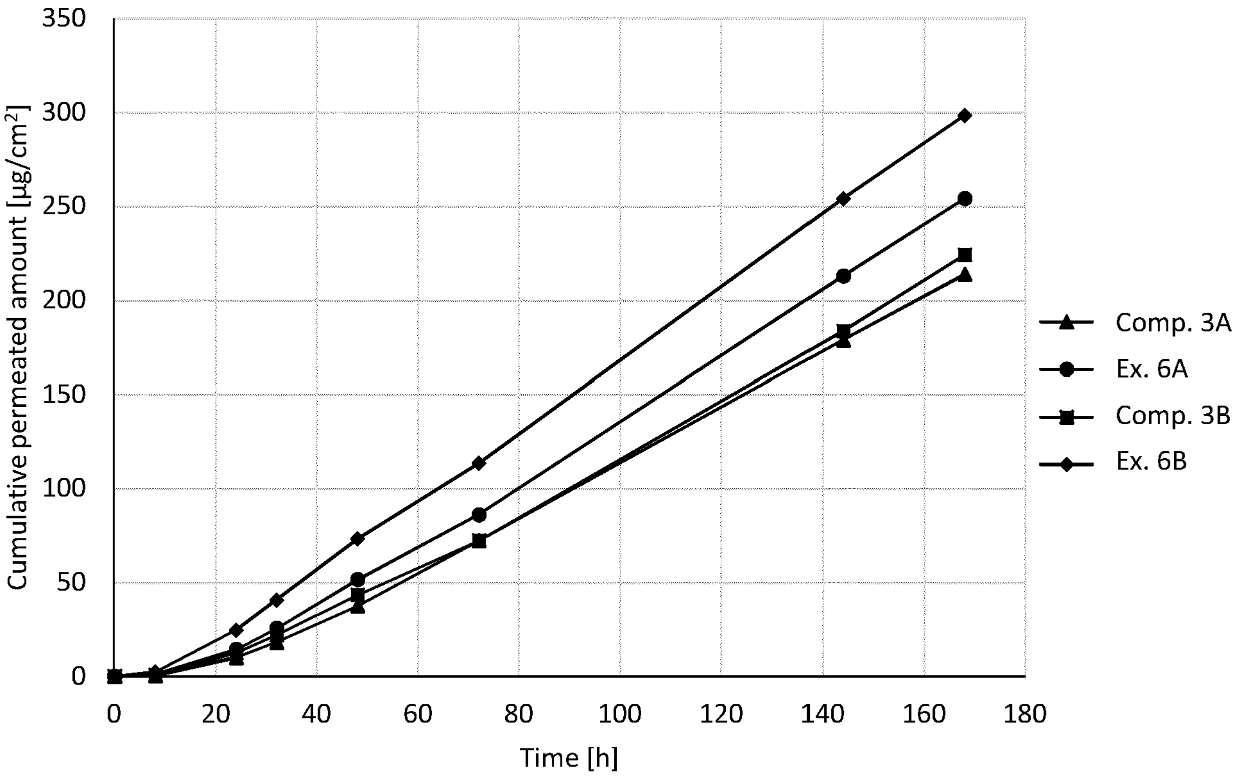
350
300
250
200
150
100
50
0
Cumulative permeated amount [µg/cm2]
0
20
40
60
80
100
120
140
160
180
Time [h]
Comp. 3A
Ex. 6A
Comp. 3B
Ex. 6B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2011022199 A2
- WO 2014028049 A1
- WO 2016130408 A1
- EP 2599847 A
- WO 2016130408 A
- WO 2007145996 A
- EP 2599847 A1
- WO 2010124187 A
- US 5656279 A
- US 5891076 A
- EP 0322118 A
- US 4991574 A
- US 4831070 A
- US 3983298 A
- US 2877255 A, Clark
- JP SHO62198739660 A, Mogi
- US 5446185 A
- US 5493040 A, Cobb
- US 3419593 A
- US 20070202245 A